(19) Europäisches Patentamt
European Patent Office
Office européen des brevets

(11) EP 4 245 763 A1

(12) **EUROPEAN PATENT APPLICATION**
published in accordance with Art. 153(4) EPC

(43) Date of publication:
**20.09.2023 Bulletin 2023/38**

(21) Application number: **21891898.5**

(22) Date of filing: **10.11.2021**

(51) International Patent Classification (IPC):
***C07H 15/04*** (2006.01) ***A61K 39/12*** (2006.01)
***A61K 39/145*** (2006.01) ***A61K 39/165*** (2006.01)
***A61K 39/20*** (2006.01) ***A61K 39/215*** (2006.01)
***A61K 39/235*** (2006.01) ***A61K 39/35*** (2006.01)
***A61K 39/36*** (2006.01) ***A61K 39/39*** (2006.01)
***A61P 31/04*** (2006.01) ***A61P 31/12*** (2006.01)
***A61P 31/16*** (2006.01) ***A61P 33/02*** (2006.01)
***A61P 37/08*** (2006.01) ***A61P 39/02*** (2006.01)

(52) Cooperative Patent Classification (CPC):
**A61K 39/12; A61K 39/145; A61K 39/165; A61K 39/20; A61K 39/215; A61K 39/235; A61K 39/35; A61K 39/36; A61K 39/39; A61P 31/04; A61P 31/12; A61P 31/16; A61P 33/02; A61P 37/08; A61P 39/02;** (Cont.)

(86) International application number:
**PCT/JP2021/041323**

(87) International publication number:
**WO 2022/102652 (19.05.2022 Gazette 2022/20)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **11.11.2020 JP 2020188350**

(71) Applicant: **Daiichi Sankyo Company, Limited**
**Tokyo 103-8426 (JP)**

(72) Inventors:
- **KOBAYASHI Hiroyuki**
  **Tokyo 103-8426 (JP)**
- **OKA Tatsuya**
  **Tokyo 103-8426 (JP)**
- **FUKUYAMA Yoshiko**
  **Tokyo 103-8426 (JP)**

(74) Representative: **Marks & Clerk LLP**
**15 Fetter Lane**
**London EC4A 1BW (GB)**

(54) **NOVEL AMINOALKYL GLUCOSAMINIDE 4-PHOSPHATE DERIVATIVE**

(57) The present invention provides a novel compound or a pharmaceutically acceptable salt thereof which has a TLR4 activating effect and can be used as an immunostimulant or an adjuvant in vaccines or allergen immunotherapy. The present invention provides a compound represented by the general formula (I) or a pharmaceutically acceptable salt thereof. In this context, X, Y, Z and n in the formula (I) are each as defined herein.

EP 4 245 763 A1

[Formula 1]

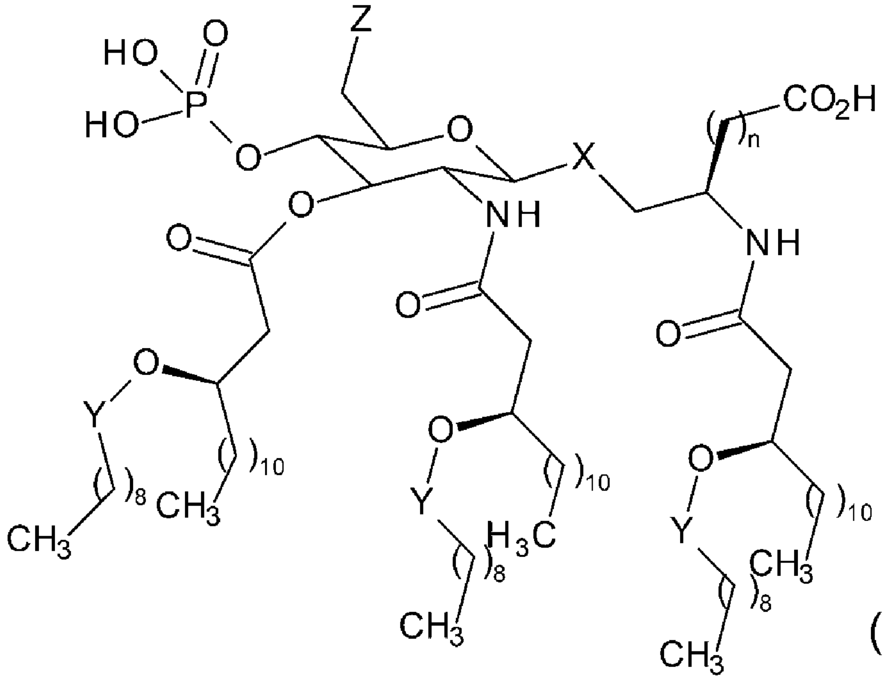

(I)

(52) Cooperative Patent Classification (CPC): (Cont.)
**C07H 15/04;** Y02A 50/30; Y02P 20/55

## Description

### Technical Field

The present invention relates to a novel compound having an immunostimulatory effect. Particularly, the present invention relates to a novel aminoalkylglucosaminide 4-phosphate derivative that potentiates the drug efficacy of a vaccine or allergen immunotherapy, etc.

### Background Art

Having used attenuated viruses or inactivated viruses in vaccines for infection, vaccine preparations with fewer safety problems have been developed by enhancing the purity of antigens in the vaccine preparations in terms of specification and safety. However, this causes a new problem of reduced immunogenicity, resulting in the addition of adjuvants for the purpose of potentiating the drug efficacy of vaccines (Non Patent Reference 1).

Endotoxin of the outer membrane of Gram-negative bacterial cell wall, which was found in 1892, is now recognized as lipopolysaccharide (LPS) and known as a substance that induces shock symptoms or systemic inflammatory response. On the other hand, a small amount of endotoxin is also known to exhibit therapeutic effects on some diseases. In vaccine research, the possibility for endotoxin to be capable of potentiating vaccine effects has been known since the 1800s (Non Patent Reference 2).

The presence of receptors that recognize pathogen-associated molecular patterns (PAMPs) was proposed in 1989 (Non Patent Reference 3). This concept was supported by the finding of mammalian Tool-like receptors (TLRs), and TLR4, a receptor of LPS, was found in 1998 (Non Patent Reference 4).

Lipid A has been isolated as a constituent of LPS, and lipid A has been found to be important for intracellular signaling activation mediated by TLR4 (Non Patent References 5 and 6). Monophosphoryl lipid A (MPLA) was screened for from LPS fractions of *Salmonella typhimurium* in 1982 (Non Patent Reference 7) and has been developed as an adjuvant for injectable vaccines and used in prophylactic vaccines for uterine cervical cancer, vaccines for hepatitis B, or the like. In these vaccine preparations, MPLA is shown to exhibit an immunostimulatory effect and enhance anti-viral antigen-specific IgG production in blood, thereby improving drug efficacy. The usefulness of MPLA in allergen immunotherapy (AIT) has also been confirmed, and MPLA is shown to induce allergen-specific IgG in a short period of time, thereby suppressing allergic symptoms (Non

Patent Reference 8).

The route of infection through the mucosa is known for many human pathogens, and secretory IgA which resides on the mucosal surface is important for mucosal protection against viruses or bacteria (Non Patent Reference 9). However, general injectable vaccines for infection cannot efficiently induce mucosal IgA. On the other hand, mucosal vaccines have been energetically developed because immunity mediated by the mucosa efficiently induces mucosal IgA (Non Patent Reference 10). However, such vaccines have technical hurdles in clinical application, and only a small number of vaccines have been launched. The importance of adjuvants is mentioned as one of the techniques for clinical application (Non Patent Reference 11).

AIT has been found as a therapy that alleviates allergic symptoms by the subcutaneous administration (subcutaneous immunotherapy: SCIT) of an allergen causative of allergic rhinitis (Non Patent Reference 12). Under a putative mechanism of AIT, induced allergen-specific IgG, particularly, IgG4, captures the allergen so that the binding of the allergen to IgE on effector cells such as mast cells is competitively inhibited (Non Patent References 13 to 15). Particularly, clinical results showing that allergic rhinitis symptoms ascribable to a cat antigen were improved by the administration of an allergen-specific IgG4 preparation against the cat antigen (Non Patent Reference 16) strongly suggests that in AIT as well, induced allergen-specific IgG4 is a major mechanism for the exertion of AIT drug efficacy.

SCIT has a challenge in its versatility because of the necessity of weekly subcutaneous administration usually lasting for 3 to 5 years or the risk of inducing severe systemic allergic response has been pointed out (Non Patent Reference 17). Accordingly, sublingual immunotherapy (SLIT) was studied as AIT directed to higher safety, and sublingual administration preparations were approved in 2011 by the Food and Drug Administration (FDA). Then, SLIT against various allergens has spread rapidly because of safety as well as convenience. On the other hand, the treatment period of SLIT is also as long as 3 to 5 years. Therefore, there are unmet needs for the exertion of therapeutic effects in a shorter period of time and high drug efficacy.

Although AIT targeting food allergy is not practiced by SCIT, particularly, due to high safety concerns, oral immunotherapy (OIT) has been studied as a substitute. A plurality of comparative tests of OIT and SLIT using a peanut antigen have been conducted. It is generally recognized that OIT is excellent in drug efficacy while a feature of SLIT is excellent safety. However, there are unmet needs for higher safety and high drug efficacy. The development of a mucosal

immunostimulant (adjuvant) for SLIT preparations is expected as one approach thereto (Non Patent Reference 18).

CRX-527 is known as an analogous compound of lipid A (Patent Reference 1).

## Citation List

### Patent References

Patent Reference 1: WO1998/50399

### Non Patent References

Non Patent Reference 1: McKee AS, et al., BMC biology. 2010; 8: 37
Non Patent Reference 2: Arakawa T. Expert review of vaccines. 2011; 10 (1): 1-5
Non Patent Reference 3: Janeway CA, Jr. Cold Spring Harbor symposia on quantitative biology. 1989; 54 Pt 1: 1-13
Non Patent Reference 4: Poltorak A, et al., Science. 1998; 282 (5396): 2085-8
Non Patent Reference 5: Luderitz O, Galanos C, Lehmann V, Nurminen M, Rietschel ET, Rosenfelder G, et al., The Journal of infectious diseases. 1973; 128: Suppl: 17-29 Non Patent Reference 6: Mata-Haro V, et al., Science. 2007; 316 (5831): 1628-32
Non Patent Reference 7: Qureshi N, et al., The Journal of biological chemistry. 1982; 257 (19): 11808-15
Non Patent Reference 8: Drachenberg KJ, et al., Allergy. 2001; 56 (6): 498-505
Non Patent Reference 9: McGhee JR, Fujihashi K. PLoS biology. 2012; 10 (9): e1001397
Non Patent Reference 10: Lycke N. Nature reviews Immunology. 2012; 12 (8): 592-605
Non Patent Reference 11: Holmgren J, Czerkinsky C. Nature medicine. 2005; 11 (4 Suppl): S45-53
Non Patent Reference 12: Noon L. Lancet. 1911; 177 (4580): 1572-3
Non Patent Reference 13: Shamji MH, Durham SR. The Journal of allergy and clinical immunology. 2017; 140 (6): 1485-98
Non Patent Reference 14: Larsen JN, et al., Drug discovery today. 2016; 21 (1): 26-37
Non Patent Reference 15: Akdis M, Akdis CA. The Journal of allergy and clinical immunology. 2014; 133 (3): 621-31
Non Patent Reference 16: Orengo JM, et al., Nature communications. 2018; 9 (1): 1421
Non Patent Reference 17: CSM update: desensitizing vaccines. Committee on the safety of medicines. Br Med J. 1986; 293: 948
Non Patent Reference 18: Nowak-Wegrzyn A, et al., Current opinion in allergy and clinical immunology. 2019; 19 (6): 606-13

## Summary of Invention

### Technical Problem

The present invention provides a novel compound or a pharmaceutically acceptable salt thereof which has a TLR4 activating effect and can be used as an immunostimulant or adjuvant in vaccines or allergen immunotherapy.

### Solution to Problem

The present invention relates to the following (1) to (15).

(1) A compound represented by the general formula (I) :

[Formula 1]

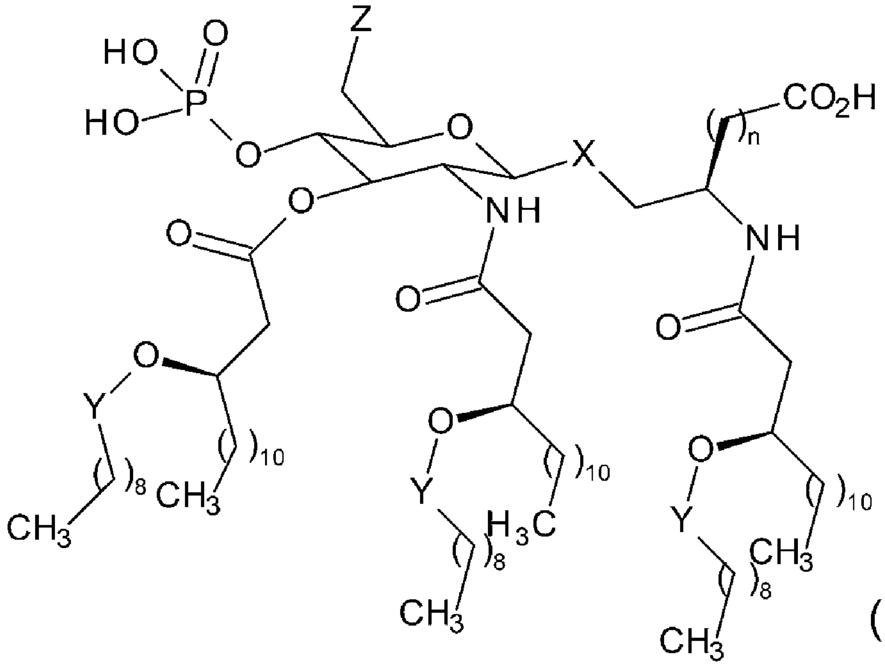

(I)

wherein

X represents an oxygen atom or $CH_2$,
Y represents $CH_2$ or C=O,
Z represents a halogen atom or $OR^1$,
$R^1$ represents a hydrogen atom or the following formula (II) :

[Formula 2]

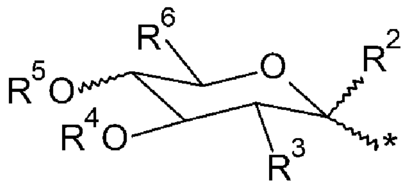

(II)

wherein

$R^2$ represents a hydrogen atom or a carboxy group,
$R^3$ represents a hydrogen atom, a hydroxy group, or an acetylamino group,
$R^4$ represents a hydrogen atom or the following formula (III):

[Formula 3]

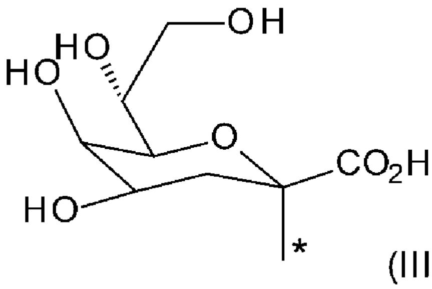

(III)

$R^5$ represents a hydrogen atom or a phosphoric acid group, and
$R^6$ represents a hydroxymethyl group, a methyl phosphate group, a carboxy group, or a (1S)-1,2-dihydroxyethyl group, and

n represents 0 or 1,

or a pharmaceutically acceptable salt thereof,

with the proviso that a compound is excluded wherein X represents an oxygen atom, n represents 0, Z represents $OR^1$, and $R^1$ represents a hydrogen atom.

(2) The compound according to (1) or a pharmaceutically acceptable salt thereof, wherein in the formula (I),

X represents an oxygen atom or $CH_2$,
Y represents C=O,
Z represents the following formula (IV):

[Formula 4]

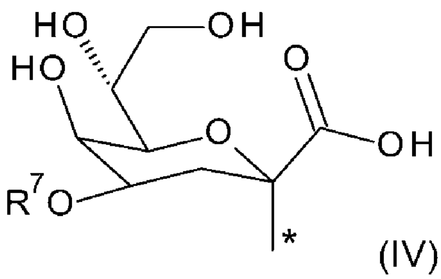

(IV)

wherein
$R^7$ represents a hydrogen atom or the formula (III), and
n represents 0 or 1,
with the proviso that a compound is excluded wherein X represents $CH_2$, n represents 1, and $R^7$ represents the formula (III).

(3) The compound according to (1) or a pharmaceutically acceptable salt thereof, wherein in the formula (I),

X represents an oxygen atom,
Y represents C=O,
Z represents the following formula (V),

[Formula 5]

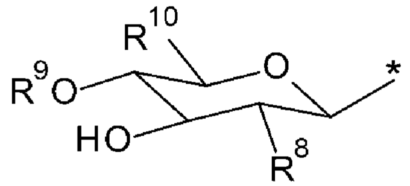

(V)

wherein

$R^8$ represents a hydroxy group or an acetylamino group,
$R^9$ represents a hydrogen atom or a phosphoric acid group, and
$R^{10}$ represents a hydroxymethyl group, a methyl phosphate group, or a carboxy group, and

n represents 0.

(4) Any one compound selected from the following group:

(3R)-3-{[(3R)-3-(decanoyloxy)tetradecanoyl]amino}-4-({3-O-[(3R)-3-(decanoyloxy)tetradecanoyl]-2-{[(3R)-3-(decanoyloxy)tetradecanoyl]amino}-2-deoxy-6-O-(3-deoxy-α-D-manno-oct-2-ulopyranonosyl)-4-O-phosphono-β-D-glucopyranosyl}oxy)butanoic acid,
(2S)-2-{ [(3R)-3-(decanoyloxy)tetradecanoyl]amino}-3-{ [3-deoxy-α-D-manno-oct-2-ulopyranonosyl-(2→4)-3-deoxy-α-D-manno-oct-2-ulopyranonosyl-(2→6)-3-O-[(3R)-3-(decanoyloxy)tetradecanoyl]-2-{[(3R)-3-(decanoyloxy)tetradecanoyl]amino}-2-deoxy-4-O-phosphono-P-D-glucopyranosyl]oxy}propanoic acid,
(2S)-2-{[(3R)-3-(decanoyloxy)tetradecanoyl]amino}-3-({3-O-[(3R)-3-(decanoyloxy)tetradecanoyl]-2-{[(3R)-3-(decanoyloxy)tetradecanoyl]amino}-2-deoxy-6-O-D-glucopyranuronosyl-4-O-phosphono-β-D-glucopyranosyl}oxy)propanoic acid,
6,10-anhydro-8-O-[(3R)-3-(decanoyloxy)tetradecanoyl]-3,7-bis{[(3R)-3-(decanoyloxy)tetradecanoyl]amino}-2,3,4,5,7-pentadeoxy-11-O-(3-deoxy-α-D-manno-oct-2-ulopyranonosyl)-9-O-phosphono-D-erythro-L-galacto-undecanoic acid, and
5,9-anhydro-7-O-[(3R)-3-(decanoyloxy)tetradecanoyl]-2,6-bis{[(3R)-3-(decanoyloxy)tetradecanoyl]amino}-2,3,4,6-tetradeoxy-10-O-(3-deoxy-α-D-manno-oct-2-ulopyranonosyl)-8-O-phosphono-D-erythro-L-galacto-decanoic acid,
or a pharmaceutically acceptable salt thereof.

(5) (3R)-3-{[(3R)-3-(Decanoyloxy)tetradecanoyl]amino}-4-({3-O-[(3R)-3-(decanoyloxy)tetradecanoyl]-2-{[(3R)-3-(decanoyloxy)tetradecanoyl]amino}-2-deoxy-6-O-(3-deoxy-α-D-manno-oct-2-ulopyranonosyl)-4-O-phosphono-β-D-glucopyranosyl}oxy)butanoic acid or a pharmaceutically acceptable salt thereof.
(6) A pharmaceutical composition comprising a compound according to any one of (1) to (5) or a pharmaceutically acceptable salt thereof.
(7) A pharmaceutical composition comprising a compound according to any one of (1) to (5) or a pharmaceutically acceptable salt thereof and an antigen.
(8) A pharmaceutical composition wherein a compound according to any one of (1) to (5) or a pharmaceutically acceptable salt thereof and an antigen are administered in combination at the same time or at different times.
(9) The pharmaceutical composition according to (7) or (8), wherein the antigen is one or more selected from the group consisting of an attenuated virus, an inactivated virus, and a recombinant protein of a viral structural protein of influenza virus, adenovirus, rubella virus, mumps virus, RS virus, enterovirus, rotavirus, norovirus, or coronavirus, Japanese cedar pollen, cypress pollen, birch pollen, ragweed pollen, goldenrod pollen, Japanese hop pollen, orchard grass pollen, spinach pollen, black pine pollen, narrow leaf cattail pollen, red pine pollen, chrysanthemum pollen, artemisia pollen, timothy pollen, Bermuda grass pollen, Kentucky grass pollen, meadow fescue grass pollen, orchard grass pollen, redtop grass pollen, perennial ryegrass pollen, sweet vernal grass pollen, fat hen pollen, mite, cat hair, chicken egg, milk, peanut, wheat, and buckwheat.
(10) The pharmaceutical composition according to any one of (6) to (9) for the prevention or treatment of viral

infection, allergy disease, bacterial infection and bacterium-derived toxin, cancer, or intracellular parasitic protozoa.

(11) The pharmaceutical composition according to any one of (6) to (9) for the prevention or treatment of influenza virus, coronavirus, RS virus, norovirus, or rotavirus infection.

(12) The pharmaceutical composition according to any one of (6) to (9) for the prevention or treatment of allergy disease caused by Japanese cedar pollen, cypress pollen, birch pollen, ragweed pollen, goldenrod pollen, Japanese hop pollen, orchard grass pollen, spinach pollen, black pine pollen, narrow leaf cattail pollen, red pine pollen, chrysanthemum pollen, artemisia pollen, timothy pollen, Bermuda grass pollen, Kentucky grass pollen, meadow fescue grass pollen, orchard grass pollen, redtop grass pollen, perennial ryegrass pollen, sweet vernal grass pollen, fat hen pollen (*Chenopodium album* [white goosefoot or lamb's quarters]), mite, cat hair, chicken egg, milk, peanut, wheat, or buckwheat.

(13) A TLR4 activator comprising a compound according to any one of (1) to (5) or a pharmaceutically acceptable salt thereof.

(14) An immunostimulant comprising a compound according to any one of (1) to (5) or a pharmaceutically acceptable salt thereof.

(15) The immunostimulant according to (14), wherein the immunostimulant is a vaccine adjuvant.

Advantageous Effects of Invention

The aminoalkylglucosaminide 4-phosphate derivative of the present invention or a pharmaceutically acceptable salt thereof has a TLR4 activating effect and is effective for the prevention or treatment of viral infection, allergy disease, bacterial infection and bacterium-derived toxin, cancer, or a disease caused by intracellular parasitic protozoan.

Brief Description of Drawings

[Figure 1] Figure 1 shows time-dependent change in egg albumin-specific IgG concentration in blood after sublingual administration of a compound described in Example 2(2e) at the same time with egg albumin.

[Figure 2] Figure 2 shows time-dependent change in egg albumin-specific IgA concentration in blood after sublingual administration of the compound described in Example 2(2e) at the same time with egg albumin.

[Figure 3] Figure 3 shows time-dependent change in Japanese cedar pollen antigen-specific IgG titer in blood after sublingual administration of the compound described in Example 2(2e) at the same time with Japanese cedar pollen antigen extracts.

[Figure 4] Figure 4 shows time-dependent change in mite antigen extract-specific IgG titer in blood after sublingual administration of the compound described in Example 2(2e) at the same time with mite antigen extracts.

[Figure 5] Figure 5 shows time-dependent change in ragweed pollen antigen extract-specific IgG titer in blood after sublingual administration of the compound described in Example 2(2e) at the same time with ragweed pollen antigen extracts.

[Figure 6] Figure 6 shows time-dependent change in timothy pollen antigen extract-specific IgG titer in blood after sublingual administration of the compound described in Example 2(2e) at the same time with timothy pollen antigen extracts.

[Figure 7] Figure 7 shows time-dependent change in peanut antigen extract-specific IgG titer in blood after sublingual administration of the compound described in Example 2(2e) at the same time with peanut antigen extracts.

[Figure 8] Figure 8 shows time-dependent change in milk antigen-specific IgG titer in blood after sublingual administration of the compound described in Example 2(2e) at the same time with a milk antigen.

[Figure 9] Figure 9 shows an anti-Cry j 1 IgG concentration in blood after sublingual administration of a compound described in Example 24 at the same time with a Japanese cedar pollen antigen.

[Figure 10] Figure 10 shows an anti-Cry j 1 IgA concentration in blood after sublingual administration of the compound described in Example 24 at the same time with a Japanese cedar pollen antigen.

[Figure 11] Figure 11 shows an anti-Cry j 1 IgA concentration in nasal wash after sublingual administration of the compound described in Example 24 at the same time with a Japanese cedar pollen antigen.

[Figure 12] Figure 12 shows the amount of IL-10 produced by Cry j 1 stimulation from cervical lymph node immune cells after sublingual administration of the compound described in Example 24 at the same time with a Japanese cedar pollen antigen.

[Figure 13] Figure 13 shows the amount of IFN-$\gamma$ produced by Cry j 1 stimulation from cervical lymph node immune cells after sublingual administration of the compound described in Example 24 at the same time with a Japanese cedar pollen antigen.

[Figure 14] Figure 14 shows the amount of IL-4 produced by Cry j 1 stimulation from cervical lymph node immune

cells after sublingual administration of the compound described in Example 24 at the same time with a Japanese cedar pollen antigen.

[Figure 15] Figure 15 shows the amount of mast cell degranulation via anti-Cry j 1 IgE by Cry j 1 stimulation.

[Figure 16] Figure 16 shows the amount of mast cell degranulation in a Cry j 1 concentration-dependent manner.

[Figure 17] Figure 17 shows the inhibitory effect of IgG in blood on mast cell degranulation reaction ascribable to Cry j 1 after sublingual administration of the compound described in Example 24 at the same time with a Japanese cedar pollen antigen.

[Figure 18] Figure 18 shows an anti-RBD IgG concentration in blood after sublingual administration of the compound described in Example 2(2e) at the same time with a recombinant protein of novel coronavirus receptor binding domain (RBD).

[Figure 19] Figure 19 shows an anti-RBD IgA concentration in blood after sublingual administration of the compound described in Example 2(2e) at the same time with a recombinant RBD protein.

[Figure 20] Figure 20 shows anti-RBD IgA (OD) in nasal wash after sublingual administration of the compound described in Example 2(2e) at the same time with a recombinant RBD protein.

[Figure 21] Figure 21 shows the inhibitory activity of nasal wash against the binding between a recombinant RBD protein and a recombinant hACE2 protein after sublingual administration of the compound described in Example 2(2e) at the same time with a recombinant novel coronavirus RBD protein.

Description of Embodiments

In the present invention, "*" represents a binding site with a carbon atom or an oxygen atom.

"Wavy line" in the formula (II) of the present invention represents that a substituent is located at the axial or equatorial position.

In the present invention, "halogen atom" is, for example, a fluorine atom, a chlorine atom, a bromine atom, or an iodine atom. The halogen atom is preferably a fluorine atom.

Next, preferred substituents in the general formula (I) will be described.

X is preferably an oxygen atom.

Y is preferably C=O.

Z is preferably the following formula (VI) or (VII):

[Formula 6]

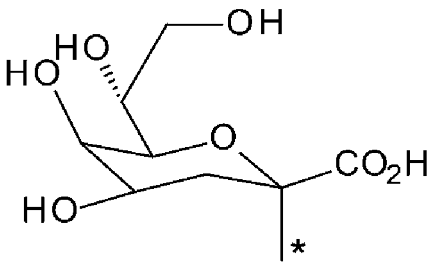

(VI)

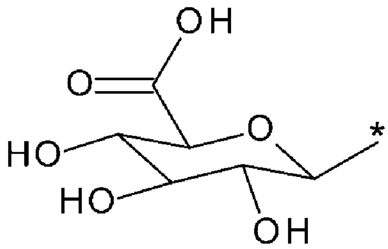

(VII)

n is preferably 1.

As for a preferred combination of X, Y, Z and n, X is an oxygen atom, Y is C=O, Z is the formula (VI), and n is 1.

As for another preferred combination of X, Y, Z and n, X is an oxygen atom, Y is C=O, Z is the formula (VII), and n is 0.

A preferred compound of the present invention is (3R)-3-{[(3R)-3-(decanoyloxy)tetradecanoyl]amino}-4-({3-O-[(3R)-3-(decanoyloxy)tetradecanoyl]-2-{[(3R)-3-(decanoyloxy)tetradecanoyl]amino}-2-deoxy-6-O-(3-deoxy-α-D-manno-oct-2-ulopyranonosyl)-4-O-phosphono-β-D-glucopyranosyl}oxy)butanoic acid or a pharmaceutically acceptable

salt thereof.

A preferred compound of the present invention is (2S)-2-{[(3R)-3-(decanoyloxy)tetradecanoyl]amino}-3-{[3-deoxy-α-D-manno-oct-2-ulopyranonosyl-(2→4)-3-deoxy-(α-D-manno-oct-2-ulopyranonosyl-(2→6)-3-O-[(3R)-3-(decanoyloxy)tetradecanoyl]-2-{[(3R)-3-(decanoyloxy)tetradecanoyl]amino}-2-deoxy-4-O-phosphono-P-D-glucopyranosyl]oxy}propanoic acid or a pharmaceutically acceptable salt thereof.

A preferred compound of the present invention is (2S)-2-{[(3R)-3-(decanoyloxy)tetradecanoyl]amino}-3-({3-O-[(3R)-3-(decanoyloxy)tetradecanoyl]-2-{[(3R)-3-(decanoyloxy)tetradecanoyl]amino}-2-deoxy-6-O-D-glucopyranuronosyl-4-O-phosphono-β-D-glucopyranosyl}oxy)propanoic acid or a pharmaceutically acceptable salt thereof.

A preferred compound of the present invention is 6,10-anhydro-8-O-[(3R)-3-(decanoyloxy)tetradecanoyl]-3,7-bis{[(3R)-3-(decanoyloxy)tetradecanoyl]amino}-2,3,4,5,7-pentadeoxy-11-O-(3-deoxy-α-D-manno-oct-2-ulopyranonosyl)-9-O-phosphono-D-erythro-L-galacto-undecanoic acid or a pharmaceutically acceptable salt thereof.

A preferred compound of the present invention is 5,9-anhydro-7-O-[(3R)-3-(decanoyloxy)tetradecanoyl]-2,6-bis{[(3R)-3-(decanoyloxy)tetradecanoyl]amino}-2,3,4,6-tetradeoxy-10-O-(3-deoxy-α-D-manno-oct-2-ulopyranonosyl)-8-O-phosphono-D-erythro-L-galacto-decanoic acid or a pharmaceutically acceptable salt thereof.

A more preferred compound of the present invention is meglumine (3R)-3-{[(3R)-3-(decanoyloxy)tetradecanoyl]amino}-4-({3-O-[(3R)-3-(decanoyloxy)tetradecanoyl]-2-{[(3R)-3-(decanoyloxy)tetradecanoyl]amino}-2-deoxy-6-O-(3-deoxy-α-D-manno-oct-2-ulopyranonosyl)-4-O-phosphono-β-D-glucopyranosyl}oxy)butanoate.

A more preferred compound of the present invention is sodium (3R)-3-{[(3R)-3-(decanoyloxy)tetradecanoyl]amino}-4-({3-O-[(3R)-3-(decanoyloxy)tetradecanoyl]-2-{[(3R)-3-(decanoyloxy)tetradecanoyl]amino}-2-deoxy-6-O-(3-deoxy-α-D-manno-oct-2-ulopyranonosyl)-4-O-phosphono-β-D-glucopyranosyl}oxy)butanoate.

The compound represented by the general formula (I) of the present invention or the pharmaceutically acceptable salt thereof can be used as an active ingredient or an additive in a medicament. Whether it is treated as an active ingredient or an additive depends on the law of each country.

The compound represented by the general formula (I) of the present invention can be prepared as a pharmaceutically acceptable salt thereof, if desired. The pharmaceutically acceptable salt thereof refers to a salt that has no significant toxicity and can be used as a medicament. The compound represented by the general formula (I) of the present invention can be reacted with a base to form a salt.

Examples thereof can include: alkali metal salts such as sodium salt, potassium salt, and lithium salt; alkaline earth metal salts such as calcium salt and magnesium salt; metal salts such as aluminum salt and iron salt; inorganic salts such as ammonium salt; and amine salts including organic salts such as t-butylamine salt, t-octylamine salt, dibenzylamine salt, morpholine salt, glucosamine salt, phenyl glycine alkyl ester salt, ethylenediamine salt, guanidine salt, diethylamine salt, triethylamine salt, dicyclohexylamine salt, N,N'-dibenzylethylenediamine salt, chloroprocaine salt, procaine salt, diethanolamine salt, triethanolamine salt, N-benzylphenethylamine salt, piperazine salt, tetramethylammonium salt, tris(hydroxymethyl)aminomethane salt, and meglumine salt. The pharmaceutically acceptable salt is preferably meglumine salt or sodium salt, more preferably meglumine salt.

The compound represented by the general formula (I) of the present invention or the pharmaceutically acceptable salt thereof may form a hydrate by incorporating a water molecule when left in the atmosphere or recrystallized. Such a hydrate is also encompassed by the compound or the salt of the present invention.

The compound represented by the general formula (I) of the present invention or the pharmaceutically acceptable salt thereof may form a solvate by absorbing a certain solvent when left in a solvent or recrystallized. Such a solvate is also encompassed by the compound or the salt of the present invention.

A compound that is converted to a compound represented by the general formula (I) which serves as an active ingredient in the pharmaceutical composition of the present invention by reaction through an enzyme, gastric acid, or the like under physiological conditions *in vivo,* i.e., a compound that is converted to a compound represented by the general formula (I) by enzymatic oxidation, reduction, hydrolysis, or the like, or a compound that is converted to a compound represented by the general formula (I) by hydrolysis or the like caused by gastric acid or the like, is included as a "pharmaceutically acceptable prodrug compound" in the scope of the present invention.

Examples of the prodrug can include, when the compound represented by the general formula (I) has an amino group, compounds in which the amino group is acylated, alkylated, or phosphorylated (e.g., compounds in which the amino group is eicosanoylated, alanylated, pentylaminocarbonylated, (5-methyl-2-oxo-1,3-dioxolen-4-yl)methoxycarbonylated, tetrahydrofuranylated, pyrrolidylmethylated, pivaloyloxymethylated, or tert-butylated). Examples thereof include, when the compound represented by the general formula (I) has a hydroxy group, compounds in which the hydroxy group is acylated, alkylated, phosphorylated, or borated (e.g., compounds in which the hydroxy group is acetylated, palmitoylated, propanoylated, pivaloylated, succinylated, fumarylated, alanylated, or dimethylaminomethylcarbonylated). Examples thereof include, when the compound represented by the general formula (I) has a carboxy group, compounds in which the carboxy group is esterified or amidated (e.g., compounds in which the carboxy group is ethyl esterified, phenyl esterified, carboxymethyl esterified, dimethylaminomethyl esterified, pivaloyloxymethyl esterified, ethoxycarbonyloxyethyl esterified, or methylamidated).

The prodrug according to the present invention can be produced from the compound represented by the general formula (I) by a method known in the art. The prodrug according to the present invention also includes a compound that is converted to a compound represented by the general formula (I) under physiological conditions as described in "Iyakuhin No Kaihatsu (Development of Pharmaceuticals in English)", Vol. 7, Bunshi Sekkei (Molecular Design in English), Hirokawa-Shoten Ltd., 1990, pp. 163-198.

The compound represented by the general formula (I) of the present invention or the pharmaceutically acceptable salt thereof encompasses all stereoisomers.

For the compound represented by the general formula (I) of the present invention or the pharmaceutically acceptable salt thereof, its isomers and mixtures of these isomers are all represented by a single formula, i.e., the general formula (I). Thus, the present invention includes all of these isomers and even mixtures of these isomers at arbitrary ratios.

The compound represented by the general formula (I) of the present invention or the pharmaceutically acceptable salt thereof may also contain unnatural proportions of atomic isotopes at one or more of the atoms constituting such a compound. Examples of the atomic isotopes include deuterium ($^{2}H$), tritium ($^{3}H$), iodine-125 ($^{125}I$), and carbon-14 ($^{14}C$). The compound may be radiolabeled with a radioisotope such as tritium ($^{3}H$), iodine-125 ($^{125}I$), or carbon-14 ($^{14}C$). The radiolabeled compound is useful as a therapeutic or prophylactic agent, a research reagent (e.g., an assay reagent), and a diagnostic agent (e.g., an *in vivo* diagnostic imaging agent). All isotopic variants of the compound of the present invention are included in the scope of the present invention, regardless of being radioactive or not.

In the present invention, "antigen" means a generic name for substances that induce an immune response. Particularly, a substance containing an antigen that causes an allergic response is also referred to as an "allergen". For example, attenuated viruses, inactivated viruses, recombinant proteins of viral structural proteins, various pollens, insects, organisms, and foods are known as antigens or allergens. Examples thereof include attenuated viruses, inactivated viruses, and recombinant proteins of viral structural proteins of influenza virus, coronavirus, RS virus, norovirus, or rotavirus, attenuated viruses, inactivated viruses, and recombinant proteins of viral structural proteins of influenza virus, adenovirus, rubella virus, mumps virus, RS virus, enterovirus, rotavirus, norovirus, or coronavirus, Japanese cedar pollen, cypress pollen, birch pollen, ragweed pollen, goldenrod pollen, Japanese hop pollen, orchard grass pollen, spinach pollen, black pine pollen, narrow leaf cattail pollen, red pine pollen, chrysanthemum pollen, artemisia pollen, timothy pollen (timothy grass), Bermuda grass pollen, Kentucky grass pollen, meadow fescue grass pollen, orchard grass pollen, redtop grass pollen, perennial ryegrass pollen, sweet vernal grass pollen, fat hen pollen (*Chenopodium album* [white goosefoot or lamb's quarters]), mite, cat hair, chicken egg, milk, peanut, wheat, and buckwheat.

More specifically, for example, Japanese cedar pollen (Cry j 1, Cry j 2, and Cry j 3), cypress pollen (Cha o 1, Cha o 2, and Cha o 3), birch pollen (Bet v 1, Bet v 2, Bet v 3, Bet v 4, Bet v 6, Bet v 7, and Bet v 8), ragweed pollen (short ragweed pollen, Amb a 1, Amb a 2, Amb a 3, Amb a 4, Amb a 5, Amb a 6, Amb a 7, Amb a 8, Amb a 9, Amb a 10, Amb a 11, and Amb a 12), goldenrod pollen, Japanese hop pollen, orchard grass pollen, spinach pollen, black pine pollen, narrow leaf cattail pollen, red pine pollen, chrysanthemum pollen, artemisia pollen, timothy pollen (timothy grass, Phl p 1, Phl p 2, Phl p 4, Phl p 5, Phl p 6, Phl p 7, Phl p 11, Phl p 12, and Phl p 13), Bermuda grass pollen (Cyn d 1), Kentucky grass pollen (Poa p 1, Poa p 5, and Poa p 9), meadow fescue grass pollen (Fes e 1, Fes e 3, Fes e 4, and Fes e 5), orchard grass pollen (Dac g 1, Dac g 2, and Dac g 5), redtop grass pollen (Agr a 1), perennial ryegrass pollen (Lol p 1, Lol p 2, Lol p 3, Lol p 5, and Lol p 9), sweet vernal grass pollen (Ant o 1), fat hen pollen (*Chenopodium album* [White Goosefoot or Lamb's quarters], Che a 1, Che a 2, and Che a 3), mite (Der f 1, Der f 2, Der f 3 to 39, Der p 1, Der p 2, and Der p 3 to 38), cat hair, chicken egg (Gal d 1, Gal d 2, Gal d 3, Gal d 4, and Gal d 5), milk (Bos d 4, Bos d 5, Bos d 6, Bos d 7, Bos d 8, Bos d 9, Bos d 10, Bos d 11, and Bos d 12), peanut (Ara h 1, Ara h 2, Ara h 3, Ara h 4, Ara h 5, Ara h 6, Ara h 7, Ara h 8, Ara h 9, Ara h 10, Ara h 11, Ara h 12, Ara h 13, Ara h 14, Ara h 15, Ara h 16, and Ara h 17), wheat (Tri a 14, Tri a 15, Tri a 19, Tri a 20, Tri a 21, Tri a 26, Tri a 28, Tri a 29, Tri a 30, and Tri a 36), or buckwheat or allergen extracts therefrom can be used as the allergen.

In the present invention, "viral infection" refers to a state of being infected by a virus through the swallowing or inhalation of the virus, insect biting, trauma or sexual contact, etc., and also includes a state of a developed disease derived from infection by a virus.

In the present invention, "allergy disease" means a systemic or local pathological condition in the living body based on an immune response caused by the entry of an allergen into the body. Examples of the allergy disease include allergic rhinitis, food allergy disease, and atopic dermatitis.

Responses ascribable to allergy can be classified into immediate allergic response and non-immediate allergic response. Immediate allergic response refers to a response that is caused by the release of a chemical transmitter such as histamine or leukotriene from mast cells when the mast cells (which reside in the skin, the gut mucosa, the bronchial mucosa, the nasal mucosa, conjunctiva, and the like) in a state bound with an IgE antibody encounter an antigen. Non-immediate allergic response is a response independent from an IgE antibody, and the possibility of involvement of T cells has been suggested.

"Ig" is an abbreviation of immunoglobulin and refers to an antibody. The antibody is a protein that is produced and released by B cells, and binds to foreign matter, such as a pathogen, which has entered the body.

"IgE" is a human serum immunoglobulin and is particularly involved in allergic response or the like.

The avoidance of a causative antigen or the removal thereof, drug therapy with an antiallergic drug or the like, and allergen immunotherapy (AIT) are known as methods for coping with allergy disease and typical methods for treating allergy disease.

In the present invention, "TLR4" means Toll-like receptor 4 and is a receptor that recognizes a molecule characteristic of a pathogen. The activation of TLR4 is known to promote the induction of IgG and IgA specific for an antigen.

"IgG" is a human serum immunoglobulin and is involved in the detoxification of risk factors and the recognition of antigen-antibody complexes by leucocytes or macrophages.

"IgA" is a human serum immunoglobulin, which exists abundantly in serum as well as nasal discharge, saliva, breast milk, intestinal fluid, and the like, and is involved in mucosal immunity.

In the present invention, "adjuvant" means a substance that is administered at the same time or sequentially with an antigen or an allergen and used for enhancing immune response to the antigen or the allergen.

In the present invention, "treatment" means recovery from, remission of, alleviation of and/or delay of exacerbation of clinical symptoms of viral infection, allergy disease, bacterial infection and bacterium-derived toxin, cancer, or a disease caused by intracellular parasitic protozoa in a patient having the disease.

In the present invention, "prevention" means reduction in incidence rate of viral infection, allergy disease, bacterial infection and bacterium-derived toxin, cancer, or a disease caused by intracellular parasitic protozoa. Prevention includes reduction in risk of progression of viral infection, allergy disease, bacterial infection and bacterium-derived toxin, cancer, or a disease caused by intracellular parasitic protozoan, or reduction in worsening of the disease. The present invention induces protective immune response in humans and is therefore effective for the prevention of the disease.

The compound represented by the general formula (I) of the present invention or the pharmaceutically acceptable salt thereof can be administered in various forms. Examples of the dosage form include tablets, capsules, granules, emulsions, pills, powders, and syrups (solutions) for oral administration and injections (intravenous, intramuscular, subcutaneous, or intraperitoneal administration), drip infusions, and suppositories (rectal administration) for parenteral administration. These various preparations can be formulated in accordance with routine methods using aids that may be conventionally used in the field of pharmaceutical formulation techniques such as excipients, binders, disintegrants, lubricants, corrigents, solubilizers, suspending agents, and coating agents, in addition to the active ingredient.

For use as a tablet, examples of carriers that can be used include: excipients such as lactose, saccharose, sodium chloride, glucose, urea, starch, calcium carbonate, kaolin, crystalline cellulose, and silicic acid; binders such as water, ethanol, propanol, simple syrup, glucose solutions, starch solutions, gelatin solutions, carboxymethylcellulose, shellac, methylcellulose, potassium phosphate, and polyvinylpyrrolidone; disintegrants such as dry starch, sodium alginate, agar powder, laminaran powder, sodium bicarbonate, calcium carbonate, polyoxyethylene sorbitan fatty acid esters, sodium lauryl sulfate, monoglyceride stearate, starch, and lactose; disintegration inhibitors such as saccharose, stearin, cocoa butter, and hydrogenated oil; absorption promoters such as quaternary ammonium salts and sodium lauryl sulfate; moisturizing agents such as glycerin and starch; adsorbents such as starch, lactose, kaolin, bentonite, and colloidal silicic acid; and lubricants such as purified talc, stearate, boric acid powder, and polyethylene glycol. Alternatively, tablets coated in a conventional manner, for example, sugar coated tablets, gelatin coated tablets, enteric coated tablets, film coated tablets, double layer tablets, and multilayered tablets may be prepared, if necessary.

For use as a pill, examples of carriers that can be used include: excipients such as glucose, lactose, cocoa butter, starch, hydrogenated plant oil, kaolin, and talc; binders such as gum arabic powder, powdered tragacanth, gelatin, and ethanol; and disintegrants such as laminaran and agar.

For use as a suppository, conventional carriers known in the art can be widely used. Examples thereof include polyethylene glycol, cocoa butter, higher alcohols, esters of higher alcohols, gelatin, and semisynthetic glyceride.

For use as an injection or a sublingual liquid, solutions, emulsions, or suspensions can be used. These solutions, emulsions, or suspensions are preferably sterilized and adjusted to be isotonic to blood. Any solvent that can be used as a medical diluent can be used without limitations in the production of these solutions, emulsions, or suspensions. Examples thereof include water, ethanol, propylene glycol, ethoxylated isostearyl alcohol, polyoxylated isostearyl alcohol, and polyoxyethylene sorbitan fatty acid esters. In this case, each preparation may contain common salt, glucose, or glycerin in an amount sufficient for preparing an isotonic solution. Also, each preparation may contain a conventional solubilizer, buffer, soothing agent, and the like.

These preparations may also contain a colorant, a preservative, a fragrance, a flavor, a sweetener, and the like, if necessary, and may further contain an additional pharmaceutical product.

The amount of the compound contained in each of these preparations is not particularly limited and is appropriately selected in a wide range. The composition usually contains 0.5 to 70% by weight, preferably 1 to 30% by weight of the compound based on the total weight.

The amount of the compound used differs depending on the symptoms, age, etc. of the patient (warm-blooded animal, particularly, a human). The daily dose for oral administration to an adult human is 10 mg (preferably 1 mg) as the upper limit and 0.001 mg as the lower limit and is desirably administered 0 to 3 times a day according to the symptoms.

Next, a typical method for producing the compound represented by the general formula (I) will be described. The compound of the present invention can be produced by various production methods. The production method shown below is given for illustrative purposes. It should be understood that the present invention is not limited by this example.

The compound represented by the general formula (I) of the present invention or a pharmaceutically acceptable salt thereof can be produced by use of various production methods known in the art through the use of features based on the type of its backbone or a substituent. The methods known in the art are methods described in, for example, "Organic Functional Group Preparations", 2nd ed., Academic Press, Inc., 1989 and "Comprehensive Organic Transformations", VCH Publishers Inc., 1989.

Depending on the type of functional group present in the compound, the functional group in a starting material or an intermediate may be protected with an appropriate protective group, or may be replaced with a group that can be readily converted to the functional group. Such an approach may be effective for the production technique.

Examples of such a functional group include an amino group, a hydroxy group, and a carboxy group. Examples of their protective groups include protective groups described in T.W. Greene and P.G. Wuts, "Protective Groups in Organic Synthesis (4th ed., John Wiley & Sons, Inc., 2006)"

The protective group or the group that can be readily converted to the functional group can be appropriately selected for use according to the reaction conditions of each production method for compound production.

According to such a method, reaction can be carried out after introduction of the group, followed by the removal of the protective group or the conversion to the desired group according to the need to obtain the desired compound.

The prodrug of the compound can be produced by, as in the protective group mentioned above, introducing a particular group into a starting material or an intermediate, or carrying out the reaction using the obtained compound. The reaction for producing the prodrug can be carried out by use of a method generally known to those skilled in the art such as conventional esterification, amidation, dehydration, or hydrogenation.

Hereinafter, a compound number shown in each reaction formula is used in order to indicate a compound. Specifically, a compound is referred to as "(1)", etc. The same applies to compounds of the other numbers.

In methods A to C given below, X, Y, and n in each formula are as defined above.

Abbreviations used in this paragraph, Examples and tables have the following meaning.

Ac: acetyl group, Bn: benzyl group, Boc: tert-butoxycarbonyl group, Cbz: benzyloxycarbonyl group, $CDCl_3$: deuterated chloroform, $CD_3OD$: deuterated methanol, $D_2O$: heavy water, DMSO: dimethyl sulfoxide, TBDPS: tert-butyldiphenylsilyl group, TES: triethylsilyl group, R: (3R)-3-(decanoyloxy) tetradecanoyl, and R': (3R)-3-(decyloxy) tetradecanoyl.

Method A

The compound represented by (1) of the present invention or the pharmaceutically acceptable salt thereof can be produced in accordance with method A described below.

[Formula 7]

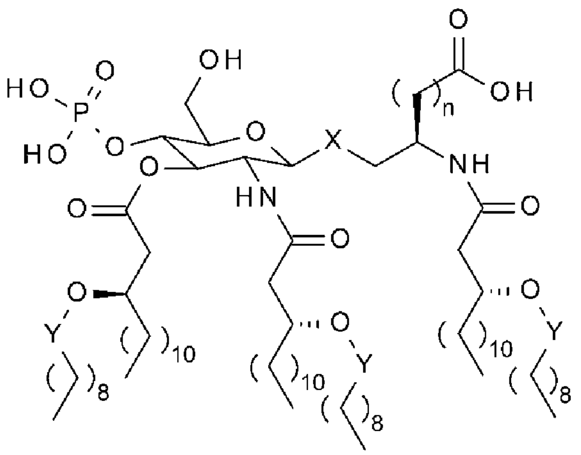

(1)

[Formula 8]

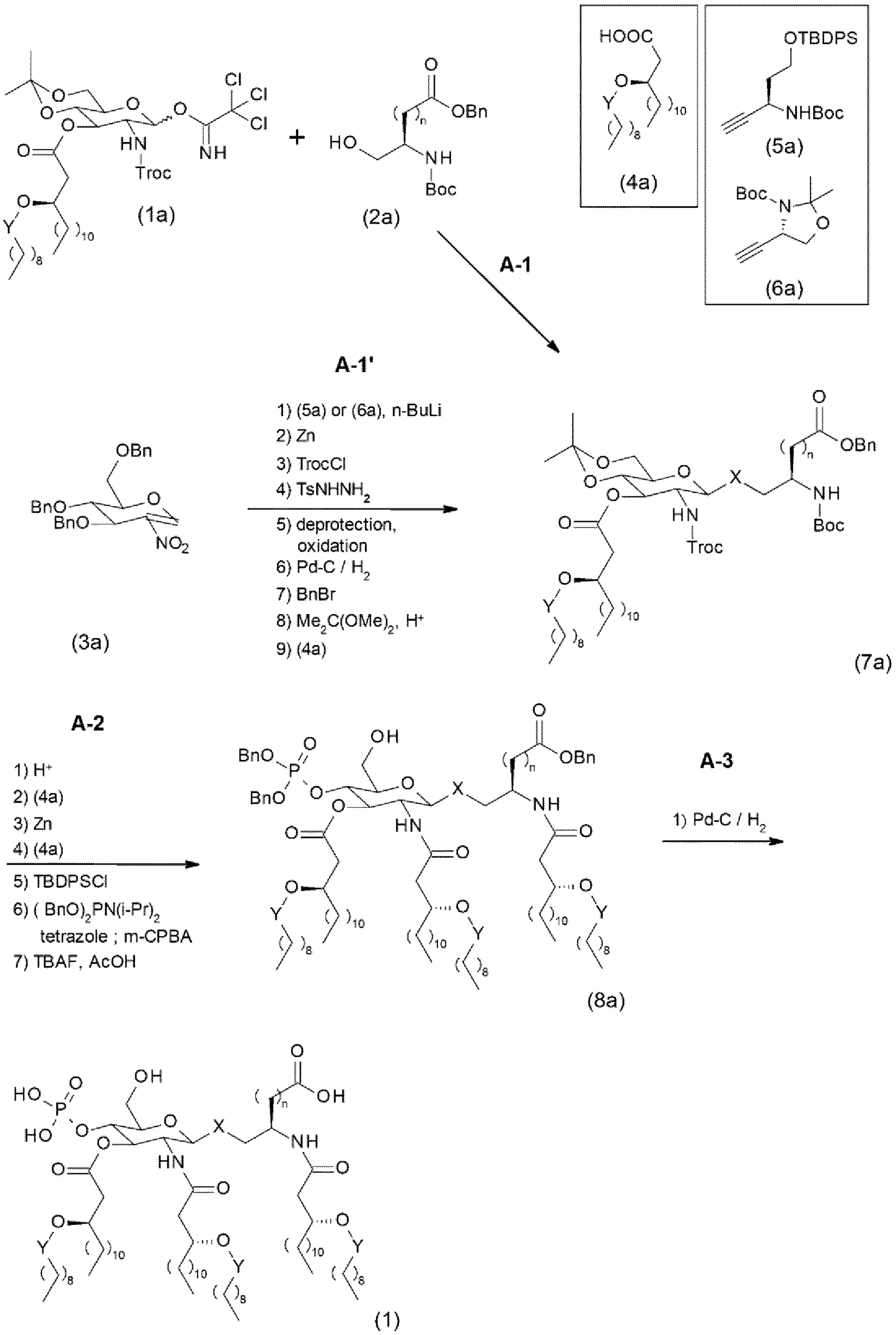


(Step A-1)

This step is the step of subjecting (1a) to glycosylation with (2a) using a Lewis acid under ice cooling to produce (7a), when X is an oxygen atom. A preferred starting material for synthesizing (1a) is allyl 2-deoxy-4,6-O-(1-methylethylidene)-2-{[(2,2,2-trichloroethoxy)carbonyl]amino}-α-D-glucopyranoside which can be prepared from glucosamine hydrochloride by use of procedures described in the report from Imoto et al. (Tetrahedron Lett. 1985, 26, 1545-1548).

(Step A-1')

This step is the step of producing (7a) from (3a) through a carbon-carbon bond formation reaction with alkyne (5a) or (6a), when X is a carbon atom. C-Glycosyl alkyne is formed by the coupling of a lithiated alkyne and (3a) under a condition of low temperature, followed by the reduction of the nitro group, the protection of the primary amine, and the reduction of the alkyne under a heating condition to synthesize C-glycosyl amino acid. In the case of using alkyne (5a), the protective group on the hydroxy group is deprotected, and the resulting hydroxy group is then converted to an aldehyde by Dess-Martin oxidation, which is then converted to a carboxylic acid by Pinnick oxidation. In the case of using alkyne (6a), the deprotection and oxidation reaction of the acetal group are performed at the same time using Jones reagent to prepare a carboxylic acid. Next, the benzyl protective group is deprotected by hydrogenation, followed by the protection of the carboxylic acid with a benzyl group under a heating condition, the acetal protection of the hydroxy groups at the 4- and 6-positions of glucosamine, and the acylation of the hydroxy group at the 3-position using (4a) to synthesize (7a) .

(Step A-2)

This step is the step of producing (8a) from (7a) by deprotection, acylation with (4a), and phosphorylation at the 4-position. The acetal group of (7a) is deprotected with a mixed solvent of acetic acid and water under a heating condition, and the Boc protective group on the primary amine is removed by acid treatment, followed by the amidation of the resulting primary amine with (4a). Next, the Troc protective group is removed through reduction reaction, followed by the amidation of the primary amine with (4a). The hydroxy group at the 6-position of glucosamine is temporarily protected with a silyl protective group, and the hydroxy group at the 4-position is then phosphorylated. Subsequently, the silyl protective group is deprotected to synthesize (8a).

(Step A-3)

This step is the step of deprotecting the benzyl protective group of (8a) by hydrogenation to produce (1).

Method B

The compound represented by (2) of the present invention or the pharmaceutically acceptable salt thereof can be produced in accordance with method B described below.

[Formula 9]

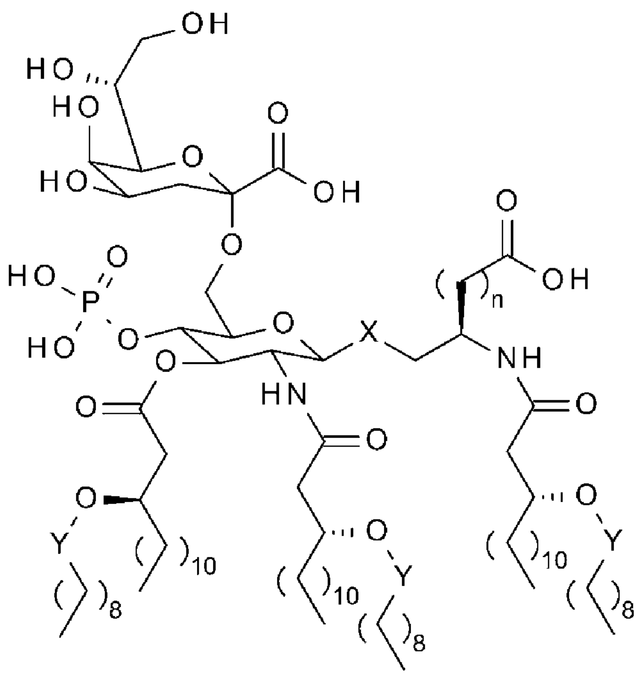

(2)

The step of producing (2) from (2a) can be performed in the same manner as in A-3 of method A.

[Formula 10]

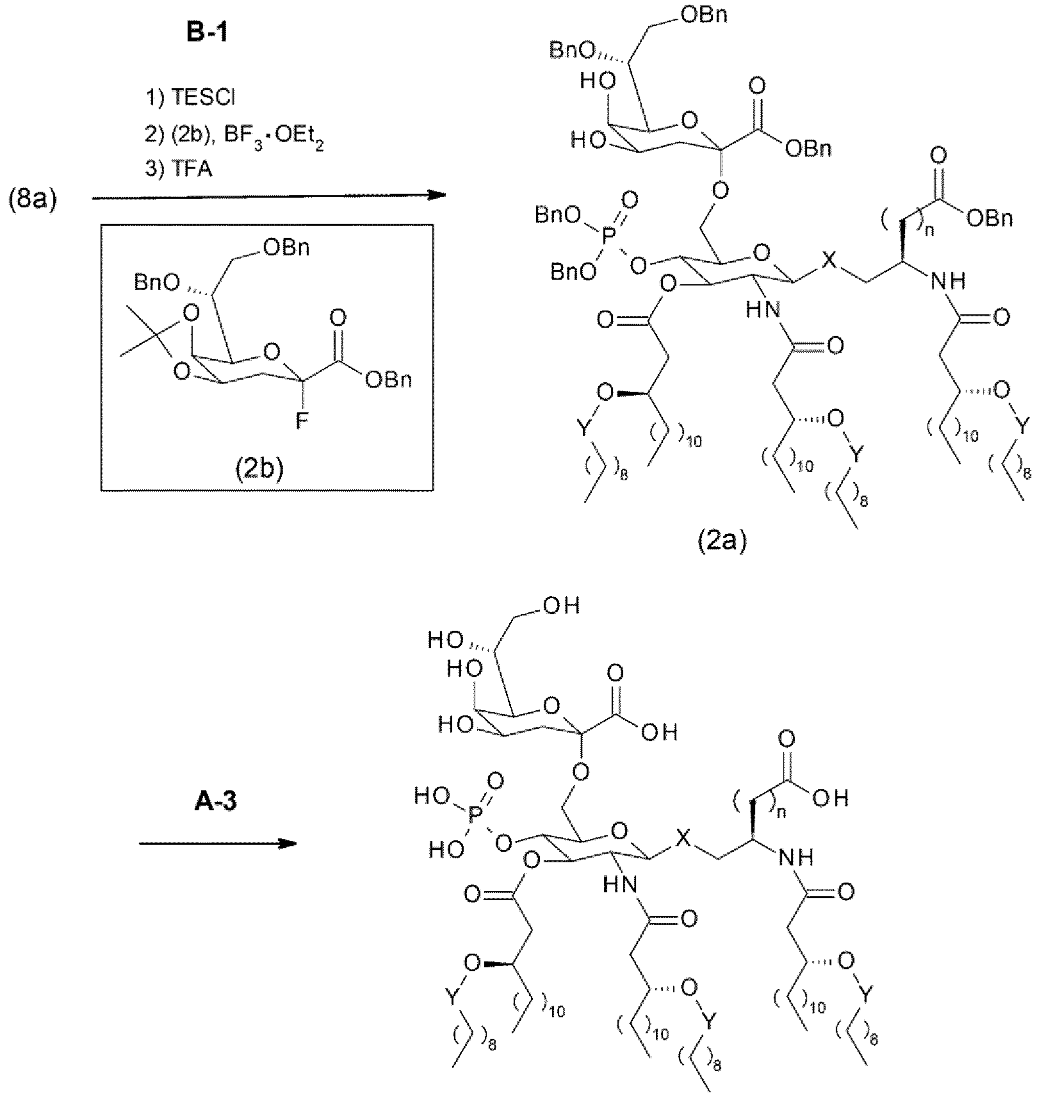


(8a)

(2b)

(2a)

(2)

(Step B-1)

This step is the step of producing (2a) by the glycosylation of (8a) obtained in method A and a KDO (2-keto-3-deoxyoctulosonic acid) unit (2b). The hydroxy group at the 6-position of the glucosamine of (8a) is protected with TES, and a coupling reaction with (2b) is performed using a Lewis acid under ice cooling, followed by the deprotection of the acetal protective group by acid treatment to synthesize (2a).

Method C

The compound represented by (3) of the present invention or the pharmaceutically acceptable salt thereof can be produced in accordance with method C described below.

[Formula 11]

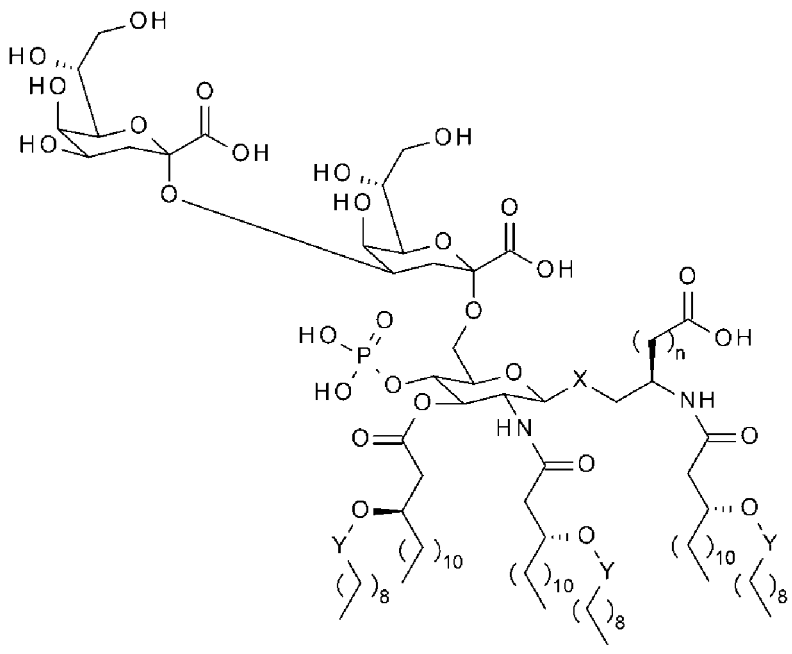

(3)

The step of producing (3) from (3a) can be performed in the same manner as in A-3 of method A.

[Formula 12]

(2a)

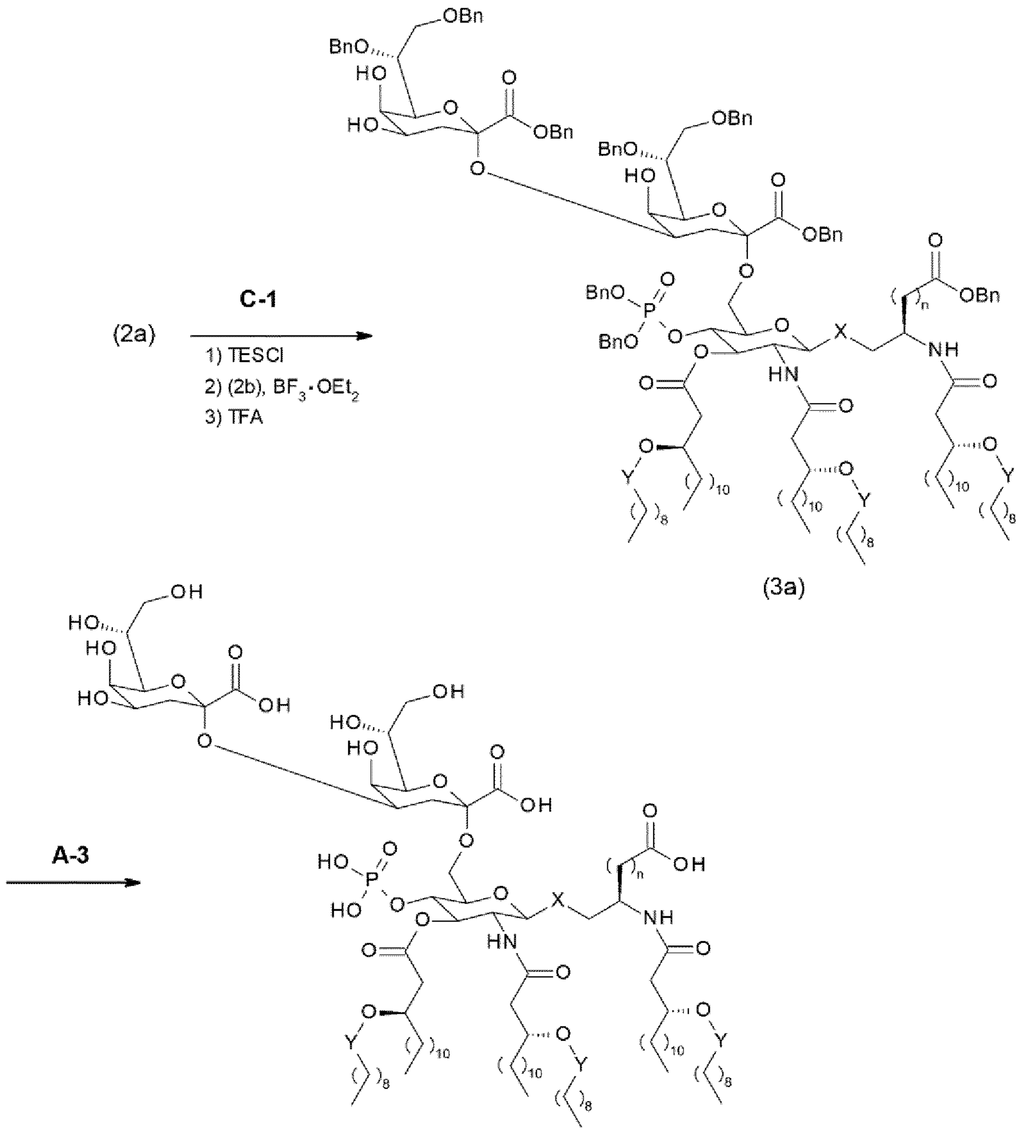


(3a)

(3)

(Step C-1)

This step is the step of producing (3a) by the glycosylation of (2a) obtained in method B and a KDO unit (2b). The hydroxy group at the 4-position of the KDO unit of (2a) is protected with TES, and a coupling reaction with (2b) is performed using a Lewis acid under ice cooling, followed by the deprotection of the acetal protective group by acid treatment to synthesize (3a).

In the methods A to C described above, the acetal protective group is preferably a dimethylacetal group and may be a benzylidene acetal group or the like. The acetal protective groups for two hydroxy groups may be protective groups independent from each other. The protective group on the hydroxy group is preferably a benzyl group or a tert-butyldiphenylsilyl group and may be a tert-butyldimethylsilyl group, an allyl group, a benzyloxycarbonyl group, or the like. The protective group on the primary amine is preferably a 2,2,2-trichloroethylcarbonyl group or a tert-butyloxycarbonyl group and may be an allyloxycarbonyl group, a benzyloxycarbonyl group, a 9-fluorenylmethyloxycarbonyl group, or the like. The protective group on the phosphoric acid or the carboxylic acid is preferably a benzyl group and may be an allyl group, a tert-butyl group, a phenyl group, or the like. The low-temperature condition is -100 to -20°C, preferably -80 to -50°C. The ice cooling is -20 to 10°C, preferably -10 to 5°C. The heating condition is 35 to 130°C, preferably 50°C to

100°C. A temperature condition that is not described is -10 to 100°C, preferably 15°C to 35°C.

Examples

Hereinafter, the present invention will be described in more detail with reference to the Examples. However, the scope of the present invention is not limited by these examples, and these examples are not construed in a limited manner by any means. In the present specification, reagents, solvents and starting materials are readily available from commercial suppliers, unless otherwise specified.

Proton nuclear magnetic resonance spectra ($^1$H-NMR) were measured using a 400 MHz nuclear magnetic resonance apparatus manufactured by JEOL Ltd., a 400 MHz nuclear magnetic resonance apparatus manufactured by Varian, Inc., or a 500 MHz nuclear magnetic resonance apparatus manufactured by Varian, Inc. Spectral data were indicated by chemical shifts (which were indicated by relative ppm (δ) with tetramethylsilane as a standard substance), the number of protons, multiplicity of peak splitting (which was indicated by s: singlet; d: doublet; t: triplet; q: quadruplet; m: multiplet; br: broad, etc.), and, if expressed, J values (unit: Hz) as spin coupling constants.

Mass spectra (MS m/z) were measured by electrospray ionization (ESI) .

Silica gel column chromatography was performed using a commercially available packed column and automatic preparative separation and purification apparatus (Isorela One manufactured by Biotage Japan Ltd., EPCLC-W-Prep2XY manufactured by Yamazen Corp., Purif-α2 manufactured by Shoko Science Co., Ltd., etc.), and only a plurality of solvent species used in a mobile phase were described. Elution was performed under observation by thin layer chromatography (TLC) which adopted silica gel 60 $F_{254}$ or 60 $NH_2$ $F_{254}$s manufactured by Merck KGaA, $NH_2$ silica gel 60 $F_{254}$ plate manufactured by Wako Pure Chemical Industries, Ltd. or CHROMATOREX NH TLC manufactured by Fuji Silysia Chemical Ltd. as a TLC plate, the mobile phase used in column chromatography as a developing solvent, and a UV detector or a chromogenic reagent as a detection method.

(Example 1) Ammonium (3R)-3-{[(3R)-3-(decanoyloxy)tetradecanoyl]amino}-4-({3-O-[(3R)-3-(decanoyloxy)tetradecanoyl]-2-{ [(3R)-3-(decanoyloxy)tetradecanoyl]amino}-2-deoxy-4-O-phosphono-β-D-glucopyranosyl}oxy)butanoate

[Formula 13]

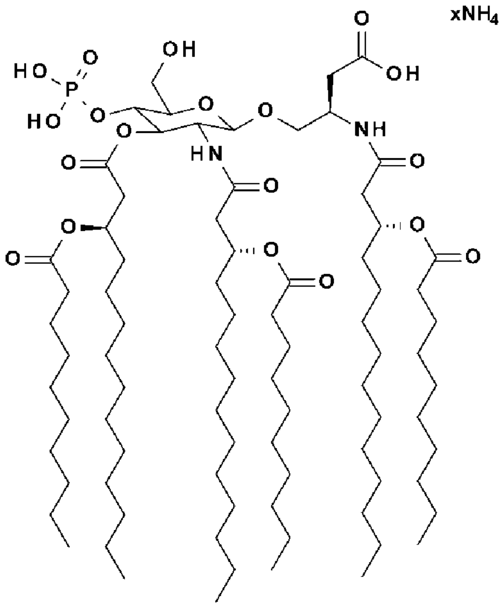

(1a) Allyl 3-O-[(3R)-3-(decanoyloxy)tetradecanoyl]-2-deoxy-4,6-O-(1-methylethylidene)-2-{[(2,2,2-trichloroethoxy)carbonyl]amino}-α-D-glucopyranoside

To a solution of allyl 2-deoxy-4,6-O-(1-methylethylidene)-2-{[(2,2,2-trichloroethoxy)carbonyl]amino}-α-D-glucopyranoside (7.70 g) (Tetrahedron Letters 1985, 26 (12), 1545-1548) in dichloromethane (80 mL), (3R)-3-(decanoyloxy)tetradecanoic acid (6.0 g) (Tetrahedron Letters 2006, 47 (13), 2087-2092), 4-dimethylaminopyridine (55.5 mg), and 1-ethyl-3-(3-dimethylaminopropyl)carbodiimide hydrochloride (3.49 g) were added at room temperature, and the mixture was stirred overnight at the same temperature . The reaction was terminated by the addition of a saturated aqueous solution of sodium bicarbonate to the reaction mixture, followed by extraction with ethyl acetate. The organic layer was washed with saturated saline and then dried over anhydrous sodium sulfate. The drying agent was filtered off, and the filtrate was concentrated under reduced pressure. The residue was purified by silica gel column chromatography [n-hexane/ethyl acetate] to obtain the title compound (9.51 g).

(1b) 3-O-[(3R)-3-(Decanoyloxy)tetradecanoyl]-2-deoxy-4,6-O-(1-methylethylidene)-2-{[(2,2,2-trichloroethoxy)carbonyl]amino}-D-glucopyranose

To a solution of the compound (5.47 g) obtained in Example 1(1a) in tetrahydrofuran (50 mL), 1,5-cyclooctadienebis(methyldiphenylphosphine)iridium(I) hexafluorophosphate (284 mg) was added at room temperature, and the mixture was stirred at the same temperature for 1 minute under a hydrogen atmosphere. After further stirring for 2 hours under a nitrogen atmosphere, water (10 mL), pyridine (1.6 mL), and iodine (3.41 g) were added at the same temperature, and the mixture was stirred for 2 hours. The reaction was terminated by the addition of a 5% aqueous sodium thiosulfate solution to the reaction mixture, followed by extraction with ethyl acetate. The organic layer was washed with saturated saline and then dried over anhydrous sodium sulfate. The drying agent was filtered off, and the filtrate was concentrated under reduced pressure. The residue was purified by silica gel column chromatography [n-hexane/ethyl acetate] to obtain the title compound (1.89 g).

(1c) Benzyl (3R)-3-[(tert-butoxycarbonyl)amino]-4-[(3-O-[(3R)-3-(decanoyloxy)tetradecanoyl]-2-deoxy-4,6-O-(1-methylethylidene)-2-{ [(2,2,2-trichloroethoxy)carbonyl]amino}-β-D-glucopyranosyl)oxy]butanoate

To a solution of the compound (930 mg) obtained in Example 1(1b) in dichloromethane (10 mL), trichloroacetonitrile (1.2 mL) and 1,8-diazabicyclo[5.4.0]undec-7-ene (0.036 mL) were added at 0°C, and the mixture was stirred at the same temperature for 1 hour. After concentration of the reaction mixture, the residue was purified by silica gel column chromatography [n-hexane/ethyl acetate] to obtain a product (1.07 g). To a solution of the product in dichloromethane (10 mL), benzyl (3R)-3-[(tert-butoxycarbonyl)amino]-4-hydroxybutanoate (500 mg) and molecular sieve 4A, 1/16 (300 mg) were added at room temperature, and the mixture was stirred at the same temperature for 20 minutes. Then, trimethylsilyl trifluoromethanesulfonate (0.021 mL) was added at 0°C, and the mixture was stirred at the same temperature for 3 hours. The reaction was terminated by the addition of triethylamine. Then, the molecular sieve was filtered off, and the filtrate was concentrated under reduced pressure. The residue was purified by silica gel column chromatography [n-hexane/ethyl acetate] to obtain the title compound (370 mg).

(1d) Benzyl (3R)-3-amino-4-[(3-0-[(3R)-3-(decanoyloxy)tetradecanoyl]-2-deoxy-2-{[(2,2,2-trichloroethoxy)carbonyl]amino}-β-D-glucopyranosyl)oxy]butanoate

To a solution of the compound (370 mg) obtained in Example 1(1c) in acetic acid (10 mL), water (1 mL) was added at room temperature, and the mixture was stirred at 60°C for 2 hours. The reaction mixture was concentrated to obtain a product (350 mg). This product was combined with a product (310 mg) obtained in the same manner as above, and the resulting product (660 mg) was dissolved in dichloromethane (20 mL). To the solution, trifluoroacetic acid (4 mL) was added at room temperature, and the mixture was stirred at the same temperature for 1 hour. The reaction was terminated by the addition of a saturated aqueous solution of sodium bicarbonate to the reaction mixture, followed by extraction with ethyl acetate. The organic layer was washed with saturated saline and then dried over anhydrous sodium sulfate. The drying agent was filtered off, and the filtrate was concentrated under reduced pressure. The residue was purified by silica gel column chromatography [ethyl acetate/methanol] to obtain the title compound (470 mg).

(1e) Benzyl (3R)-3-{[(3R)-3-(decanoyloxy)tetradecanoyl]amino}-4-[(3-O-[(3R)-3-(decanoyloxy)tetradecanoyl]-2-deoxy-2-{[(2,2,2-trichloroethoxy)carbonyl]amino}-β-D-glucopyranosyl)oxy]butanoate

To a solution of the compound (470 mg) obtained in Example 1(1d) in tetrahydrofuran-methanol (1:1, 8 mL), (3R)-3-(decanoyloxy)tetradecanoic acid (400 mg) and 4-(4,6-dimethoxy-1,3,5-triazin-2-yl)-4-methylmorpholinium chloride (420 mg) were added at room temperature, and the mixture was stirred overnight at the same temperature . The reaction was terminated by the addition of 0.5 N hydrochloric acid to the reaction mixture, followed by extraction with ethyl acetate. The organic layer was washed with saturated saline and then dried over anhydrous sodium sulfate. The drying agent was filtered off, and the filtrate was concentrated under reduced pressure. The residue was purified by

silica gel column chromatography [n-hexane/ethyl acetate/dichloromethane] to obtain the title compound (520 mg).

(1f) Benzyl (3R)-4-({2-amino-3-O-[(3R)-3-(decanoyloxy)tetradecanoyl]-2-deoxy-β-D-glucopyranosyl}oxy)-3-{[(3R)-3-(decanoyloxy)tetradecanoyl]amino}butanoate

To a solution of the compound (520 mg) obtained in Example 1(1e) in tetrahydrofuran (5 mL), acetic acid (10 mL) and a zinc powder (520 mg) were added at room temperature, and the mixture was stirred at the same temperature for 1 hour. Zinc was filtered off, and the filtrate was concentrated under reduced pressure. The residue was purified by silica gel column chromatography [ethyl acetate/methanol] to obtain the title compound (450 mg).

(1g) Benzyl (3R)-3-{[(3R)-3-(decanoyloxy)tetradecanoyl]amino}-4-[(3-O-[(3R)-3-(decanoyloxy)tetradecanoyl]-2-{[(3R)-3-(decanoyloxy)tetradecanoyl]amino}-2-deoxy-β-D-glucopyranosyl)oxy]butanoate

To a solution of the compound (450 mg) obtained in Example 1(1f) in tetrahydrofuran-methanol (1:1, 8 mL), (3R)-3-(decanoyloxy)tetradecanoic acid (317 mg) and 4-(4,6-dimethoxy-1,3,5-triazin-2-yl)-4-methylmorpholinium chloride (330 mg) were added at room temperature, and the mixture was stirred overnight at the same temperature. The reaction was terminated by the addition of 0.5 N hydrochloric acid to the reaction mixture, followed by extraction with ethyl acetate. The organic layer was washed with saturated saline and then dried over anhydrous sodium sulfate. The drying agent was filtered off, and the filtrate was concentrated under reduced pressure. The residue was purified by silica gel column chromatography [n-hexane/ethyl acetate] to obtain the title compound (476 mg).

(1h) Benzyl (3R)-4-[(6-O-[tert-butyl(diphenyl)silyl]-3-O-[(3R)-3-(decanoyloxy)tetradecanoyl]-2-{[(3R)-3-(decanoyloxy)tetradecanoyl]amino}-2-deoxy-β-D-glucopyranosyl)oxy]-3-{[(3R)-3-(decanoyloxy)tetradecanoyl]amino}butanoate

To a solution of the compound (1.90 g) obtained in Example 1(1g) in dichloromethane (20 mL), tert-butyldiphenylchlorosilane (410 mg) and imidazole (21 mg) were added at room temperature, and the mixture was stirred at the same temperature for 2 hours. The reaction was terminated by the addition of a saturated aqueous solution of sodium bicarbonate to the reaction mixture, followed by extraction with ethyl acetate. The organic layer was washed with saturated saline and then dried over anhydrous sodium sulfate. The drying agent was filtered off, and the filtrate was concentrated under reduced pressure. The residue was purified by silica gel column chromatography [n-hexane/ethyl acetate] to obtain the title compound (2.08 g).

(1i) Benzyl (3R)-4-[(4-O-[bis(benzyloxy)phosphoryl]-6-O-[tert-butyl(diphenyl)silyl]-3-O-[(3R)-3-(decanoyloxy)tetradecanoyl]-2-{[(3R)-3-(decanoyloxy)tetradecanoyl]amino}-2-deoxy-(3-D-glucopyranosyl)oxy]-3-{[(3R)-3-(decanoyloxy)tetradecanoyl]amino}butanoate

To a solution of the compound (2.08 g) obtained in Example 1(1h) in dichloromethane (25 mL), dibenzyl N,N-diisopropylphosphoramidite (0.626 mL) and 1H-tetrazole (166 mg) were added at room temperature, and the mixture was stirred at the same temperature for 1 hour. 3-Chloroperbenzoic acid (400 mg) was added at 0°C, and the mixture was stirred at the same temperature for 15 minutes. The reaction was terminated by the addition of a 5% aqueous sodium thiosulfate solution and a saturated aqueous solution of sodium bicarbonate to the reaction mixture, followed by extraction with ethyl acetate. The organic layer was washed with saturated saline and then dried over anhydrous sodium sulfate. The drying agent was filtered off, and the filtrate was concentrated under reduced pressure. The residue was purified by silica gel column chromatography [n-hexane/ethyl acetate] to obtain the title compound (2.39 g).

(1j) Benzyl (3R)-4-[(4-O-[bis(benzyloxy)phosphoryl]-3-O-[(3R)-3-(decanoyloxy)tetradecanoyl]-2-{[(3R)-3-(decanoyloxy)tetradecanoyl]amino}-2-deoxy-β-D-glucopyranosyl)oxy]-3-{[(3R)-3-(decanoyloxy)tetradecanoyl]amino}butanoate

To a solution of the compound (2.39 g) obtained in Example 1(1i) in tetrahydrofuran (20 mL), acetic acid (0.34 mL) and a 1 M solution of tetrabutylammonium fluoride in tetrahydrofuran (5.94 mL) were added at room temperature, and the mixture was stirred overnight at the same temperature. The reaction was terminated by the addition of water to the reaction mixture, followed by extraction with ethyl acetate. The organic layer was washed with saturated saline and then dried over anhydrous sodium sulfate. The drying agent was filtered off, and the filtrate was concentrated under reduced pressure. The residue was purified by silica gel column chromatography [n-hexane/ethyl acetate] to obtain the title compound (1.90 g).

(1k) Bisammonium (3R)-3-{[(3R)-3-(decanoyloxy)tetradecanoyl]amino}-4-({3-O-[(3R)-3-(decanoyloxy)tetradecanoyl]-2-{[(3R)-3-(decanoyloxy)tetradecanoyl]amino}-2-deoxy-4-O-phosphono-P-D-glucopyranosyl}oxy)butanoate

To a solution of the compound (88.9 mg) obtained in Example 1(1j) in tetrahydrofuran (3 mL), 10% palladium on carbon (60.0 mg) was added at room temperature, and the mixture was stirred at the same temperature for 8 hours under a hydrogen atmosphere. Palladium catalyst was filtered off, and the filtrate was then concentrated under reduced pressure. To a solution of the residue in tetrahydrofuran (10 mL), a 4% solution of ammonia in methanol (0.25 mL) was added at -78°C, and the mixture was concentrated under reduced pressure at room temperature. The residue was washed with acetonitrile and collected by filtration to obtain the title compound (57.1 mg).

(Example 2) Ammonium (3R)-3-{[(3R)-3-(decanoyloxy)tetradecanoyl]amino}-4-({3-O-[(3R)-3-(decanoyloxy)tetradecanoyl]-2-{[(3R)-3-(decanoyloxy)tetradecanoyl]amino}-2-deoxy-6-O-(3-deoxy-α-D-manno-oct-2-ulopyranonosyl)-4-O-phosphono-β-D-glucopyranosyl}oxy)butanoate

[Formula 14]

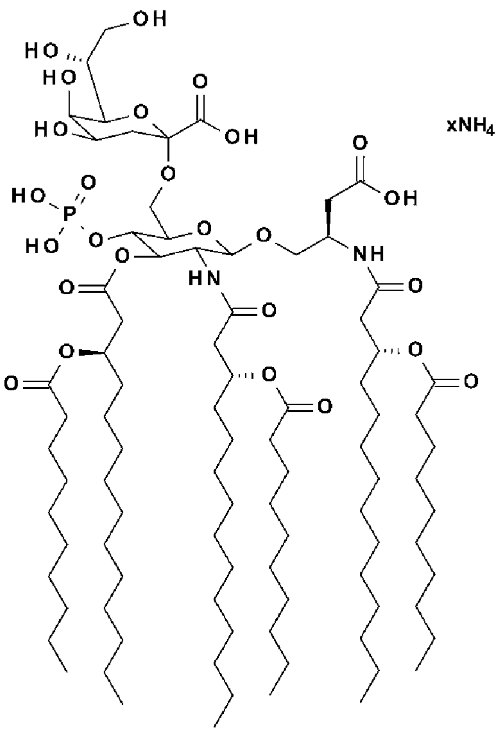

(2a) Benzyl (3R)-4-({4-O-[bis(benzyloxy)phosphoryl]-3-O-[(3R)-3-(decanoyloxy)tetradecanoyl]-2-{[(3R)-3-(decanoyloxy)tetradecanoyl]amino}-2-deoxy-6-O-(triethylsilyl)-β-D-glucopyranosyl}oxy)-3-{[(3R)-3-(decanoyloxy)tetradecanoyl]amino}butanoate

To a solution of the compound (394 mg) obtained in Example 1(1j) in dichloromethane (5 mL), triethylamine (0.12 mL), 4-dimethylaminopyridine (27.2 mg), and trichloroethylsilane (0.049 mL) were added at room temperature, and the mixture was stirred at the same temperature for 1 hour. The reaction mixture was purified by silica gel column chromatography [n-hexane/ethyl acetate] to obtain the title compound (384 mg).

(2b) Benzyl (3R)-4-[(6-O-[1-benzyl-7,8-di-O-benzyl-3-deoxy-4,5-O-(1-methylethylidene)-α-D-manno-oct-2-ulopyranonosyl]-4-O-[bis(benzyloxy)phosphoryl]-3-O-[(3R)-3-(decanoyloxy)tetradecanoyl]-2-{ [(3R)-3-(decanoyloxy)tetradecanoyl]amino}-2-deoxy-β-D-glucopyranosyl)oxy]-3-{ [(3R)-3-(decanoyloxy)tetradecanoyl]amino}butanoate

To a solution of the compound (384 mg) obtained in Example 2(2a) in dichloromethane (4 mL), benzyl

(3aR,4R,6S,7aR)-4-[(1R)-1,2-bis(benzyloxy)ethyl]-6-fluoro-2,2-dimethyltetrahydro-2H,4H-[1,3]dioxolo[4,5-c]pyran-6-carboxylate (336 mg) (Angewandte Chemie, International Edition 2001, 40, 1475-1480) and molecular sieve 5A, 1/16 (1.0 g) were added, and the mixture was stirred at the temperature for 15 minutes. A boron trifluoride-diethyl ether complex (0.255 mL) was added to the reaction mixture at 0°C, and the mixture was stirred at the same temperature for 20 minutes. The reaction was terminated by the addition of triethylamine. Then, the molecular sieve was filtered off, and the filtrate was concentrated under reduced pressure. The residue was purified by silica gel column chromatography [n-hexane/ethyl acetate] to obtain the title compound (267 mg).

(2c) Benzyl (3R)-4-{[6-O-(1-benzyl-7,8-di-O-benzyl-3-deoxy-α-D-manno-oct-2-ulopyranonosyl)-4-O-[bis(benzyloxy)phosphoryl]-3-O-[(3R)-3-(decanoyloxy)tetradecanoyl]-2-{[(3R)-3-(decanoyloxy)tetradecanoyl]amino}-2-deoxy-β-D-glucopyranosyl]oxy}-3-{[(3R)-3-(decanoyloxy)tetradecanoyl]amino}butanoate

To a solution of the compound (267 mg) obtained in Example 2(2b) in dichloromethane (10 mL), water (0.48 mL) and trifluoroacetic acid (0.72 mL) were added at room temperature, and the mixture was stirred at the same temperature for 30 minutes. The reaction was terminated by the addition of a saturated aqueous solution of sodium bicarbonate to the reaction mixture, followed by extraction with ethyl acetate. The organic layer was washed with saturated saline and then dried over anhydrous sodium sulfate. The drying agent was filtered off, and the filtrate was concentrated under reduced pressure. The residue was purified by silica gel column chromatography [n-hexane/ethyl acetate] to obtain the title compound (237 mg).

(2d) (3R)-3-{[(3R)-3-(Decanoyloxy)tetradecanoyl]amino}-4-({3-O-[(3R)-3-(decanoyloxy)tetradecanoyl]-2-{[(3R)-3-(decanoyloxy)tetradecanoyl]amino}-2-deoxy-6-O-(3-deoxy-α-D-manno-oct-2-ulopyranonosyl)-4-O-phosphono-β-D-glucopyranosyl}oxy)butanoic acid

To a solution of the compound (600 mg) obtained in Example 2(2c) in tetrahydrofuran (12 mL), 10% palladium on carbon (360 mg) was added at room temperature, and the mixture was stirred at the same temperature for 7 hours under a hydrogen atmosphere. Palladium catalyst was filtered off, and the filtrate was then concentrated under reduced pressure to obtain the title compound (420 mg).

(2e) Ammonium (3R)-3-{[(3R)-3-(decanoyloxy)tetradecanoyl]amino}-4-({3-O-[(3R)-3-(decanoyloxy)tetradecanoyl]-2-{[(3R)-3-(decanoyloxy)tetradecanoyl]amino}-2-deoxy-6-O-(3-deoxy-α-D-manno-oct-2-ulopyranonosyl)-4-O-phosphono-β-D-glucopyranosyl}oxy)butanoate

To a solution of the compound (420 mg) obtained in Example 2(2d) in tetrahydrofuran (20 mL), a 4% solution of ammonia in methanol (1.2 mL) was added at -78°C, and the mixture was concentrated under reduced pressure at room temperature. The residue was washed with acetonitrile and collected by filtration to obtain the title compound (421 mg).

(Example 3) Ammonium (3R)-3-{[(3R)-3-(decanoyloxy)tetradecanoyl]amino}-4-{ [3-deoxy-α-D-manno-oct-2-ulopyranonosyl-(2→4)-3-deoxy-α-D-manno-oct-2-ulopyranonosyl-(2→6)-3-O-[(3R)-3-(decanoyloxy)tetradecanoyl]-2-{ [(3R)-3-(decanoyloxy)tetradecanoyl]amino}-2-deoxy-4-O-phosphono-P-D-glucopyranosyl]oxy}butanoate

[Formula 15]

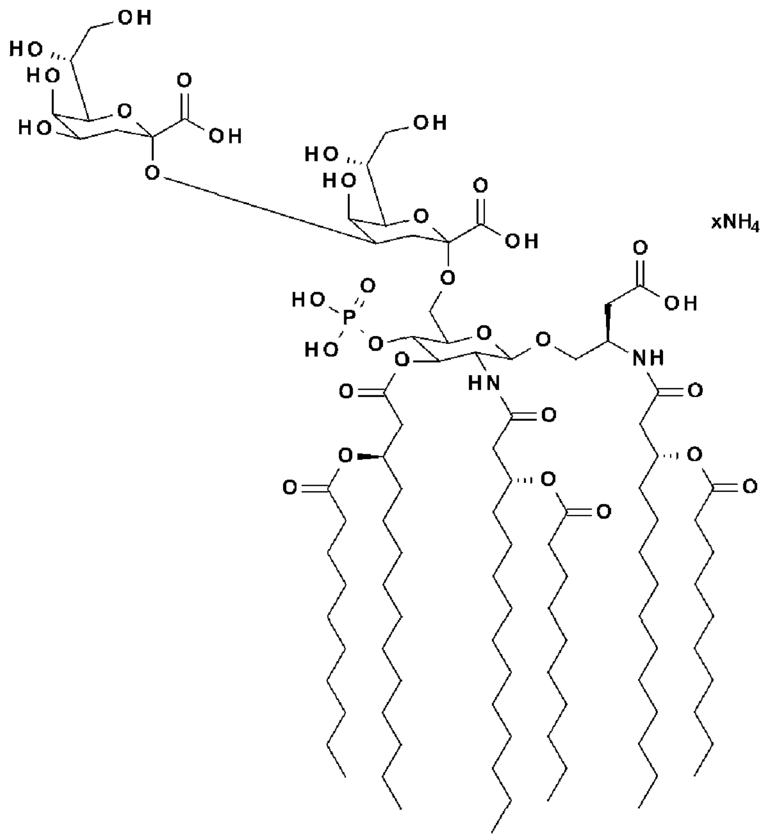

(3a) Benzyl (3R)-4-[(6-O-[1-benzyl-7,8-di-O-benzyl-3-deoxy-4-O-(triethylsilyl)-α-D-manno-oct-2-ulopyranonosyl]-4-O-[bis(benzyloxy)phosphoryl]-3-O-[(3R)-3-(decanoyloxy)tetradecanoyl]-2-{[(3R)-3-(decanoyloxy)tetradecanoyl]amino}-2-deoxy-β-D-glucopyranosyl)oxy]-3-{[(3R)-3-(decanoyloxy)tetradecanoyl]amino}butanoate

To a solution of the compound (620 mg) obtained in Example 2(2c) in dichloromethane (7 mL), triethylamine (0.38 mL), 4-dimethylaminopyridine (33.5 mg), and trichloroethylsilane (0.232 mL) were added at room temperature, and the mixture was stirred at the same temperature for 1 hour. The reaction mixture was purified by silica gel column chromatography [n-hexane/ethyl acetate] to obtain the title compound (606 mg).

(3b) Benzyl (3R)-4-{[1-benzyl-7,8-di-O-benzyl-3-deoxy-4,5-O-(1-methylethylidene)-α-D-manno-oct-2-ulopyranonosyl-(2→4)-1-benzyl-7,8-di-O-benzyl-3-deoxy-α-D-manno-oct-2-ulopyranonosyl-(2→6)-4-O-[bis(benzyloxy)phosphoryl]-3-O-[(3R)-3-(decanoyloxy)tetradecanoyl]-2-{ [(3R)-3-(decanoyloxy)tetradecanoyl]amino}-2-deoxy-β-D-glucopyranosyl]oxy}-3-{[(3R)-3-(decanoyloxy)tetradecanoyl]amino}butanoate

To a solution of the compound (606 mg) obtained in Example 3(3a) in dichloromethane (5 mL), benzyl (3aR,4R,6S,7aR)-4-[(1R)-1,2-bis(benzyloxy)ethyl]-6-fluoro-2,2-dimethyltetrahydro-2H,4H-[1,3]dioxolo[4,5-c]pyran-6-carboxylate (655 mg) and molecular sieve 5A, 1/16 (2.0 g) were added at room temperature, and the mixture was stirred at the same temperature for 20 minutes. A boron trifluoride-diethyl ether complex (0.0415 mL) was added to the reaction mixture at 0°C, and the mixture was stirred at the same temperature for 1 hour. The reaction was terminated by the addition of triethylamine. Then, the molecular sieve was filtered off, and the filtrate was concentrated under reduced pressure. The residue was purified by silica gel column chromatography [n-hexane/ethyl acetate] to obtain the title compound (383 mg).

(3c) Benzyl (3R)-4-{[1-benzyl-7,8-di-O-benzyl-3-deoxy-α-D-manno-oct-2-ulopyranonosyl-(2→4)-1-benzyl-7,8-di-O-benzyl-3-deoxy-α-D-manno-oct-2-ulopyranonosyl-(2→6)-4-O-[bis(benzyloxy)phosphoryl]-3-O-[(3R)-3-(decanoyloxy)tetradecanoyl]-2-{[(3R)-3-(decanoyloxy)tetradecanoyl]amino}-2-deoxy-β-D-glucopyranosyl]oxy}-3-{[(3R)-3-(decanoyloxy)tetradecanoyl]amino}butanoate

To a solution of the compound (484 mg) obtained in Example 3(3b) in dichloromethane (12 mL), water (0.24 mL) and trifluoroacetic acid (0.36 mL) were added at room temperature, and the mixture was stirred at the same temperature for 3 hours. The reaction was terminated by the addition of a saturated aqueous solution of sodium bicarbonate to the reaction mixture, followed by extraction with ethyl acetate. The organic layer was washed with saturated saline and then dried over anhydrous sodium sulfate. The drying agent was filtered off, and the filtrate was concentrated under reduced pressure. The residue was purified by silica gel column chromatography [n-hexane/ethyl acetate] to obtain the title compound (269 mg).

(3d) Ammonium (3R)-3-{[(3R)-3-(decanoyloxy)tetradecanoyl]amino}-4-{[3-deoxy-α-D-manno-oct-2-ulopyranonosyl-(2→4)-3-deoxy-α-D-manno-oct-2-ulopyranonosyl-(2→6)-3-O-[(3R)-3-(decanoyloxy)tetradecanoyl]-2-{[(3R)-3-(decanoyloxy)tetradecanoyl]amino}-2-deoxy-4-O-phosphono-P-D-glucopyranosyl]oxy}butanoate

The title compound (151 mg) was obtained through reaction in the same manner as in Example 1(1k) using the compound (236 mg) obtained in Example 3(3c).

(Example 4) Ammonium (2S)-2-{ [(3R)-3-(decanoyloxy)tetradecanoyl]amino}-3-({3-O-[(3R)-3-(decanoyloxy)tetradecanoyl]-2-{[(3R)-3-(decanoyloxy)tetradecanoyl]amino}-2-deoxy-6-O-(3-deoxy-α-D-manno-oct-2-ulopyranonosyl)-4-O-phosphono-β-D-glucopyranosyl}oxy)propanoate

[Formula 16]

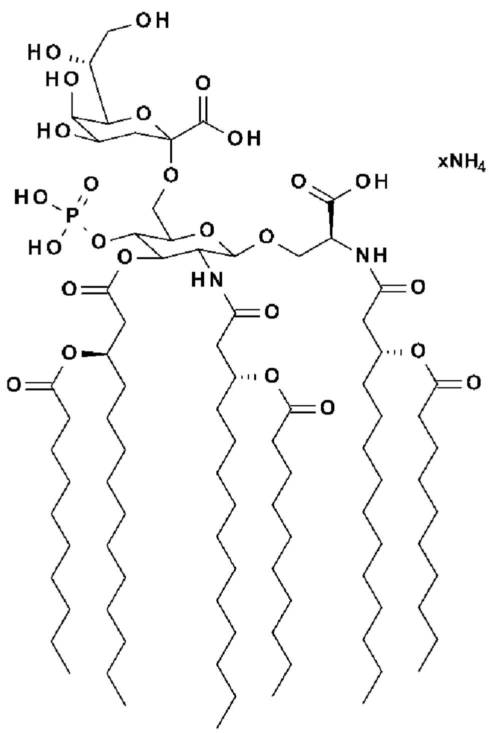

(4a) Benzyl (2S)-3-({4-O-[bis(benzyloxy)phosphoryl]-3-O-[(3R)-3-(decanoyloxy)tetradecanoyl]-2-{ [(3R)-3-(decanoyloxy)tetradecanoyl]amino}-2-deoxy-6-O-(triethylsilyl)-β-D-glucopyranosyl}oxy)-2-{[(3R)-3-(decanoyloxy)tetradecanoyl]amino}propanoate

The title compound (1.49 g) was obtained through reaction in the same manner as in Example 2(2a) using benzyl (2S)-3-[(4-O-[bis(benzyloxy)phosphoryl]-3-O-[(3R)-3-(decanoyloxy)tetradecanoyl]-2-{ [(3R)-3-(decanoyloxy)tetradecanoyl]amino}-2-deoxy-β-D-glucopyranosyl)oxy]-2-{ [(3R)-3-(decanoyloxy)tetradecanoyl]amino}propanoate (1.40 g) (Bioorganic & Medicinal Chemistry Letters 2008, 18, 5350-5354).

(4b) Benzyl (2S)-3-[(6-O-[1-benzyl-7,8-di-O-benzyl-3-deoxy-4,5-O-(1-methylethylidene)-α-D-manno-oct-2-ulopyranonosyl]-4-O-[bis(benzyloxy)phosphoryl]-3-O-[(3R)-3-(decanoyloxy)tetradecanoyl]-2-{[(3R)-3-(decanoyloxy)tetradecanoyl]amino}-2-deoxy-β-D-glucopyranosyl)oxy]-2-{[(3R)-3-(decanoyloxy)tetradecanoyl]amino}propanoate

The title compound (1.39 g) was obtained through reaction in the same manner as in Example 2(2b) using the compound (1.49 g) obtained in Example 4(4a).

(4c) Benzyl (2S)-3-{[6-O-(1-benzyl-7,8-di-O-benzyl-3-deoxy-α-D-manno-oct-2-ulopyranonosyl)-4-O-[bis(benzyloxy)phosphoryl]-3-O-[(3R)-3-(decanoyloxy)tetradecanoyl]-2-{[(3R)-3-(decanoyloxy)tetradecanoyl]amino}-2-deoxy-β-D-glucopyranosyl]oxy}-2-{[(3R)-3-(decanoyloxy)tetradecanoyl]amino}propanoate

The title compound (1.25 g) was obtained through reaction in the same manner as in Example 2(2c) using the compound (1.39 g) obtained in Example 4(4b).

(4d) Ammonium (2S)-2-{[(3R)-3-(decanoyloxy)tetradecanoyl]amino}-3-({3-O-[(3R)-3-(decanoyloxy)tetradecanoyl]-2-{ [(3R)-3-(decanoyloxy)tetradecanoyl]amino}-2-deoxy-6-O-(3-deoxy-α-D-manno-oct-2-ulopyranonosyl)-4-O-phosphono-β-D-glucopyranosyl}oxy)propanoate

The title compound (213 mg) was obtained through reaction in the same manner as in Example 1(1k) using the compound (309 mg) obtained in Example 4(4c).

(Example 5) Ammonium (2S)-2-{[(3R)-3-(decanoyloxy)tetradecanoyl]amino}-3-{[3-deoxy-α-D-manno-oct-2-ulopyranonosyl-(2→4)-3-deoxy-α-D-manno-oct-2-ulopyranonosyl-(2→6)-3-O-[(3R)-3-(decanoyloxy)tetradecanoyl]-2-{ [(3R)-3-(decanoyloxy)tetradecanoyl]amino}-2-deoxy-4-O-phosphono-P-D-glucopyranosyl]oxy}propanoate

[Formula 17]

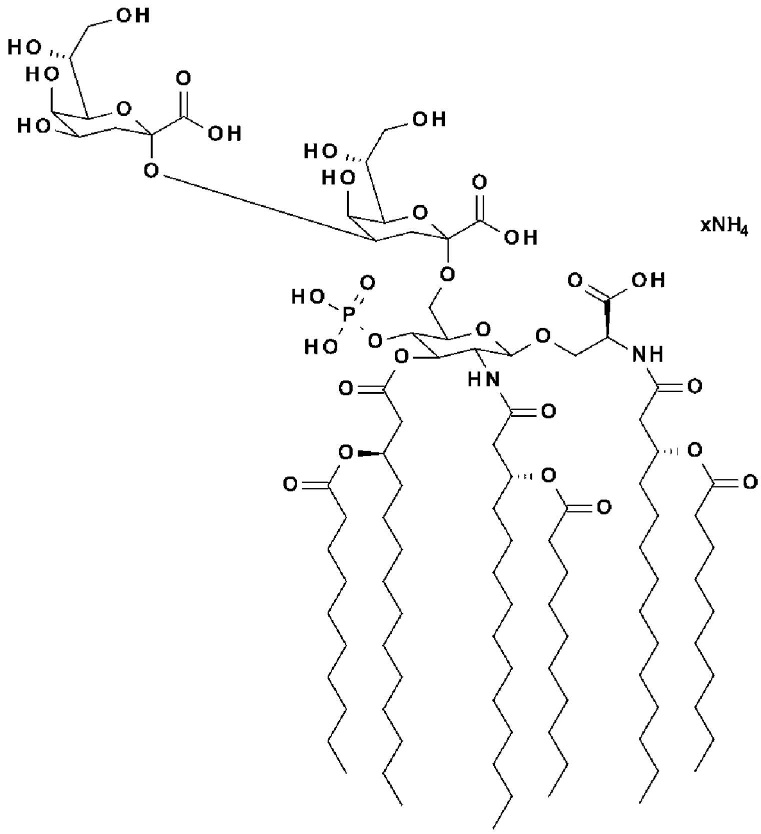

(5a) Benzyl (2S)-3-[(6-O-[1-benzyl-7,8-di-O-benzyl-3-deoxy-4-O-(triethylsilyl)-α-D-manno-oct-2-ulopyranonosyl]-4-O-[bis(benzyloxy)phosphoryl]-3-O-[(3R)-3-(decanoyloxy)tetradecanoyl]-2-{[(3R)-3-(decanoyloxy)tetradecanoyl]amino}-2-deoxy-β-D-glucopyranosyl)oxy]-2-{[(3R)-3-(decanoyloxy)tetradecanoyl]amino}propanoate

The title compound (614 mg) was obtained through reaction in the same manner as in Example 3(3a) using the compound (619 mg) obtained in Example 4(4c).

(5b) Benzyl (2S)-3-{[1-benzyl-7,8-di-O-benzyl-3-deoxy-4,5-O-(1-methylethylidene)-α-D-manno-oct-2-ulopyranonosyl-(2→4)-1-benzyl-7,8-di-O-benzyl-3-deoxy-α-D-manno-oct-2-ulopyranonosyl-(2→6)-4-O-[bis(benzyloxy)phosphoryl]-3-O-[(3R)-3-(decanoyloxy)tetradecanoyl]-2-{[(3R)-3-(decanoyloxy)tetradecanoyl]amino}-2-deoxy-β-D-glucopyranosyl]oxy}-2-{[(3R)-3-(decanoyloxy)tetradecanoyl]amino}propanoate

The title compound (527 mg) was obtained through reaction in the same manner as in Example 3(3b) using the compound (614 mg) obtained in Example 5(5a).

(5c) Benzyl (2S)-3-{[1-benzyl-7,8-di-O-benzyl-3-deoxy-α-D-manno-oct-2-ulopyranonosyl-(2→4)-1-benzyl-7,8-di-O-benzyl-3-deoxy-α-D-manno-oct-2-ulopyranonosyl-(2→6)-4-O-[bis(benzyloxy)phosphoryl]-3-O-[(3R)-3-(decanoyloxy)tetradecanoyl]-2-{[(3R)-3-(decanoyloxy)tetradecanoyl]amino}-2-deoxy-β-D-glucopyranosyl]oxy}-2-{[(3R)-3-(decanoyloxy)tetradecanoyl]amino}propanoate

The title compound (319 mg) was obtained through reaction in the same manner as in Example 3(3c) using the compound (484 mg) obtained in Example 5(5b).

5(5d) Ammonium (2S)-2-{[(3R)-3-(decanoyloxy)tetradecanoyl]amino}-3-{[3-deoxy-α-D-manno-oct-2-ulopyranonosyl-(2→4)-3-deoxy-α-D-manno-oct-2-ulopyranonosyl-(2→6)-3-O-[(3R)-3-(decanoyloxy)tetradecanoyl]-2-{ [(3R)-3-(decanoyloxy)tetradecanoyl]amino}-2-deoxy-4-O-phosphono-β-D-glucopyranosyl]oxy}propanoate

The title compound (167 mg) was obtained through reaction in the same manner as in Example 1(1k) using the compound (264 mg) obtained in Example 5(5c).

(Example 6) Ammonium (2S)-2-{[(3R)-3-(decanoyloxy)tetradecanoyl]amino}-3-({3-O-[(3R)-3-(decanoyloxy)tetradecanoyl]-2-{[(3R)-3-(decanoyloxy)tetradecanoyl]amino}-2-deoxy-6-O-D-glucopyranuronosyl-4-O-phosphono-β-D-glucopyranosyl}oxy)propanoate

[Formula 18]

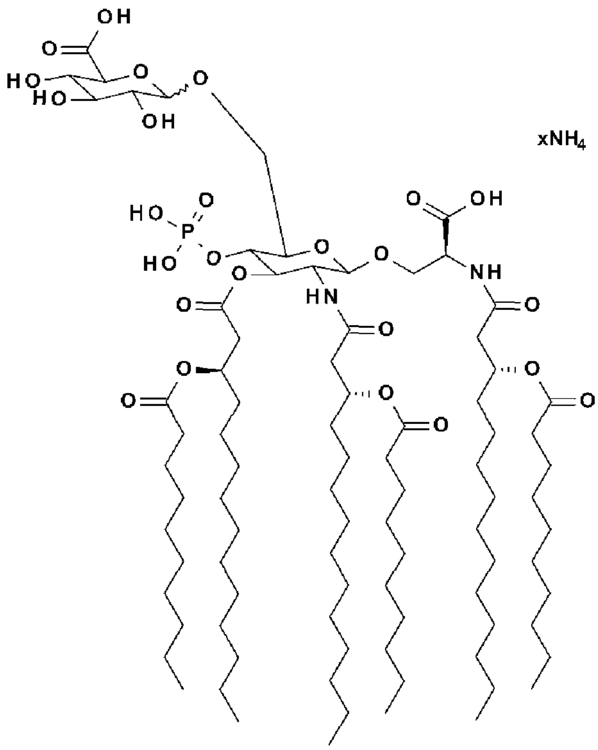

(6a) Benzyl (2S)-3-{[6-O-(6-benzyl-2,3,4-tri-O-benzyl-D-glucopyranuronosyl)-4-O-[bis(benzyloxy)phosphoryl]-3-O-[(3R)-3-(decanoyloxy)tetradecanoyl]-2-{[(3R)-3-(decanoyloxy)tetradecanoyl]amino}-2-deoxy-β-D-glucopyranosyl]oxy}-2-{[(3R)-3-(decanoyloxy)tetradecanoyl]amino}propanoate

To a solution of benzyl 2,3,4-tri-O-benzyl-D-glucopyranuronate (170 mg) in dichloromethane (3 mL), trichloroacetonitrile (0.31 mL) and 1,8-diazabicyclo[5.4.0]undec-7-ene (0.009 mL) were added at 0°C, and the mixture was stirred at the same temperature for 1 hour. After concentration of the reaction mixture, the residue was purified by silica gel column chromatography [n-hexane/ethyl acetate] to obtain a product (201 mg). To a solution of the product in dichloromethane (3 mL), benzyl (2S)-3-[(4-O-[bis(benzyloxy)phosphoryl]-3-O-[(3R)-3-(decanoyloxy)tetradecanoyl]-2-{[(3R)-3-(decanoyloxy)tetradecanoyl]amino}-2-deoxy-β-D-glucopyranosyl)oxy]-2-{[(3R)-3-(decanoyloxy)tetradecanoyl]amino}propanoate (150 mg) and molecular sieve 4A, 1/16 (100 mg) were added at room temperature, and the mixture was stirred at the same temperature for 20 minutes. Then, trimethylsilyl trifluoromethanesulfonate (0.003 mL) was added at 0°C, and the mixture was stirred at the same temperature for 30 minutes. The reaction was terminated by the addition of triethylamine. Then, the molecular sieve was filtered off, and the filtrate was concentrated under reduced pressure. The residue was purified by silica gel column chromatography [n-hexane/ethyl acetate] to obtain a less-polar diastereomer (6a-1, 48.7 mg) and a more-polar diastereomer (6a-2, 59.7 mg) of the title compound.

(6b) Ammonium (2S)-2-{[(3R)-3-(decanoyloxy)tetradecanoyl]amino}-3-({3-O-[(3R)-3-(decanoyloxy)tetradecanoyl]-2-{[(3R)-3-(decanoyloxy)tetradecanoyl]amino}-2-deoxy-6-O-D-glucopyranuronosyl-4-O-phosphono-β-D-glucopyranosyl}oxy)propanoate

The title compound (29.4 mg) was obtained through reaction in the same manner as in Example 1(1k) using the more-polar diastereomer (6a-2, 59.7 mg) obtained in Example 6(6a).

(Example 7) Ammonium (2S)-3-{[6-O-(2-acetamido-2-deoxy-β-D-glucopyranosyl)-3-O-[(3R)-3-(decanoyloxy)tetradecanoyl]-2-{[(3R)-3-(decanoyloxy)tetradecanoyl]amino}-2-deoxy-4-O-phosphono-β-D-glucopyranosyl]oxy}-2-{[(3R)-3-(decanoyloxy)tetradecanoyl]amino}propanoate

[Formula 19]

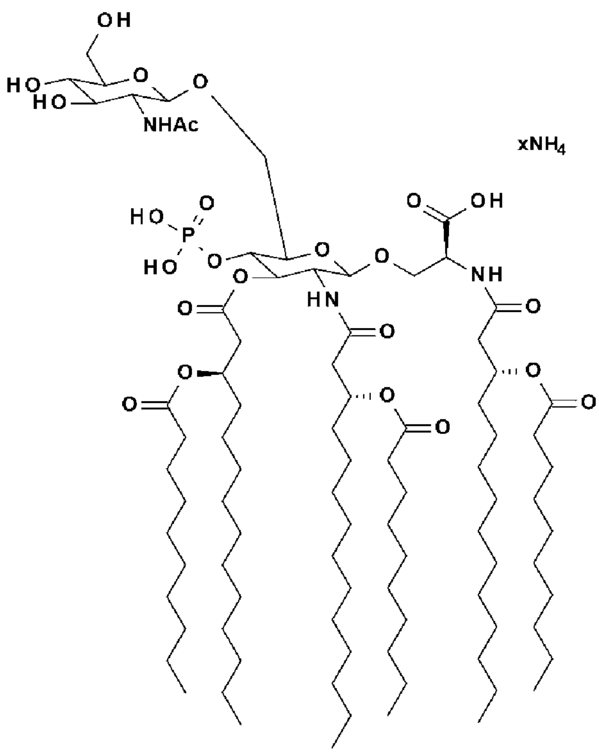

(7a) Benzyl (2S)-3-({4-O-[bis(benzyloxy)phosphoryl]-3-O-[(3R)-3-(decanoyloxy)tetradecanoyl]-2-{[(3R)-3-(decanoyloxy)tetradecanoyl]amino}-2-deoxy-6-O-(3,4,6-triO-benzyl-2-deoxy-2-{[(2,2,2-trichloroethoxy)carbonyl]amino}-β-D-glucopyranosyl)-β-D-glucopyranosyl}oxy)-2-{[(3R)-3-(decanoyloxy)tetradecanoyl]amino}propanoate

The title compound (187 mg) was obtained through reaction in the same manner as in Example 6(6a) using 3,4,6-tri-O-benzyl-2-deoxy-2-{[(2,2,2-trichloroethoxy)carbonyl]amino}-D-glucopyranose (250 mg) (Peptide Science (2009), 45th, 179-182).

(7b) Benzyl (2S)-3-{[6-O-(2-acetamido-3,4,6-tri-O-benzyl-2-deoxy-β-D-glucopyranosyl)-4-O-[bis(benzyloxy)phosphoryl]-3-O-[(3R)-3-(decanoyloxy)tetradecanoyl]-2-{[(3R)-3-(decanoyloxy)tetradecanoyl]amino}-2-deoxy-β-D-glucopyranosyl]oxy}-2-{[(3R)-3-(decanoyloxy)tetradecanoyl]amino}propanoate

To a solution of the compound (187 mg) obtained in Example 7(7a) in acetic acid (4 mL), a zinc powder (200 mg) was added at room temperature, and the mixture was stirred at the same temperature for 1 hour. Zinc was filtered off, and the filtrate was concentrated under reduced pressure. The residue was purified by silica gel column chromatography [dichloromethane/methanol] to obtain a product (90.6 mg). To a solution of the product in dichloromethane-methanol (1:2, 3 mL), triethylamine (0.1 mL) and acetic anhydride (0.5 mL) were added at room temperature, and the mixture was stirred at the same temperature for 15 minutes. The reaction was terminated by the addition of a saturated aqueous solution of sodium bicarbonate to the reaction mixture, followed by extraction with ethyl acetate. The organic layer was washed with saturated saline and then dried over anhydrous sodium sulfate. The drying agent was filtered off, and the filtrate was concentrated under reduced pressure. The residue was purified by silica gel column chromatography [n-hexane/ethyl acetate] to obtain the title compound (57.2 mg).

(7c) Ammonium (2S)-3-{[6-O-(2-acetamido-2-deoxy-β-D-glucopyranosyl)-3-O-[(3R)-3-(decanoyloxy)tetradecanoyl]-2-{[(3R)-3-(decanoyloxy)tetradecanoyl]amino}-2-deoxy-4-O-phosphono-β-D-glucopyranosyl]oxy}-2-{[(3R)-3-(decanoyloxy)tetradecanoyl]amino}propanoate

The title compound (28.5 mg) was obtained through reaction in the same manner as in Example 1(1k) using the compound (57.2 mg) obtained in Example 7(7b).

(Example 8) Ammonium (2S)-3-({6-O-[2-acetamido-2-deoxy-4-O-(hydroxyphosphinato)-β-D-glucopyranosyl]-3-O-[(3R)-3-(decanoyloxy)tetradecanoyl]-2-{[(3R)-3-(decanoyloxy)tetradecanoyl]amino}-2-deoxy-4-O-phosphono-β-D-glucopyranosyl}oxy)-2-{[(3R)-3-(decanoyloxy)tetradecanoyl]amino}propanoate

[Formula 20]

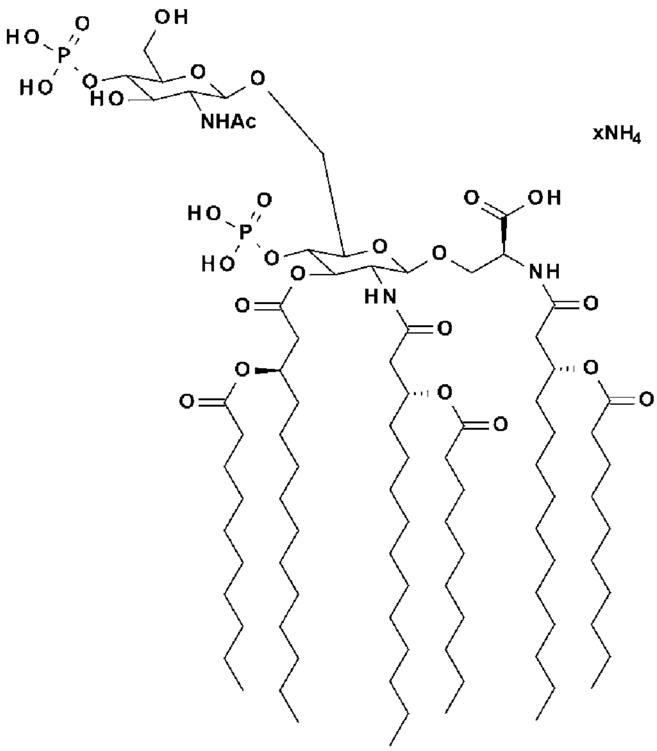

(8a) Allyl 3-O-benzyl-6-O-[(benzyloxy)carbonyl]-2-deoxy-2-{[(2,2,2-trichloroethoxy)carbonyl]amino}-α-D-glucopyranoside

To a solution of allyl 3-O-benzyl-2-deoxy-2-{[(2,2,2-trichloroethoxy)carbonyl]amino}-α-D-glucopyranoside (3.78 g) (Journal of Endotoxin Research 1994, 1 (3), 149-163) in tetrahydrofuran (30 mL), pyridine (1.26 mL) and carbobenzoxy chloride (1.67 mL) were added at room temperature, and the mixture was stirred at the same temperature for 2 hours. The reaction was terminated by the addition of a saturated aqueous solution of sodium bicarbonate to the reaction mixture, followed by extraction with ethyl acetate. The organic layer was washed with saturated saline and then dried over anhydrous sodium sulfate. The drying agent was filtered off, and the filtrate was concentrated under reduced pressure. The residue was purified by silica gel column chromatography [n-hexane/ethyl acetate/dichloromethane] to obtain the title compound (3.88 g).

(8b) Allyl 3-O-benzyl-6-O-[(benzyloxy)carbonyl]-4-O-[bis(benzyloxy)phosphoryl]-2-deoxy-2-{[(2,2,2-trichloroethoxy)carbonyl]amino}-α-D-glucopyranoside

The title compound (5.51 g) was obtained through reaction in the same manner as in Example 1(1i) using the compound (3.88 g) obtained in Example 8(8a).

(8c) 3-O-Benzyl-6-O-[(benzyloxy)carbonyl]-4-O-[bis(benzyloxy)phosphoryl]-2-deoxy-2-{[(2,2,2-trichloroethoxy)carbonyl]amino}-D-glucopyranose

The title compound (4.11 g) was obtained through reaction in the same manner as in Example 1(1b) using the compound (5.51 g) obtained in Example 8(8b).

(8d) Benzyl (2S)-3-{[6-O-(3-O-benzyl-6-O-[(benzyloxy)carbonyl]-4-O-[bis(benzyloxy)phosphoryl]-2-deoxy-2-{[(2,2,2-trichloroethoxy)carbonyl]amino}-β-D-glucopyranosyl)-4-O-[bis(benzyloxy)phosphoryl]-3-O-[(3R)-3-(decanoyloxy)tetradecanoyl]-2-{[(3R)-3-(decanoyloxy)tetradecanoyl]amino}-2-deoxy-β-D-glucopyranosyl]oxy}-2-{[(3R)-3-(decanoyloxy)tetradecanoyl]amino}propanoate

The title compound (261 mg) was obtained through reaction in the same manner as in Example 6(6a) using the compound (380 mg) obtained in Example 8(8c).

(8e) Benzyl (2S)-3-[(6-O-{2-acetamido-3-O-benzyl-6-O-[(benzyloxy)carbonyl]-4-O-[bis(benzyloxy)phosphoryl]-2-deoxy-β-D-glucopyranosyl}-4-O-[bis(benzyloxy)phosphoryl]-3-O-[(3R)-3-(decanoyloxy)tetradecanoyl]-2-{[(3R)-3-(decanoyloxy)tetradecanoyl]amino}-2-deoxy-β-D-glucopyranosyl)oxy]-2-{[(3R)-3-(decanoyloxy)tetradecanoyl]amino}propanoate

To a solution of the compound (261 mg) obtained in Example 8(8d) in acetic acid (4 mL), a zinc powder (520 mg) was added at room temperature, and the mixture was stirred at the same temperature for 1 hour. Zinc was filtered off, and the filtrate was concentrated under reduced pressure. The residue was purified by silica gel column chromatography [dichloromethane/methanol] to obtain a product (131 mg). To a solution of the product in pyridine (1 mL), acetic anhydride (1 mL) was added at room temperature, and the mixture was stirred at the same temperature for 30 minutes. The reaction mixture was concentrated under reduced pressure. Then, the residue was purified by silica gel column chromatography [n-hexane/ethyl acetate] to obtain the title compound (102 mg).

(8f) Ammonium (2S)-3-({6-O-[2-acetamido-2-deoxy-4-O-(hydroxyphosphinato)-β-D-glucopyranosyl]-3-O-[(3R)-3-(decanoyloxy)tetradecanoyl]-2-{ [(3R)-3-(decanoyloxy)tetradecanoyl]amino}-2-deoxy-4-O-phosphono-β-D-glucopyranosyl}oxy)-2-{[(3R)-3-(decanoyloxy)tetradecanoyl]amino}propanoate

The title compound (62.1 mg) was obtained through reaction in the same manner as in Example 1(1k) using the compound (102 mg) obtained in Example 8(8e).

(Example 9) Ammonium (2S)-3-({6-O-[2-acetamido-2-deoxy-6-O-(hydroxyphosphinato)-β-D-glucopyranosyl]-3-O-[(3R)-3-(decanoyloxy)tetradecanoyl]-2-{[(3R)-3-(decanoyloxy)tetradecanoyl]amino}-2-deoxy-4-O-phosphono-β-D-glucopyranosyl}oxy)-2-{[(3R)-3-(decanoyloxy)tetradecanoyl]amino}propanoate

[Formula 21]

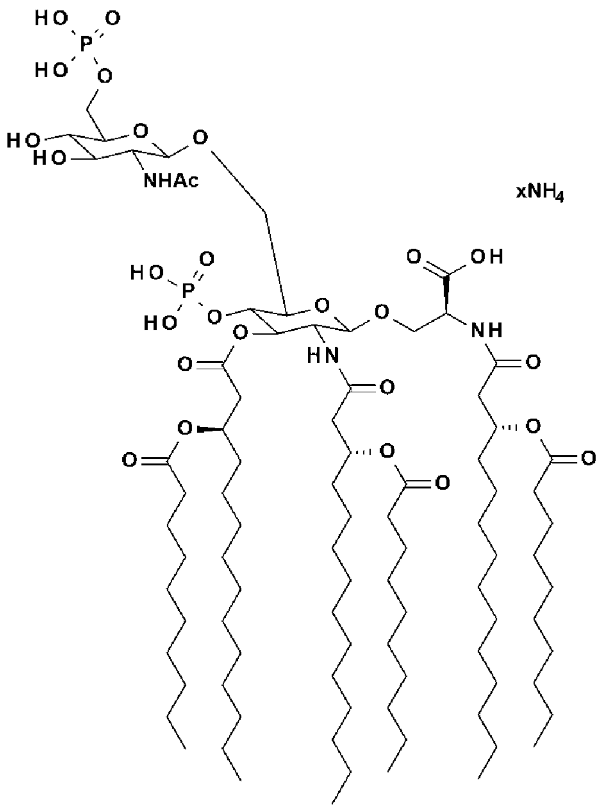

(9a) Allyl 3,4-di-O-benzyl-6-O-[bis(benzyloxy)phosphoryl]-2-deoxy-2-{[(2,2,2-trichloroethoxy)carbonyl]amino}-α-D-glucopyranoside

The title compound (4.52 g) was obtained through reaction in the same manner as in Example 1(1i) using allyl 3,4-di-O-benzyl-2-deoxy-2-{[(2,2,2-trichloroethoxy)carbonyl]amino}-α-D-glucopyranoside(3.11 g) (Synlett 2007, 1, 164-166).

(9b) 3,4-di-O-Benzyl-6-O-[bis(benzyloxy)phosphoryl]-2-deoxy-2-{[(2,2,2-trichloroethoxy)carbonyl]amino}-D-glucopyranose

The title compound (3.34 g) was obtained through reaction in the same manner as in Example 1(1b) using the compound (4.52 g) obtained in Example 9(9a).

(9c) Benzyl (2S)-3-({4-O-[bis(benzyloxy)phosphoryl]-3-O-[(3R)-3-(decanoyloxy)tetradecanoyl]-2-{[(3R)-3-(decanoyloxy)tetradecanoyl]amino}-2-deoxy-6-O-(3,4-di-O-benzyl-6-O-[bis(benzyloxy)phosphoryl]-2-deoxy-2-{[(2,2,2-trichloroethoxy)carbonyl]amino}-β-D-glucopyranosyl)-β-D-glucopyranosyl}oxy)-2-{[(3R)-3-(decanoyloxy)tetradecanoyl]amino}propanoate

The title compound (215 mg) was obtained through reaction in the same manner as in Example 6(6a) using the compound (360 mg) obtained in Example 9(9b).

(9d) Benzyl (2S)-3-[(6-O-{2-acetamido-3,4-di-O-benzyl-6-O-[bis(benzyloxy)phosphoryl]-2-deoxy-β-D-glucopyranosyl}-4-O-[bis(benzyloxy)phosphoryl]-3-O-[(3R)-3-(decanoyloxy)tetradecanoyl]-2-{[(3R)-3-(decanoyloxy)tetradecanoyl]amino}-2-deoxy-β-D-glucopyranosyl)oxy]-2-{[(3R)-3-(decanoyloxy)tetradecanoyl]amino}propanoate

The title compound (108 mg) was obtained through reaction in the same manner as in Example 8(8e) using the compound (169 mg) obtained in Example 9(9c).

(9e) Ammonium (2S)-3-({6-O-[2-acetamido-2-deoxy-6-O-(hydroxyphosphinato)-β-D-glucopyranosyl]-3-O-[(3R)-3-(decanoyloxy)tetradecanoyl]-2-{[(3R)-3-(decanoyloxy)tetradecanoyl]amino}-2-deoxy-4-O-phosphono-β-D-glucopyranosyl}oxy)-2-{[(3R)-3-(decanoyloxy)tetradecanoyl]amino}propanoate

The title compound (43.3 mg) was obtained through reaction in the same manner as in Example 1(1k) using the compound (70.9 mg) obtained in Example 9(9d).

(Example 10) Ammonium 6,10-anhydro-8-0-[(3R)-3-(decanoyloxy)tetradecanoyl]-3,7-bis{[(3R)-3-(decanoyloxy)tetradecanoyl]amino}-2,3,4,5,7-pentadeoxy-9-O-phosphono-D-erythro-L-galacto-undecanoate

[Formula 22]

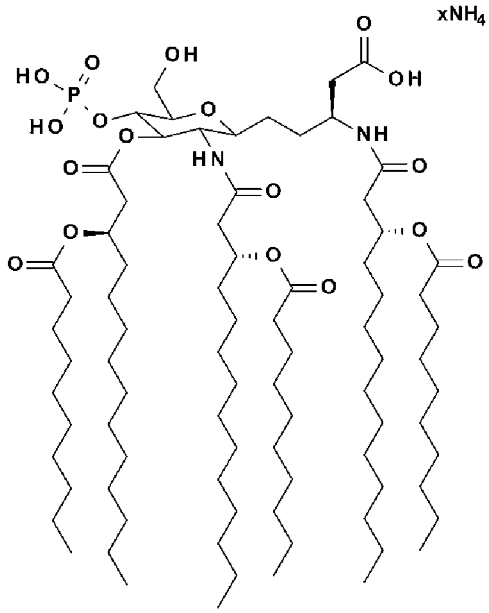

(10a) tert-Butyl [(3R)-5-{[tert-butyl(diphenyl)silyl]oxy}pent-1-yn-3-yl]carbamate

To a solution of tert-butyl [(2R)-4-{[tert-butyl(diphenyl)silyl]oxy}-1-oxobutan-2-yl]carbamate (27.7 g) (Tetrahedron Letters 2007, 48, 7279-7282) in methanol (270 mL), dimethyl (1-diazo-2-oxopropyl)phosphonate (14.1 mL) and potassium carbonate (17.4 g) were added at 0°C, and the mixture was stirred overnight at room temperature. The reaction was terminated by the addition of a saturated aqueous solution of ammonium chloride to the reaction mixture, followed by extraction with n-hexane. The organic layer was washed with saturated saline and then dried over anhydrous sodium sulfate. The drying agent was filtered off, and the filtrate was concentrated under reduced pressure. The residue was purified by silica gel column chromatography [n-hexane/ethyl acetate] to obtain the title compound (23.9 g).

(10b) 6,10-Anhydro-8,9,11-tri-O-benzyl-3-[(tert-butoxycarbonyl)amino]-1-O-[tert-butyl(diphenyl)silyl]-2,3,4,5,7-pentadeoxy-7-nitro-D-erythro-L-galacto-undec-4-ynitol

To a solution of the compound (23.9 g) obtained in Example 10(10a) in tetrahydrofuran (200 mL), a 1.6 M solution of n-butyllithium in n-hexane (75 mL) was added dropwise at -78°C, and the mixture was stirred at the same temperature for 1 hour. To a solution of 1,5-anhydro-3,4,6-tri-O-benzyl-2-deoxy-2-nitro-D-arabino-hex-1-enytol (25.2 g) (European Journal of Organic Chemistry 1998, 8, 1609-1613) in tetrahydrofuran (200 mL), the solution of the organolithium compound in tetrahydrofuran prepared above was added dropwise at -78°C. After stirring at the same temperature for 1 hour, the reaction was terminated by the addition of a saturated aqueous solution of ammonium chloride to the reaction mixture, followed by extraction with ethyl acetate. The organic layer was washed with saturated saline and then dried over anhydrous sodium sulfate. The drying agent was filtered off, and the filtrate was concentrated under reduced pressure. The residue was purified by silica gel column chromatography [n-hexane/ethyl acetate] to obtain the title

compound (18.4 g).

(10c) 7-Amino-6,10-anhydro-8,9,11-tri-O-benzyl-3-[(tert-butoxycarbonyl)amino]-1-O-[tert-butyl(diphenyl)silyl]-2,3,4,5,7-pentadeoxy-D-erythro-L-galacto-undec-4-ynitol

To the compound (4.47 g) obtained in Example 10(10b) in tetrahydrofuran (25 mL), acetic acid (25 mL) and a zinc powder (3.30 g) were added at room temperature, and the mixture was stirred at the same temperature for 7 hours. Zinc was filtered off, and the filtrate was concentrated under reduced pressure. The residue was purified by silica gel column chromatography [n-hexane/ethyl acetate] to obtain the title compound (2.78 g).

(10d) 6,10-Anhydro-8,9,11-tri-O-benzyl-3-[(tert-butoxycarbonyl)amino]-1-O-[tert-butyl(diphenyl)silyl]-2, 3, 4, 5, 7-pentadeoxy-7-{ [(2,2,2-trichloroethoxy)carbonyl]amino}-D-erythro-L-galacto-undec-4-ynitol

To a solution of the compound (2.78 g) obtained in Example 10(10c) in dichloromethane (30 mL), N,N-diisopropylethylamine (1.11 mL) and 2,2,2-trichloroethyl chloroformate (0.652 mL) were added at room temperature, and the mixture was stirred at the same temperature for 2 hours. The reaction was terminated by the addition of a saturated aqueous solution of sodium bicarbonate to the reaction mixture, followed by extraction with ethyl acetate. The organic layer was washed with saturated saline and then dried over anhydrous sodium sulfate. The drying agent was filtered off, and the filtrate was concentrated under reduced pressure. The residue was purified by silica gel column chromatography [n-hexane/ethyl acetate] to obtain the title compound (2.87 g).

(10e) 6,10-Anhydro-8,9,11-tri-O-benzyl-3-[(tert-butoxycarbonyl)amino]-1-O-[tert-butyl(diphenyl)silyl]-2, 3, 4, 5, 7-pentadeoxy-7-{ [(2,2,2-trichloroethoxy)carbonyl]amino}-D-erythro-L-galacto-undec-4-ynitol

To a solution of the compound (2.87 g) obtained in Example 10(10d) in dimethoxyethane (30 mL), p-toluenesulfonyl hydrazide (4.09 g) was added at room temperature, and a 1 M aqueous sodium acetate solution (15 mL) was added every 30 minutes in 6 divided portions at 80°C. The mixture was stirred at the same temperature for 2 hours, followed by extraction with ethyl acetate. The organic layer was washed with saturated saline and then dried over anhydrous sodium sulfate. The drying agent was filtered off, and the filtrate was concentrated under reduced pressure. The residue was purified by silica gel column chromatography [n-hexane/ethyl acetate] to obtain the title compound (2.57 g).

(10f) 6,10-Anhydro-8,9,11-tri-O-benzyl-3-[(tert-butoxycarbonyl)amino]-2,3,4,5,7-pentadeoxy-7-{[(2,2,2-trichloroethoxy)carbonyl]amino}-D-erythro-L-galacto-undecitol

To a solution of the compound (2.57 g) obtained in Example 10(10e) in tetrahydrofuran (20 mL), acetic acid (0.421 mL) and a 1 M solution of tetrabutylammonium fluoride in tetrahydrofuran (7.35 mL) were added at room temperature, and the mixture was stirred overnight at the same temperature. The reaction was terminated by the addition of water to the reaction mixture, followed by extraction with ethyl acetate. The organic layer was washed with saturated saline and then dried over anhydrous sodium sulfate. The drying agent was filtered off, and the filtrate was concentrated under reduced pressure. The residue was purified by silica gel column chromatography [n-hexane/ethyl acetate] to obtain the title compound (1.99 g).

(10g) 6,10-Anhydro-8,9,11-tri-O-benzyl-3-[(tert-butoxycarbonyl)amino]-2,3,4,5,7-pentadeoxy-7-{[(2,2,2-trichloroethoxy)carbonyl]amino}-D-erythro-L-galacto-undecose

To a solution of the compound (1.99 g) obtained in Example 10(10f) in dichloromethane (30 mL), Dess-Martin periodinane (1.25 g) was added at room temperature, and the mixture was stirred at the same temperature for 3 hours. The reaction was terminated by the addition of a saturated aqueous solution of sodium bicarbonate to the reaction mixture, followed by extraction with dichloromethane. The organic layer was washed with saturated saline and then dried over anhydrous sodium sulfate. The drying agent was filtered off, and the filtrate was concentrated under reduced pressure. The residue was purified by silica gel column chromatography [n-hexane/ethyl acetate] to obtain the title compound (1.85 g).

(10h) 6,10-Anhydro-8,9,11-tri-O-benzyl-3-[(tert-butoxycarbonyl)amino]-2,3,4,5,7-pentadeoxy-7-{[(2,2,2-trichloroethoxy)carbonyl]amino}-D-erythro-L-galacto-undecanoic acid

To a solution of the compound (1.85 g) obtained in Example 10(10g) in tert-butyl alcohol (20 mL), water (4 mL),

80% sodium chlorite (311 mg), 2-methyl-2-butene (1.5 mL), and sodium dihydrogen phosphate dihydrate (536 mg) were added at room temperature, and the mixture was stirred at the same temperature for 2 hours. Ethyl acetate was added to the reaction mixture for extraction. The organic layer was washed with saturated saline and then dried over anhydrous sodium sulfate. The drying agent was filtered off, and the filtrate was concentrated under reduced pressure. The residue was purified by silica gel column chromatography [n-hexane/ethyl acetate] to obtain the title compound (1.64 g).

(10i) Benzyl 6,10-anhydro-3-[(tert-butoxycarbonyl)amino]-2,3,4,5,7-pentadeoxy-9,11-O-(1-methylethylidene)-7-{[(2,2,2-trichloroethoxy)carbonyl]amino}-D-erythro-L-galacto-undecanoate

To a solution of the compound (1.64 g) obtained in Example 10(10h) in tetrahydrofuran (20 mL), 10% palladium on carbon (1.6 g) was added at room temperature, and the mixture was stirred at the same temperature for 8 hours under a hydrogen atmosphere. The palladium catalyst was filtered off, and the filtrate was then concentrated under reduced pressure to obtain a product. To a solution of the residue in N,N-dimethylformamide (10 mL), sodium bicarbonate (983 mg) and benzyl bromide (1.18 mL) were added at room temperature, and the mixture was stirred at 50°C for 4 hours. Sodium bicarbonate was filtered off, and the filtrate was concentrated under reduced pressure. The residue was purified by silica gel column chromatography [dichloromethane/methanol] to obtain a product. To a solution of the product in N,N-dimethylformamide (8 mL), 2,2-dimethoxypropane (8 mL) and p-toluenesulfonic acid monohydrate (23.6 mg) were added at room temperature, and the mixture was stirred overnight at the same temperature. The reaction was terminated by the addition of triethylamine, and the reaction mixture was then concentrated under reduced pressure. The residue was purified by silica gel column chromatography [n-hexane/ethyl acetate] to obtain the title compound (610 mg).

(10j) Benzyl 6,10-anhydro-3-[(tert-butoxycarbonyl)amino]-8-O-[(3R)-3-(decanoyloxy)tetradecanoyl]-2,3,4,5,7-pentadeoxy-9,11-O-(1-methylethylidene)-7-{[(2,2,2-trichloroethoxy)carbonyl]amino}-D-erythro-L-galacto-undecanoate

The title compound (380 mg) was obtained through reaction in the same manner as in Example 1(1a) using the compound (300 mg) obtained in Example 10(10i).

(10k) Benzyl 3-amino-6,10-anhydro-8-O-[(3R)-3-(decanoyloxy)tetradecanoyl]-2,3,4,5,7-pentadeoxy-7-{[(2,2,2-trichloroethoxy)carbonyl]amino}-D-erythro-L-galacto-undecanoate

The title compound (270 mg) was obtained through reaction in the same manner as in Example 1(1d) using the compound (380 mg) obtained in Example 10(10j).

(101) Benzyl 6,10-anhydro-8-O-[(3R)-3-(decanoyloxy)tetradecanoyl]-3-{[(3R)-3-(decanoyloxy)tetradecanoyl]amino}-2,3,4,5,7-pentadeoxy-7-{[(2,2,2-trichloroethoxy)carbonyl]amino}-D-erythro-L-galacto-undecanoate

The title compound (320 mg) was obtained through reaction in the same manner as in Example 1(1e) using the compound (270 mg) obtained in Example 10(10k).

(10m) Benzyl 7-amino-6,10-anhydro-8-O-[(3R)-3-(decanoyloxy)tetradecanoyl]-3-{[(3R)-3-(decanoyloxy)tetradecanoyl]amino}-2,3,4,5,7-pentadeoxy-D-erythro-L-galacto-undecanoate

The title compound (212 mg) was obtained through reaction in the same manner as in Example 1(1f) using the compound (320 mg) obtained in Example 10(101).

(10n) Benzyl 6,10-anhydro-8-O-[(3R)-3-(decanoyloxy)tetradecanoyl]-3,7-bis{[(3R)-3-(decanoyloxy)tetradecanoyl]amino}-2,3,4,5,7-pentadeoxy-D-erythro-L-galacto-undecanoate

The title compound (270 mg) was obtained through reaction in the same manner as in Example 1(1g) using the compound (212 mg) obtained in Example 10(10m).

(10o) Benzyl 6,10-anhydro-11-O-[tert-butyl(diphenyl)silyl]-8-O-[(3R)-3-(decanoyloxy)tetradecanoyl]-3,7-bis{[(3R)-3-(decanoyloxy)tetradecanoyl]amino}-2,3,4,5,7-pentadeoxy-D-erythro-L-galacto-undecanoate

The title compound (1.93 g) was obtained through reaction in the same manner as in Example 1(1h) using the compound (1.66 g) obtained in Example 10(10n).

(10p) Benzyl 6,10-anhydro-9-O-[bis(benzyloxy)phosphoryl]-11-O-[tert-butyl(diphenyl)silyl]-8-O-[(3R)-3-(decanoyloxy)tetradecanoyl]-3,7-bis{[(3R)-3-(decanoyloxy)tetradecanoyl]amino}-2,3,4,5,7-pentadeoxy-D-erythro-L-galacto-undecanoate

The title compound (2.15 g) was obtained through reaction in the same manner as in Example 1(1i) using the compound (1.93 g) obtained in Example 10(10o).

(10q) Benzyl 6,10-anhydro-9-O-[bis(benzyloxy)phosphoryl]-8-O-[(3R)-3-(decanoyloxy)tetradecanoyl]-3,7-bis{[(3R)-3-(decanoyloxy)tetradecanoyl]amino}-2,3,4,5,7-pentadeoxy-D-erythro-L-galacto-undecanoate

The title compound (1.54 g) was obtained through reaction in the same manner of Example 1(1j) using the compound (2.15 g) obtained in Example 10(10p).

(10r) Ammonium 6,10-anhydro-8-0-[(3R)-3-(decanoyloxy)tetradecanoyl]-3,7-bis{[(3R)-3-(decanoyloxy)tetradecanoyl]amino}-2,3,4,5,7-pentadeoxy-9-O-phosphono-D-erythro-L-galacto-undecanoate

The title compound (69.0 mg) was obtained through reaction in the same manner as in Example 1(1k) using the compound (106 mg) obtained in Example 10(10q).

(Example 11) Ammonium 6,10-anhydro-8-0-[(3R)-3-(decanoyloxy)tetradecanoyl]-3,7-bis{[(3R)-3-(decanoyloxy)tetradecanoyl]amino}-2,3,4,5,7-pentadeoxy-11-O-(3-deoxy-α-D-manno-oct-2-ulopyranonosyl)-9-O-phosphono-D-erythro-L-galacto-undecanoate

[Formula 23]

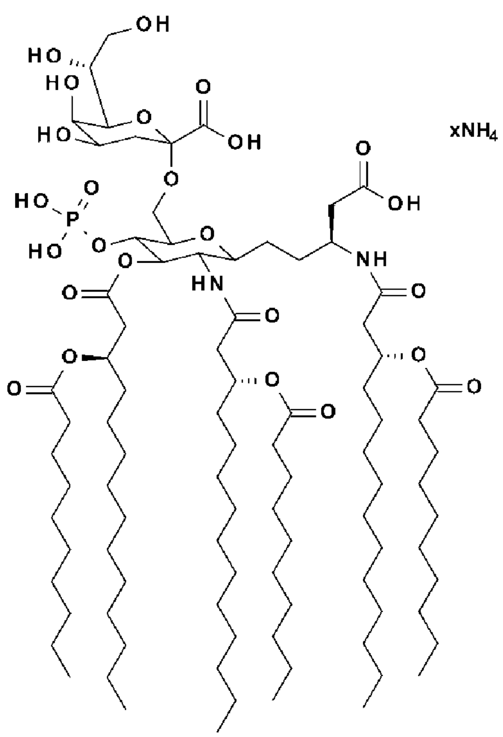

(11a) Benzyl 6,10-anhydro-9-O-[bis(benzyloxy)phosphoryl]-8-O-[(3R)-3-(decanoyloxy)tetradecanoyl]-3,7-bis{[(3R)-3-(decanoyloxy)tetradecanoyl]amino}-2,3,4,5,7-pentadeoxy-11-O-(triethylsilyl)-D-erythro-L-galacto-undecanoate

The title compound (1.49 g) was obtained through reaction in the same manner as in Example 2(2a) using the compound (1.40 g) obtained in Example 10(10q).

(11b) Benzyl 6,10-anhydro-11-O-[1-benzyl-7,8-di-O-benzyl-3-deoxy-4,5-O-(1-methylethylidene)-α-D-manno-oct-2-ulopyranonosyl]-9-O-[bis(benzyloxy)phosphoryl]-8-O-[(3R)-3-(decanoyloxy)tetradecanoyl]-3,7-bis{[(3R)-3-(decanoyloxy)tetradecanoyl]amino}-2,3,4,5,7-pentadeoxy-D-erythro-L-galacto-undecanoate

The title compound (1.28 g) was obtained through reaction in the same manner as in Example 2(2b) using the compound (1.49 g) obtained in Example 11(11a).

(11c) Benzyl 6,10-anhydro-11-O-(1-benzyl-7,8-di-O-benzyl-3-deoxy-α-D-manno-oct-2-ulopyranonosyl)-9-O-[bis(benzyloxy)phosphoryl]-8-O-[(3R)-3-(decanoyloxy)tetradecanoyl]-3,7-bis{[(3R)-3-(decanoyloxy)tetradecanoyl]amino}-2,3,4,5,7-pentadeoxy-D-erythro-L-galacto-undecanoate

The title compound (1.13 g) was obtained through reaction in the same manner as in Example 2(2c) using the compound (1.24 g) obtained in Example 11(11b).

(11d) Ammonium 6,10-anhydro-8-0-[(3R)-3-(decanoyloxy)tetradecanoyl]-3,7-bis{[(3R)-3-(decanoyloxy)tetradecanoyl]amino}-2,3,4,5,7-pentadeoxy-11-O-(3-deoxy-α-D-manno-oct-2-ulopyranonosyl)-9-O-phosphono-D-erythro-L-galacto-undecanoate

The title compound (192 mg) was obtained through reaction in the same manner as in Example 1(1k) using the compound (274 mg) obtained in Example 11(11c).

(Example 12) Ammonium 3-deoxy-α-D-manno-oct-2-ulopyranonosyl-(2→4)-3-deoxy-α-D-manno-oct-2-ulopyranonosyl-(2→11)-6,10-anhydro-8-O-[(3R)-3-(decanoyloxy)tetradecanoyl]-3,7-bis{[(3R)-3-(decanoyloxy)tetradecanoyl]amino}-2,3,4,5,7-pentadeoxy-9-O-phosphono-D-erythro-L-galacto-undecanoate

[Formula 24]

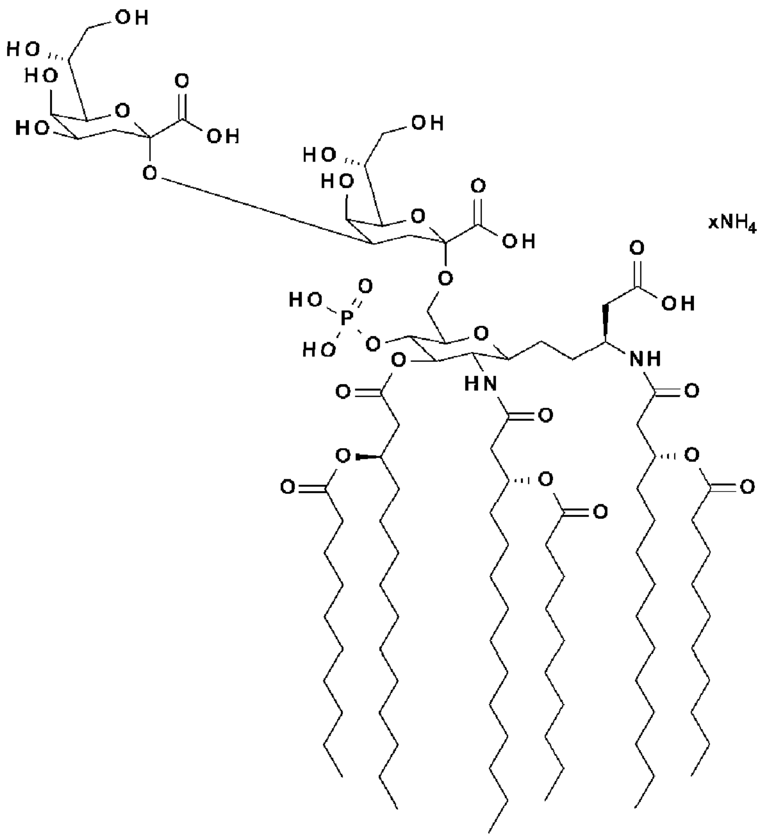

(12a) Benzyl 6,10-anhydro-11-O-[1-benzyl-7,8-di-O-benzyl-3-deoxy-4-O-(triethylsilyl)-α-D-manno-oct-2-ulopyranonosyl]-9-O-[bis(benzyloxy)phosphoryl]-8-O-[(3R)-3-(decanoyloxy)tetradecanoyl]-3,7-bis{[(3R)-3-(decanoyloxy)tetradecanoyl]amino}-2,3,4,5,7-pentadeoxy-D-erythro-L-galacto-undecanoate

The title compound (580 mg) was obtained through reaction in the same manner as in Example 3(3a) using the compound (562 mg) obtained in Example 11(11c).

(12b) Benzyl 1-benzyl-7,8-di-O-benzyl-3-deoxy-4,5-O-(1-methylethylidene)-α-D-manno-oct-2-ulopyranonosyl-(2→4)-1-benzyl-7,8-di-O-benzyl-3-deoxy-α-D-manno-oct-2-ulopyranonosyl- (2→11)-6,10-anhydro-9-O-[bis(benzyloxy)phosphoryl]-8-O-[(3R)-3-(decanoyloxy)tetradecanoyl]-3,7-bis{[(3R)-3-(decanoyloxy)tetradecanoyl]amino}-2,3,4,5,7-pentadeoxy-D-erythro-L-galacto-undecanoate

The title compound (420 mg) was obtained through reaction in the same manner as in Example 3(3b) using the compound (580 mg) obtained in Example 12(12a).

(12c) Benzyl 1-benzyl-7,8-di-O-benzyl-3-deoxy-α-D-manno-oct-2-ulopyranonosyl-(2→4)-1-benzyl-7,8-di-O-benzyl-3-deoxy-α-D-manno-oct-2-ulopyranonosyl-(2→11)-6,10-anhydro-9-O-[bis(benzyloxy)phosphoryl]-8-O-[(3R)-3-(decanoyloxy)tetradecanoyl]-3,7-bis{[(3R)-3-(decanoyloxy)tetradecanoyl]amino}-2,3,4,5,7-pentadeoxy-D-erythro-L-galacto-undecanoate

The title compound (223 mg) was obtained through reaction in the same manner as in Example 3(3c) using the compound (420 mg) obtained in Example 12(12b).

(12d) Ammonium 3-deoxy-α-D-manno-oct-2-ulopyranonosyl-(2→4)-3-deoxy-α-D-manno-oct-2-ulopyranonosyl-(2→11)-6,10-anhydro-8-O-[(3R)-3-(decanoyloxy)tetradecanoyl]-3,7-bis{ [(3R)-3-(decanoyloxy)tetradecanoyl]amino}-2,3,4,5,7-pentadeoxy-9-O-phosphono-D-erythro-L-galacto-undecanoate

The title compound (141 mg) was obtained through reaction in the same manner as in Example 1(1k) using the compound (223 mg) obtained in Example 12(12c).

(Example 13) Ammonium 5,9-anhydro-7-O-[(3R)-3-(decanoyloxy)tetradecanoyl]-2,6-bis{[(3R)-3-(decanoyloxy)tetradecanoyl]amino}-2,3,4,6-tetradeoxy-8-O-phosphono-D-erythro-L-galacto-decanoate

[Formula 25]

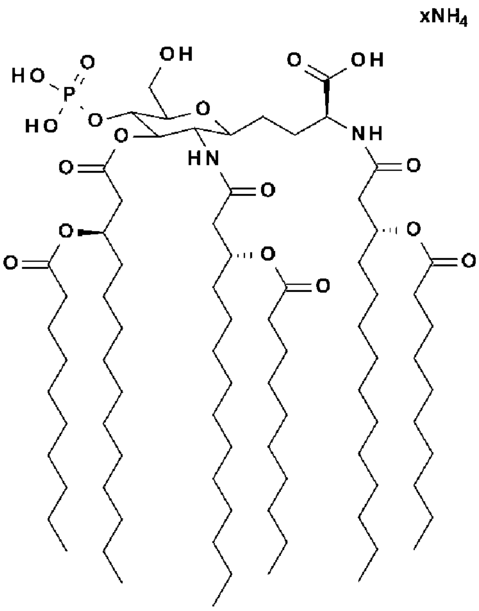

(13a) 3,7-Anhydro-5,6,8-tri-O-benzyl-1-[(4S)-3-(tert-butoxycarbonyl)-2,2-dimethyl-1,3-oxazolidin-4-ylJ-1,2,4-trideoxy-4-nitro-D-glycero-D-gulo-oct-1-ynitol

The title compound (9.34 g) was obtained through reaction in the same manner as in Example 10(10b) using tert-butyl (4S)-4-ethynyl-2,2-dimethyl-1,3-oxazolidine-3-carboxylate (5.50 g) (Tetrahedron 2007, 63, 8499-8513).

(13b) 4-Amino-3,7-anhydro-5,6,8-tri-O-benzyl-1-[(4S)-3-(tert-butoxycarbonyl)-2,2-dimethyl-1,3-oxazolidin-4-ylJ-1,2,4-trideoxy-D-glycero-D-gulo-oct-1-ynitol

The title compound (6.40 g) was obtained through reaction in the same manner as in Example 10(10c) using the compound (9.30 g) obtained in Example 13(13a).

(13c) 3,7-Anhydro-5,6,8-tri-O-benzyl-1-[(4S)-3-(tert-butoxycarbonyl)-2,2-dimethyl-1,3-oxazolidin-4-ylJ-1,2,4-trideoxy-4-{ [(2,2,2-trichloroethoxy)carbonyl]amino}-D-glycero-D-gulo-oct-1-ynitol

The title compound (7.10 g) was obtained through reaction in the same manner as in Example 10(10d) using the compound (6.40 g) obtained in Example 13(13b).

(13d) 3,7-Anhydro-5,6,8-tri-O-benzyl-1-[(4S)-3-(tert-butoxycarbonyl)-2,2-dimethyl-1,3-oxazolidin-4-ylJ-1,2,4-trideoxy-4-{ [(2,2,2-trichloroethoxy)carbonyl]amino}-D-glycero-D-gulo-octitol

The title compound (7.10 g) was obtained through reaction by the same manner as in Example 10(10e) using

the compound (7.10 g) obtained in Example 13(13c).

(13e) 5,9-anhydro-7,8,10-tri-O-benzyl-2-[(tert-butoxycarbonyl)amino]-2,3,4,6-tetradeoxy-6-{ [(2,2,2-trichloroethoxy)carbonyl]amino}-D-erythro-L-talo-decanoic acid

To a solution of the compound (6.70 g) obtained in Example 13(13d) in acetone (120 mL), Jones reagent (24 mL) was added at 0°C, and the mixture was stirred at room temperature for 1 hour. The reaction was terminated by the addition of 2-propanol at 0°C, followed by extraction with dichloromethane. The organic layer was washed with saturated saline and then dried over anhydrous sodium sulfate. The drying agent was filtered off, and the filtrate was concentrated under reduced pressure. The residue was purified by silica gel column chromatography [n-hexane/ethyl acetate] to obtain the title compound (4.71 g).

(13f) Benzyl 5, 9-anhydro-2-[(tert-butoxycarbonyl)amino]-2,3,4,6-tetradeoxy-8,10-O-(1-methylethylidene)-6-{ [(2,2,2-trichloroethoxy)carbonyl]amino}-D-erythro-L-galacto-decanoate

The title compound (930 mg) was obtained through reaction in the same manner as in Example 10(10i) using the compound (1.95 g) obtained in Example 13(13e).

(13g) Benzyl 5, 9-anhydro-2-[(tert-butoxycarbonyl)amino]-7-O-[(3R)-3-(decanoyloxy)tetradecanoyl]-2,3,4,6-tetradeoxy-8,10-O-(1-methylethylidene)-6-{ [(2,2,2-trichloroethoxy)carbonyl]amino}-D-erythro-L-galacto-decanoate

The title compound (1.23 g) was obtained through reaction in the same manner as in Example 1(1a) using the compound (930 mg) obtained in Example 13(13f).

(13h) Benzyl 2-amino-5,9-anhydro-7-O-[(3R)-3-(decanoyloxy)tetradecanoyl]-2,3,4,6-tetradeoxy-6-{[(2,2,2-trichloroethoxy)carbonyl]amino}-D-erythro-L-galacto-decanoate

The title compound (928 mg) was obtained through reaction in the same manner as in Example 1(1d) using the compound (1.23 g) obtained in Example 13(13g).

(13i) Benzyl 5,9-anhydro-7-O-[(3R)-3-(decanoyloxy)tetradecanoyl]-2-{[(3R)-3-(decanoyloxy)tetradecanoyl]amino}-2,3,4,6-tetradeoxy-6-{[(2,2,2-trichloroethoxy)carbonyl]amino}-D-erythro-L-galacto-decanoate

The title compound (1.24 g) was obtained through reaction in the same manner as in Example 1(1e) using the compound (928 mg) obtained in Example 13(13h).

(13j) Benzyl 6-amino-5,9-anhydro-7-O-[(3R)-3-(decanoyloxy)tetradecanoyl]-2-{ [(3R)-3-(decanoyloxy)tetradecanoyl]amino}-2,3,4,6-tetradeoxy-D-erythro-L-galacto-decanoate

The title compound (960 mg) was obtained through reaction in the same manner as in Example 1(1f) using the compound (1.24 g) obtained in Example 13(13i).

(13k) Benzyl 5,9-anhydro-7-O-[(3R)-3-(decanoyloxy)tetradecanoyl]-2,6-bis{ [(3R)-3-(decanoyloxy)tetradecanoyl]amino}-2,3,4,6-tetradeoxy-D-erythro-L-galacto-decanoate

The title compound (752 mg) was obtained through reaction in the same manner as in Example 1(1g) using the compound (960 mg) obtained in Example 13(13j).

(131) Benzyl 5,9-anhydro-10-O-[tert-butyl(diphenyl)silyl]-7-O-[(3R)-3-(decanoyloxy)tetradecanoyl]-2,6-bis{[(3R)-3-(decanoyloxy)tetradecanoyl]amino}-2,3,4,6-tetradeoxy-D-erythro-L-galacto-decanoate

The title compound (2.28 g) was obtained through reaction in the same manner as in Example 1(1h) using the compound (2.19 g) obtained in Example 13(13k).

(13m) Benzyl 5,9-anhydro-8-O-[bis(benzyloxy)phosphoryl]-10-O-[tert-butyl(diphenyl)silyl]-7-O-[(3R)-3-(decanoyloxy)tetradecanoyl]-2,6-bis{ [(3R)-3-(decanoyloxy)tetradecanoyl]amino}-2,3,4,6-tetradeoxy-D-erythro-L-galacto-decanoate

The title compound (2.62 g) was obtained through reaction in the same manner as in Example 1(1i) using the compound (2.28 g) obtained in Example 13(131).

(13n) Benzyl 5,9-anhydro-8-O-[bis(benzyloxy)phosphoryl]-7-O-[(3R)-3-(decanoyloxy)tetradecanoyl]-2,6-bis{ [(3R)-3-(decanoyloxy)tetradecanoyl]amino}-2,3,4,6-tetradeoxy-D-erythro-L-galacto-decanoate

The title compound (1.94 g) was obtained through reaction in the same manner as in Example 1(1j) using the compound (2.62 g) obtained in Example 13(13m).

(13o) Ammonium 5,9-anhydro-7-O-[(3R)-3-(decanoyloxy)tetradecanoyl]-2,6-bis{ [(3R)-3-(decanoyloxy)tetradecanoyl]amino}-2,3,4,6-tetradeoxy-8-O-phosphono-D-erythro-L-galacto-decanoate

The title compound (73.1 mg) was obtained through reaction in the same manner as in Example 1(1k) using the compound (103 mg) obtained in Example 13(13n).

(Example 14) Ammonium 5,9-anhydro-7-O-[(3R)-3-(decanoyloxy)tetradecanoyl]-2,6-bis{ [(3R)-3-(decanoyloxy)tetradecanoyl]amino}-2,3,4,6-tetradeoxy-10-O-(3-deoxy-α-D-manno-oct-2-ulopyranonosyl)-8-O-phosphono-D-erythro-L-galacto-decanoate

[Formula 26]

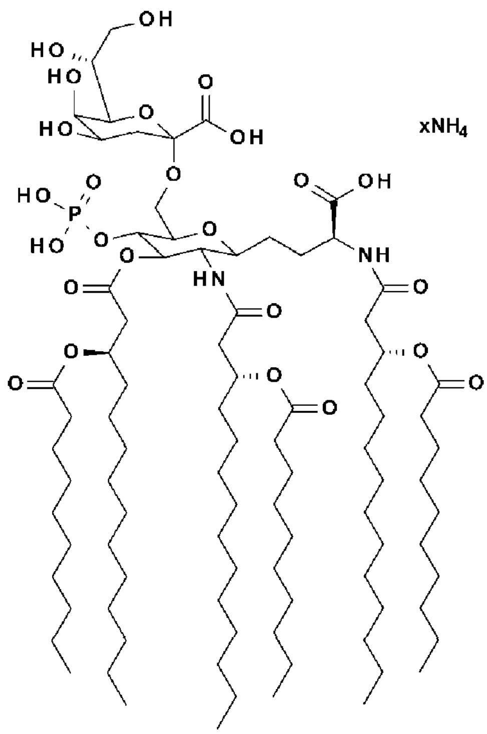

(14a) Benzyl 5,9-anhydro-8-O-[bis(benzyloxy)phosphoryl]-7-O-[(3R)-3-(decanoyloxy)tetradecanoyl]-2,6-bis{ [(3R)-3-(decanoyloxy)tetradecanoyl]amino}-2,3,4,6-tetradeoxy-10-O-(triethylsilyl)-D-erythro-L-galacto-decanoate

The title compound (1.49 g) was obtained through reaction in the same manner as in Example 2(2a) using the

compound (1.40 g) obtained in Example 13(13n).

(14b) Benzyl 5,9-anhydro-10-O-[1-benzyl-7,8-di-O-benzyl-3-deoxy-4,5-0-(1-methylethylidene)-α-D-manno-oct-2-ulopyranonosyl]-8-O-[bis(benzyloxy)phosphoryl]-7-O-[(3R)-3-(decanoyloxy)tetradecanoyl]-2,6-bis{[(3R)-3-(decanoyloxy)tetradecanoyl]amino}-2,3,4,6-tetradeoxy-D-erythro-L-galacto-decanoate

The title compound (1.19 g) was obtained through reaction in the same manner as in Example 2(2b) using the compound (1.49 g) obtained in Example 14 (14a).

(14c) Benzyl 5,9-anhydro-10-O-(1-benzyl-7,8-di-O-benzyl-3-deoxy-α-D-manno-oct-2-ulopyranonosyl)-8-O-[bis(benzyloxy)phosphoryl]-7-O-[(3R)-3-(decanoyloxy)tetradecanoyl]-2,6-bis{ [(3R)-3-(decanoyloxy)tetradecanoyl]amino}-2,3,4,6-tetradeoxy-D-erythro-L-galacto-decanoate

The title compound (1.10 g) was obtained through reaction in the same manner as in Example 2(2c) using the compound (1.19 g) obtained in Example 14 (14b).

(14d) Ammonium 5,9-anhydro-7-O-[(3R)-3-(decanoyloxy)tetradecanoyl]-2,6-bis{ [(3R)-3-(decanoyloxy)tetradecanoyl]amino}-2,3,4,6-tetradeoxy-10-O-(3-deoxy-a-D-manno-oct-2-ulopyranonosyl)-8-O-phosphono-D-erythro-L-galacto-decanoate

The title compound (196 mg) was obtained through reaction in the same manner as in Example 1(1k) using the compound (275 mg) obtained in Example 14 (14c) .

(Example 15) Ammonium 3-deoxy-α-D-manno-oct-2-ulopyranonosyl-(2→4)-3-deoxy-α-D-manno-oct-2-ulopyranonosyl-(2→10)-5,9-anhydro-7-O-[(3R)-3-(decanoyloxy)tetradecanoyl]-2,6-bis{ [(3R)-3-(decanoyloxy)tetradecanoyl]amino}-2,3,4,6-tetradeoxy-8-O-phosphono-D-erythro-L-galacto-decanoate

[Formula 27]

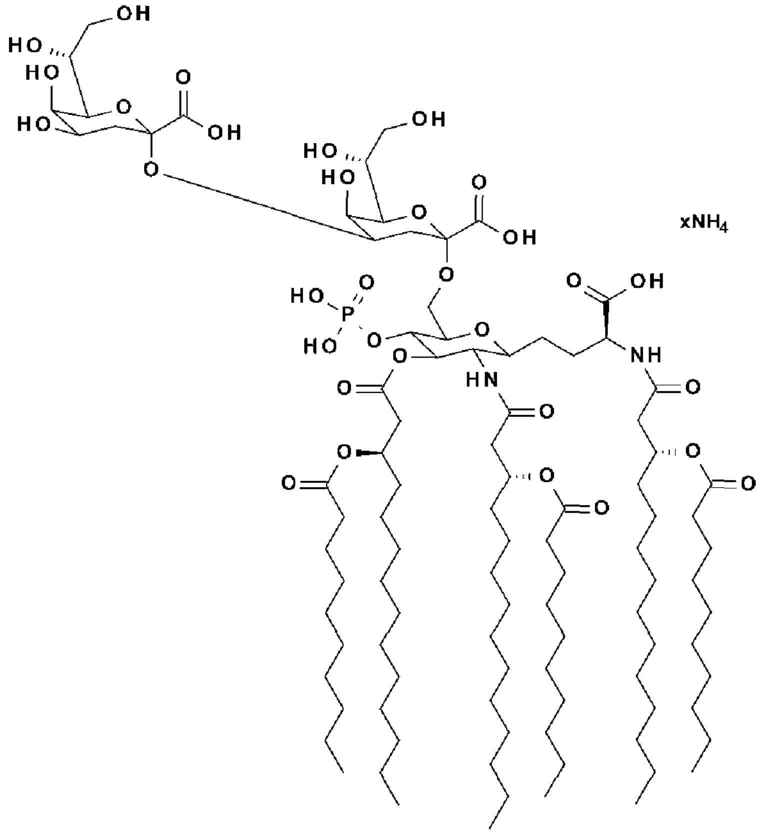

(15a) Benzyl 5,9-anhydro-10-0-[1-benzyl-7,8-di-O-benzyl-3-deoxy-4-O-(triethylsilyl)-α-D-manno-oct-2-ulopyranonosyl]-8-O-[bis(benzyloxy)phosphoryl]-7-O-[(3R)-3-(decanoyloxy)tetradecanoyl]-2,6-bis{ [(3R)-3-(decanoyloxy)tetradecanoyl]amino}-2,3,4,6-tetradeoxy-D-erythro-L-galacto-decanoate

The title compound (554 mg) was obtained through reaction in the same manner as in Example 3(3a) using the compound (541 mg) obtained in Example 14(14c).

(15b) Benzyl 1-benzyl-7,8-di-O-benzyl-3-deoxy-4,5-O-(1-methylethylidene)-α-D-manno-oct-2-ulopyranonosyl-(2→4)-1-benzyl-7,8-di-O-benzyl-3-deoxy-α-D-manno-oct-2-ulopyranonosyl-(2→10)-5,9-anhydro-8-O-[bis(benzyloxy)phosphoryl]-7-O-[(3R)-3-(decanoyloxy)tetradecanoyl]-2,6-bis{ [(3R)-3-(decanoyloxy)tetradecanoyl]amino}-2,3,4,6-tetradeoxy-D-erythro-L-galacto-decanoate

The title compound (422 mg) was obtained through reaction in the same manner as in Example 3(3b) using the compound (554 mg) obtained in Example 15(15a).

(15c) Benzyl 1-benzyl-7,8-di-O-benzyl-3-deoxy-α-D-manno-oct-2-ulopyranonosyl-(2→4)-1-benzyl-7,8-di-O-benzyl-3-deoxy-α-D-manno-oct-2-ulopyranonosyl-(2→10)-5,9-anhydro-8-O-[bis(benzyloxy)phosphoryl]-7-O-[(3R)-3-(decanoyloxy)tetradecanoyl]-2,6-bis{ [(3R)-3-(decanoyloxy)tetradecanoyl]amino}-2,3,4,6-tetradeoxy-D-erythro-L-galacto-decanoate

The title compound (246 mg) was obtained through reaction in the same manner as in Example 3(3c) using the compound (422 mg) obtained in Example 15(15b).

(15d) Ammonium 3-deoxy-α-D-manno-oct-2-ulopyranonosyl-(2→4)-3-deoxy-α-D-manno-oct-2-ulopyranonosyl-(2→10)-5,9-anhydro-7-O-[(3R)-3-(decanoyloxy)tetradecanoyl]-2,6-bis{ [(3R)-3-(decanoyloxy)tetradecanoyl]amino}-2,3,4,6-tetradeoxy-8-O-phosphono-D-erythro-L-galacto-decanoate

The title compound (145 mg) was obtained through reaction in the same manner as in Example 1(1k) using the compound (225 mg) obtained in Example 15(15c).

(Example 16) Ammonium 5,9-anhydro-7-O-[(3R)-3-(decanoyloxy)tetradecanoyl]-2,6-bis{ [(3R)-3-(decanoyloxy)tetradecanoyl]amino}-2,3,4,6,10-pentadeoxy-10-fluoro-8-O-phosphono-D-erythro-L-galacto-decanoate

[Formula 28]

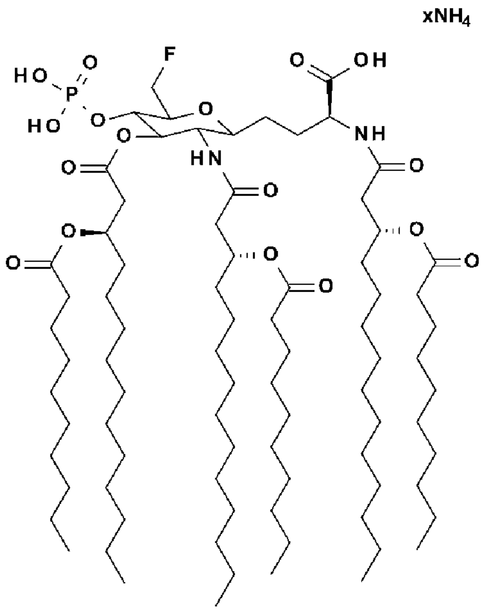

(16a) Benzyl 5,9-anhydro-8-O-[bis(benzyloxy)phosphoryl]-7-O-[(3R)-3-(decanoyloxy)tetradecanoyl]-2,6-bis{ [(3R)-3-(decanoyloxy)tetradecanoyl]amino}-2,3,4,6,10-pentadeoxy-10-fluoro-D-erythro-L-galacto-decanoate

To a solution of the compound (142 mg) obtained in Example 13(13n) in dichloromethane (2 mL), bis (2-methoxyethyl)aminosulfur trifluoride (0.05 mL) was added at 0°C, and the mixture was stirred at the same temperature for 8 hours. The reaction was terminated by the addition of a saturated aqueous solution of sodium bicarbonate to the reaction mixture, followed by extraction with ethyl acetate. The organic layer was washed with saturated saline and then dried over anhydrous sodium sulfate. The drying agent was filtered off, and the filtrate was concentrated under reduced pressure. The residue was purified by silica gel column chromatography [ethyl acetate/methanol] to obtain the title compound (90.0 mg).

(16b) Ammonium 5,9-anhydro-7-O-[(3R)-3-(decanoyloxy)tetradecanoyl]-2,6-bis{ [(3R)-3-(decanoyloxy)tetradecanoyl]amino}-2,3,4,6,10-pentadeoxy-10-fluoro-8-O-phosphono-D-erythro-L-galacto-decanoate

The title compound (56.8 mg) was obtained through reaction in the same manner as in Example 1(1k) using the compound (90.0 mg) obtained in Example 16(16a).

(Example 17) Ammonium 5,9-anhydro-7-O-[(3R)-3-(decyloxy)tetradecanoyl]-2,6-bis{ [(3R)-3-(decyloxy)tetradecanoyl]amino}-2,3,4,6-tetradeoxy-8-O-phosphono-D-erythro-L-galacto-decanoate

[Formula 29]

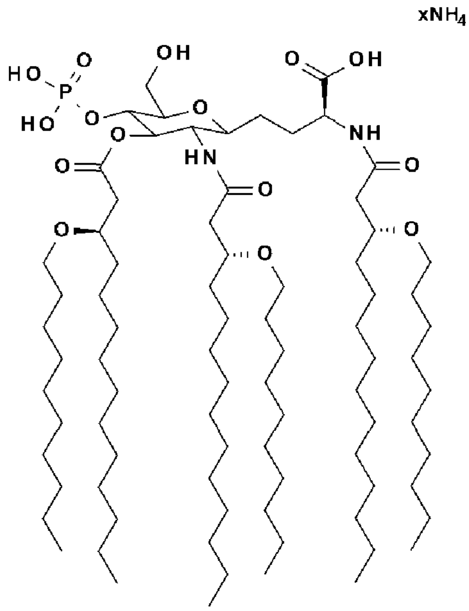

(17a) Benzyl 5,9-anhydro-2-[(tert-butoxycarbonyl)amino]-7-O-[(3R)-3-(decyloxy)tetradecanoyl]-2,3,4,6-tetradeoxy-8,10-O-(1-methylethylidene)-6-{ [(2,2,2-trichloroethoxy)carbonyl]amino}-D-erythro-L-galacto-decanoate

The title compound (2.51 g) was obtained through reaction in the same manner as in Example 1(1a) using the compound (1.47 g) obtained in Example 13(13f) and (3R)-3-(decyloxy)tetradecanoic acid (928 mg) (Bioorganic & Medicinal Chemistry Letters 2008, 18, 5350-5354).

(17b) Benzyl 2-amino-5,9-anhydro-7-O-[(3R)-3-(decyloxy)tetradecanoyl]-2,3,4,6-tetradeoxy-6-{ [(2,2,2-trichloroethoxy)carbonyl]amino}-D-erythro-L-galacto-decanoate

The title compound (444 mg) was obtained through reaction in the same manner as in Example 1(1d) using the compound (2.05 g) obtained in Example 17 (17a).

(17c) Benzyl 5,9-anhydro-7-O-[(3R)-3-(decyloxy)tetradecanoyl]-2-{[(3R)-3-(decyloxy)tetradecanoyl]amino}-2,3,4,6-tetradeoxy-6-{[(2,2,2-trichloroethoxy)carbonyl]amino}-D-erythro-L-galacto-decanoate

The title compound (590 mg) was obtained through reaction in the same manner as in Example 1(1e) using the compound (444 mg) obtained in Example 17(17b) and (3R)-3-(decyloxy)tetradecanoic acid (381 mg).

(17d) Benzyl 6-amino-5,9-anhydro-7-O-[(3R)-3-(decyloxy)tetradecanoyl]-2-{[(3R)-3-(decyloxy)tetradecanoyl]amino}-2,3,4,6-tetradeoxy-D-erythro-L-galacto-decanoate

The title compound (470 mg) was obtained through reaction in the same manner as in Example 1(1f) using the compound (590 mg) obtained in Example 17(17c).

(17e) Benzyl 5,9-anhydro-7-O-[(3R)-3-(decyloxy)tetradecanoyl]-2,6-bis{ [(3R)-3-(decyloxy)tetradecanoyl]amino}-2,3,4,6-tetradeoxy-D-erythro-L-galacto-decanoate

The title compound (570 mg) was obtained through reaction in the same manner as in Example 1(1g) using the compound (470 mg) obtained in Example 17(17d) and (3R)-3-(decyloxy)tetradecanoic acid (332 mg).

(17f) Benzyl 5,9-anhydro-10-O-[(benzyloxy)carbonyl]-7-O-[(3R)-3-(decyloxy)tetradecanoyl]-2,6-bis{[(3R)-3-(decyloxy)tetradecanoyl]amino}-2,3,4,6-tetradeoxy-D-erythro-L-galacto-decanoate

The title compound (560 mg) was obtained through reaction in the same manner as in Example 8(8a) using the compound (570 mg) obtained in Example 17(17e).

(17g) Benzyl 5,9-anhydro-10-O-[(benzyloxy)carbonyl]-8-O-[bis(benzyloxy)phosphoryl]-7-O-[(3R)-3-(decyloxy)tetradecanoyl]-2,6-bis{ [(3R)-3-(decyloxy)tetradecanoyl]amino}-2,3,4,6-tetradeoxy-D-erythro-L-galacto-decanoate

The title compound (221 mg) was obtained through reaction in the same manner as in Example 1(1i) using the compound (190 mg) obtained in Example 17(17f).

(17h) Ammonium 5,9-anhydro-7-O-[(3R)-3-(decyloxy)tetradecanoyl]-2,6-bis{ [(3R)-3-(decyloxy)tetradecanoyl]amino}-2,3,4,6-tetradeoxy-8-O-phosphono-D-erythro-L-galacto-decanoate

The title compound (144 mg) was obtained through reaction in the same manner as in Example 1(1k) using the compound (221 mg) obtained in Example 17(17g).

(Example 18) Sodium (3R)-3-{[(3R)-3-(decanoyloxy)tetradecanoyl]amino}-4-({3-O-[(3R)-3-(decanoyloxy)tetradecanoyl]-2-{ [(3R)-3-(decanoyloxy)tetradecanoyl]amino}-2-deoxy-6-O-(3-deoxy-α-D-manno-oct-2-ulopyranonosyl)-4-O-phosphono-(3-D-glucopyranosyl}oxy)butanoate

To a solution of the compound (104 mg) obtained in Example 2(2d) in tetrahydrofuran (10 mL), triethylamine (0.033 mL) was added dropwise at room temperature, and the mixture was then concentrated under reduced pressure. DOWEX-50W (1.0 g) was converted to Na salt with a 1 N aqueous sodium hydroxide solution (10 mL), then washed with water (10 mL), and charged with an aqueous solution of the compound (10 mL), followed by elution with water (10 mL). The obtained fraction was concentrated under reduced pressure, and the ion exchange operation described above was repeated twice, followed by dilution in water. The solution was lyophilized to obtain the title compound (66.5 mg).

(Example 19) Potassium (3R) -3-{[(3R)-3-(decanoyloxy)tetradecanoyl]amino}-4-({3-O-[(3R)-3-(decanoyloxy)tetradecanoyl]-2-{ [(3R)-3-(decanoyloxy)tetradecanoyl]amino}-2-deoxy-6-O-(3-deoxy-α-D-manno-oct-2-ulopyranonosyl)-4-O-phosphono-β-D-glucopyranosyl}oxy)butanoate

The title compound (52.1 mg) was obtained through reaction in the same manner as in Example 18 using the compound (104 mg) obtained in Example 2(2d) and a 1 N aqueous potassium hydroxide solution (10 mL).

(Example 20) Tetratriethanolamine (3R)-3-{[(3R)-3-(decanoyloxy)tetradecanoyl]amino}-4-({3-O-[(3R)-3-(decanoyloxy)tetradecanoyl]-2-{ [(3R)-3-(decanoyloxy)tetradecanoyl]amino}-2-deoxy-6-O-(3-deoxy-α-D-manno-oct-2-ulopyranonosyl)-4-O-phosphono-β-D-glucopyranosyl}oxy)butanoate

To a solution of the compound (96 mg) obtained in Example 2(2d) in tetrahydrofuran (3 mL), a solution of triethanolamine (0.04 mL) in tetrahydrofuran (0.4 mL) was added dropwise at room temperature, and the mixture was then concentrated under reduced pressure. The obtained residue was washed with acetonitrile, and the obtained residue was dissolved in water. Then, the solution was lyophilized to obtain the title compound (116 mg).

(Example 21) Monomeglumine (3R)-3-{[(3R)-3-(decanoyloxy)tetradecanoyl]amino}-4-({3-O-[(3R)-3-(decanoyloxy)tetradecanoyl]-2-{ [(3R)-3-(decanoyloxy)tetradecanoyl]amino}-2-deoxy-6-O-(3-deoxy-α-D-manno-oct-2-ulopyranonosyl)-4-O-phosphono-β-D-glucopyranosyl}oxy)butanoate

To a solution of the compound (320 mg) obtained in Example 2(2d) in tetrahydrofuran (5 mL), a solution of meglumine (36 mg) in methanol (5 mL) was added dropwise at room temperature, and the mixture was then concentrated under reduced pressure. The obtained residue was washed with acetonitrile and isopropyl alcohol in order, and the obtained residue was dissolved in water. Then, the solution was lyophilized to obtain the title compound (260 mg).

(Example 22) Dimeglumine (3R)-3-{[(3R)-3-(decanoyloxy)tetradecanoyl]amino}-4-({3-O-[(3R)-3-(decanoyloxy)tetradecanoyl]-2-{ [(3R)-3-(decanoyloxy)tetradecanoyl]amino}-2-deoxy-6-O-(3-deoxy-α-D-manno-oct-2-ulopyranonosyl)-4-O-phosphono-β-D-glucopyranosyl}oxy)butanoate

The title compound (336 mg) was obtained through reaction in the same manner as in Example 21 using the compound (300 mg) obtained in Example 2(2d) and meglumine (68 mg) .

(Example 23) Trimeglumine (3R)-3-{[(3R)-3-(decanoyloxy)tetradecanoyl]amino}-4-({3-O-[(3R)-3-(decanoyloxy)tetradecanoyl]-2-{ [(3R)-3-(decanoyloxy)tetradecanoyl]amino}-2-deoxy-6-O-(3-deoxy-α-D-manno-oct-2-ulopyranonosyl)-4-O-phosphono-β-D-glucopyranosyl}oxy)butanoate

The title compound (352 mg) was obtained through reaction in the same manner as in Example 21 using the compound (279 mg) obtained in Example 2(2d) and meglumine (95 mg) .

(Example 24) Tetrameglumine (3R)-3-{[(3R)-3-(decanoyloxy)tetradecanoyl]amino}-4-({3-O-[(3R)-3-(decanoyloxy)tetradecanoyl]-2-{ [(3R)-3-(decanoyloxy)tetradecanoyl]amino}-2-deoxy-6-O-(3-deoxy-α-D-manno-oct-2-ulopyranonosyl)-4-O-phosphono-β-D-glucopyranosyl}oxy)butanoate

The title compound (1.08 g) was obtained through reaction in the same manner as in Example 21 using the compound (780 mg) obtained in Example 2(2d) and meglumine (354 mg) .

[Table 1-1]

| Structure | |
|---|---|
| 1 (1a) | 1(1b) |
| 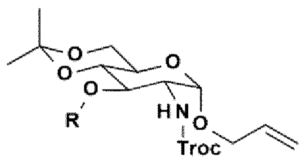 | 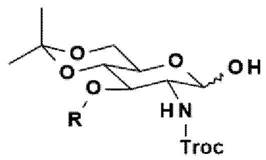 |
| 1(1c) | 1(ld) |
| 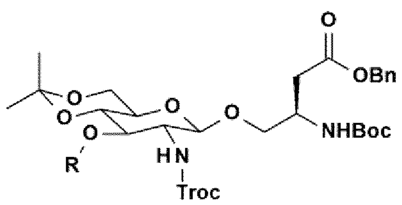 | 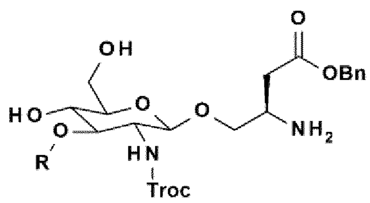 |
| 1(1e) | 1(1f) |
| 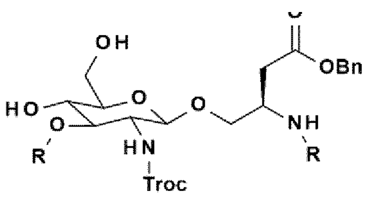 | 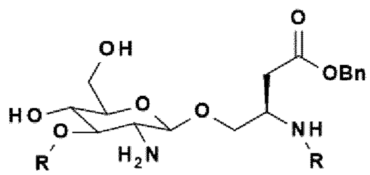 |
| 1(1g) | 1(1h) |
| 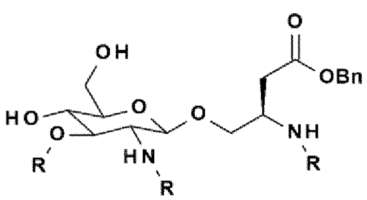 | 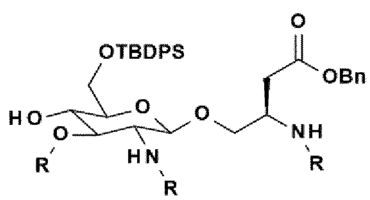 |
| 1(1i) | 1(1j) |

(continued)

| Structure | |
|---|---|
| 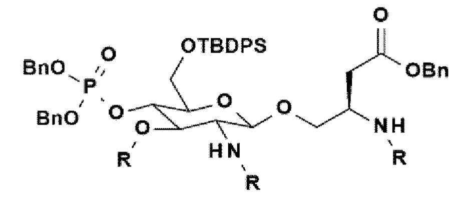 | 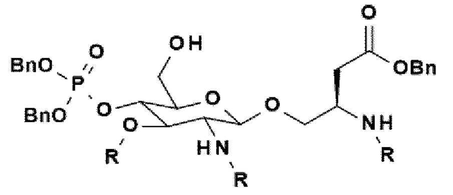 |

[Table 1-2]

| 1(1k) | 2(2a) |
|---|---|
| 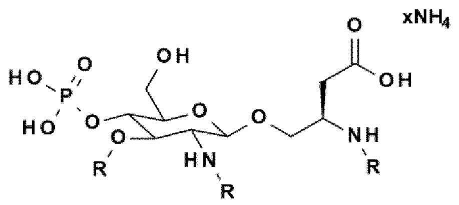 | 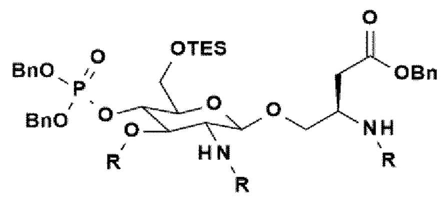 |
| 2(2b) | 2(2c) |
| 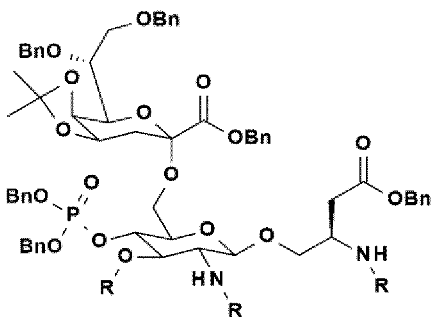 | 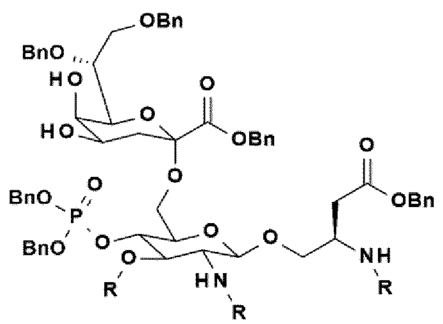 |
| 2(2d) | 2(2e) |
| 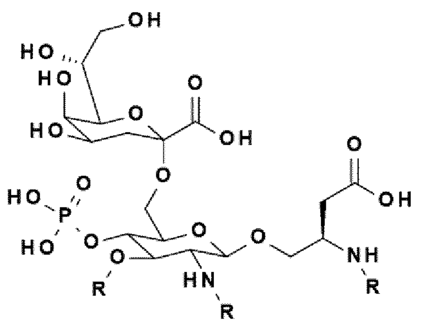 | 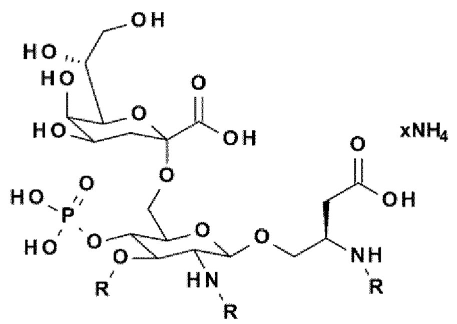 |
| 3(3a) | |

(continued)

| | |
|---|---|
| 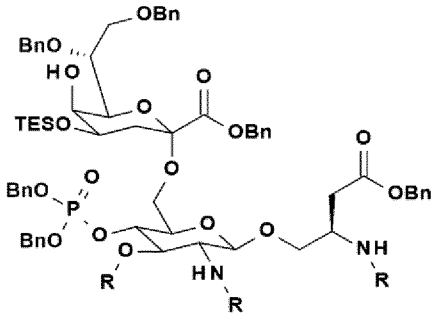 | |

[Table 1-3]

| 3(3b) |
|---|
| 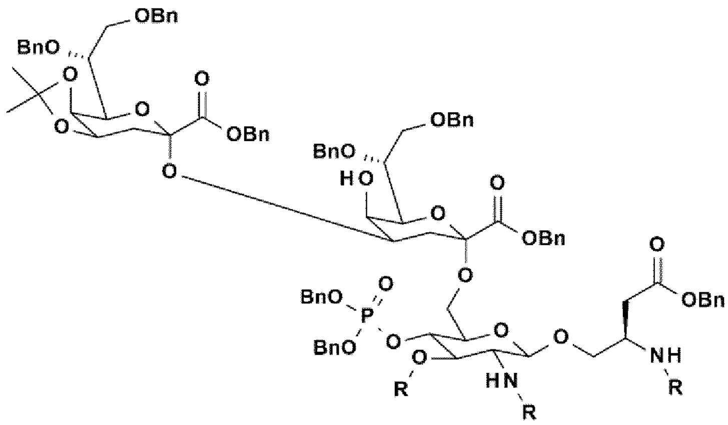 |
| 3(3c) |
| 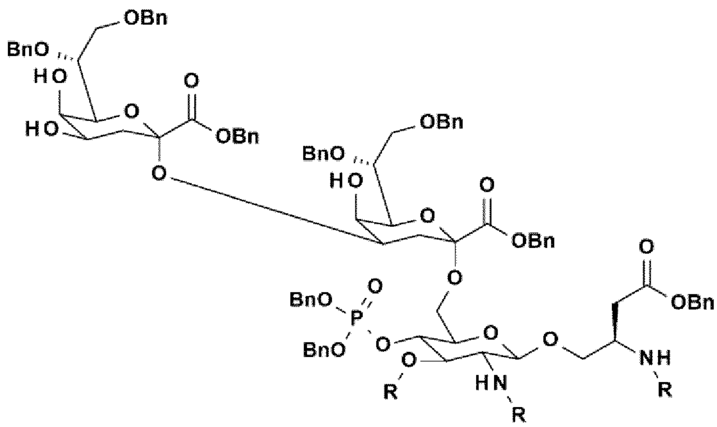 |
| 3(3d) |

(continued)

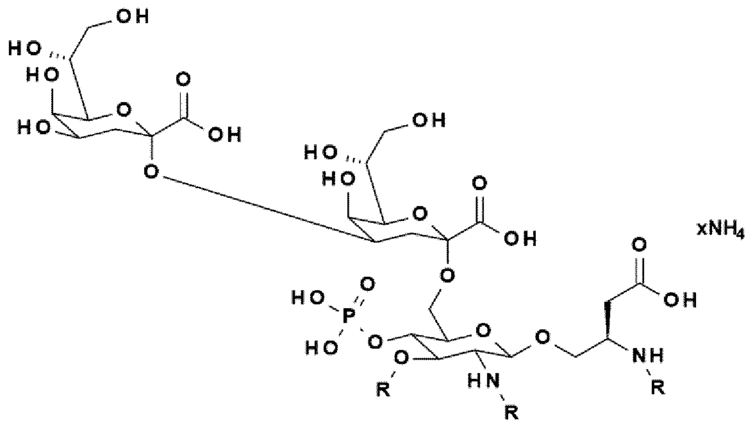

$xNH_4$

[Table 1-4]

| 4(4a) | 4(4b) |
|---|---|
| 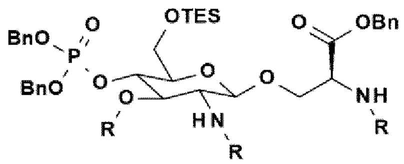 4(4c) | 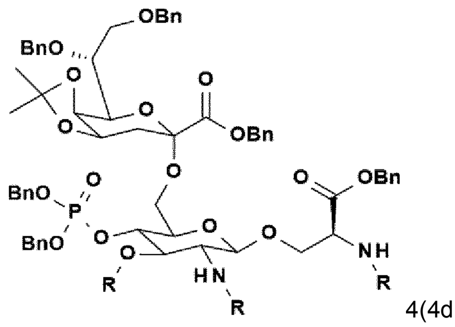 4(4d) |
| 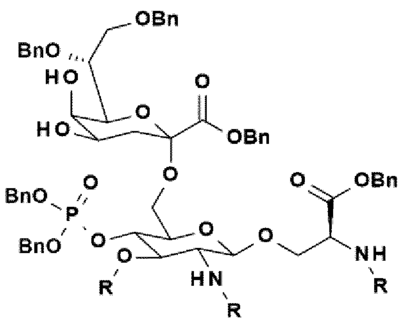 | 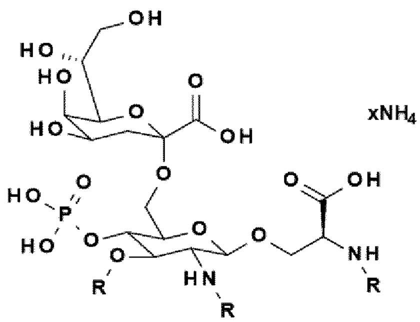 $xNH_4$ |
| 5(5a) | |
| 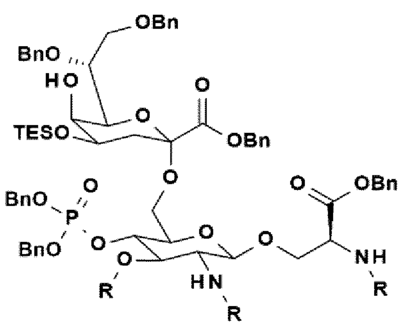 | |

[Table 1-5]

| 5(5b) |
|---|
| 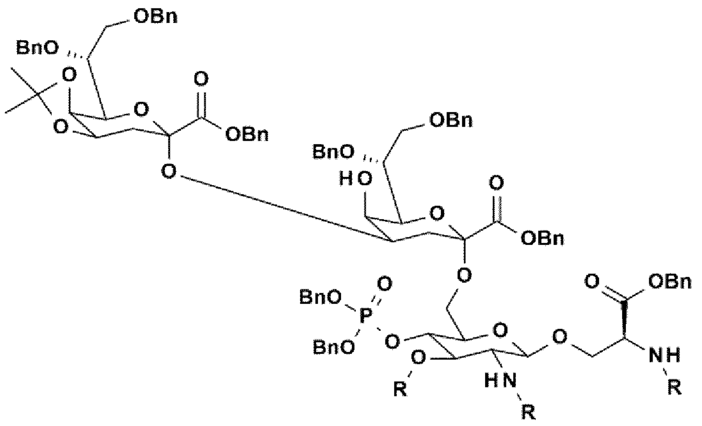 |
| 5(5c) |
| 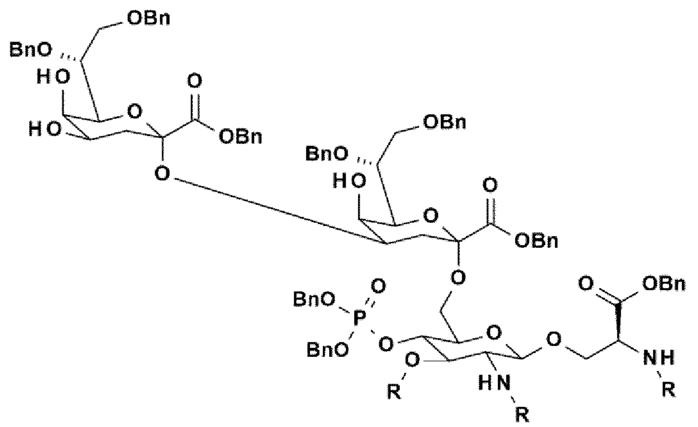 |
| 5(5d) |
| 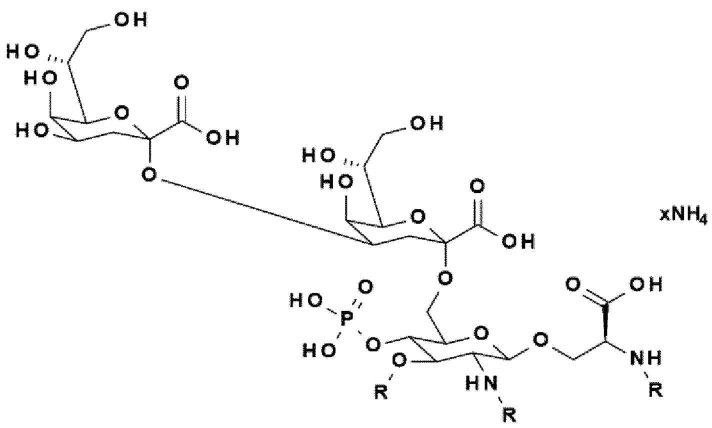 |

[Table 1-6]

| 6(6a-2) | 6(6b) |
|---|---|

(continued)

| | |
|---|---|
| 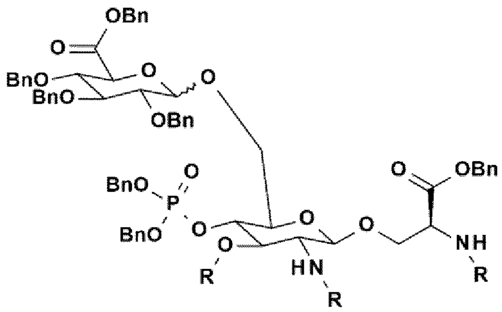 | 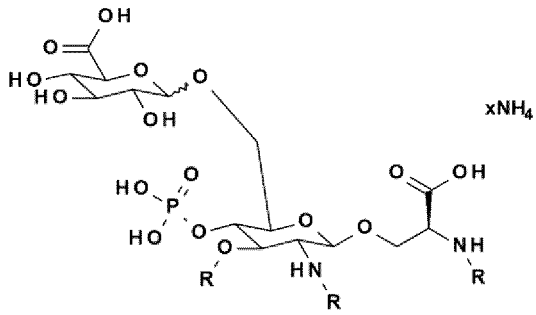 |
| 7(7a) | 7(7b) |
| 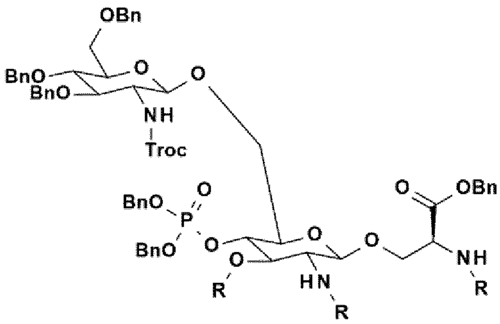 | 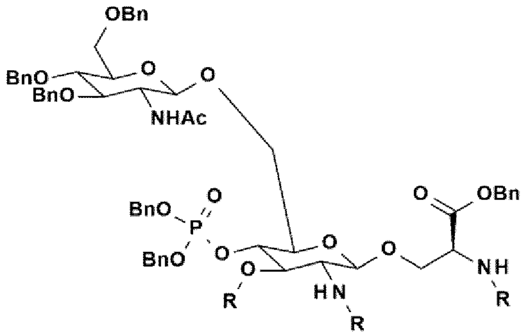 |
| 7(7c) | 8(8a) |
| 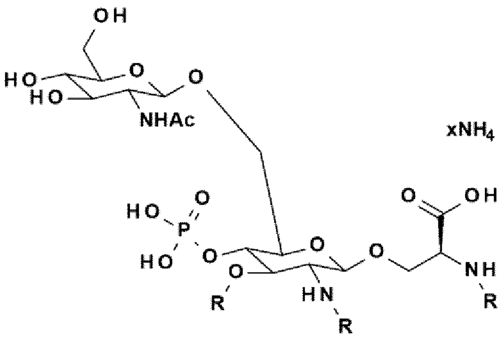 | 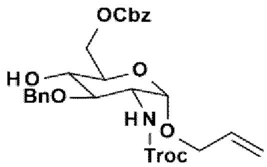 |
| 8(8b) | 8(8c) |
| 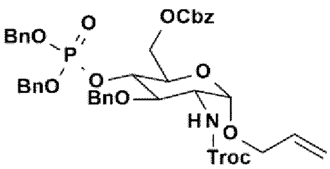 | 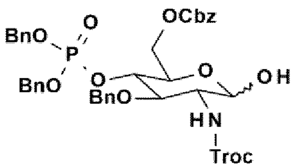 |

[Table 1-7]

| |
|---|
| 8(8d) |

(continued)

| | |
|---|---|
| 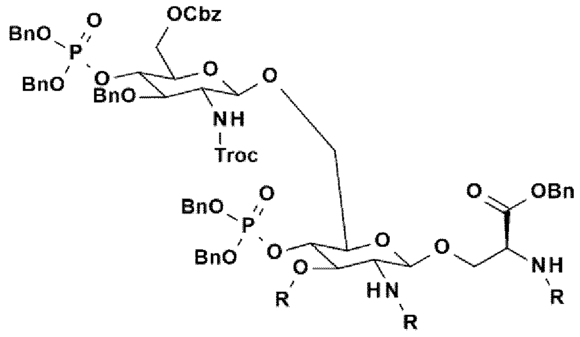 | |
| 8(8e) | |
| 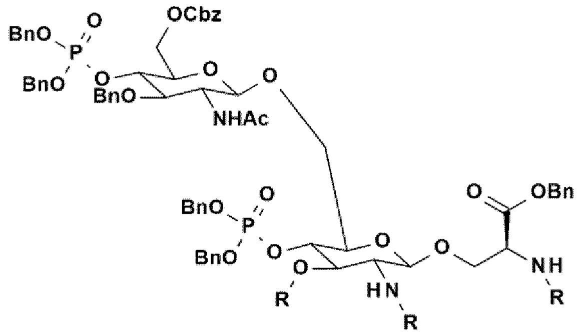 | |
| 8(8f) | |
| 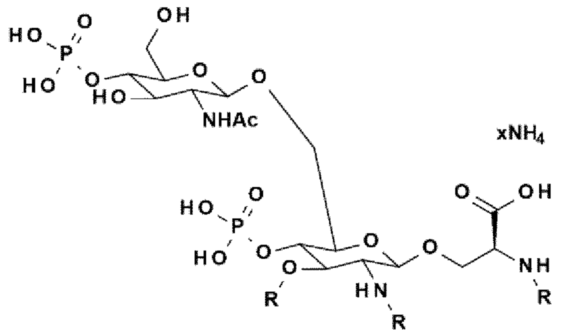 | |
| 9(9a) | 9(9b) |
| 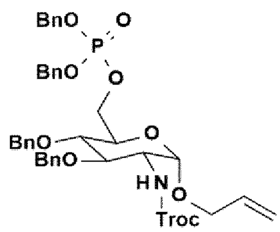 | 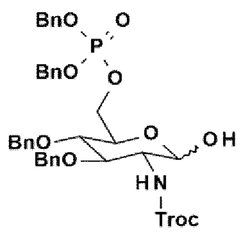 |

[Table 1-8]

| |
|---|
| 9(9c) |

(continued)

| |
|---|
| 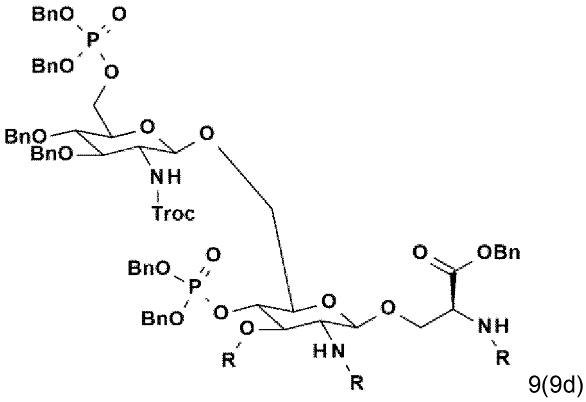 9(9d) |
| 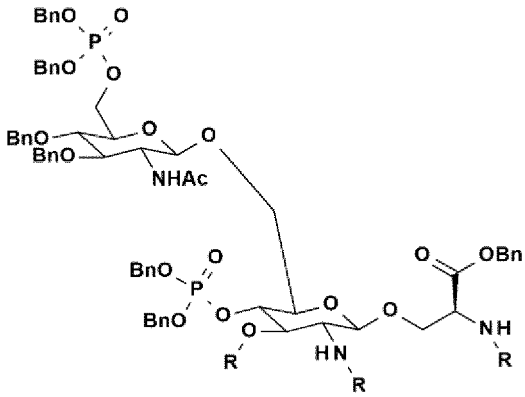 |
| 9(9e) |
| 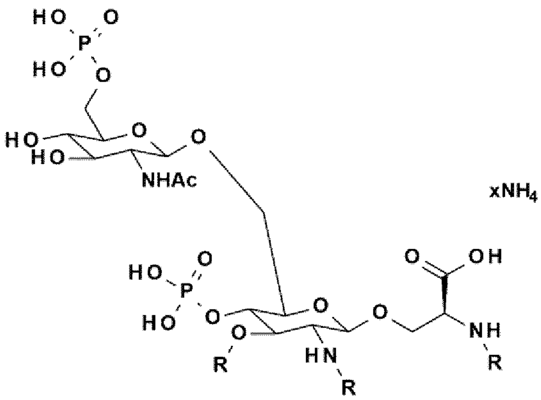 |

[Table 1-9]

| 10(10a) | 10(10b) |
|---|---|
| 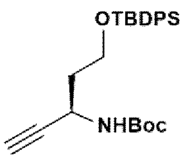 | 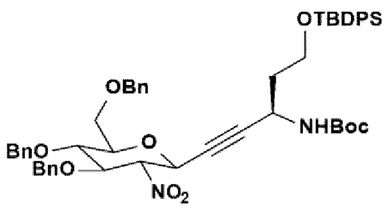 |
| 10(10c) | 10 (10d) |

(continued)

| | |
|---|---|
| 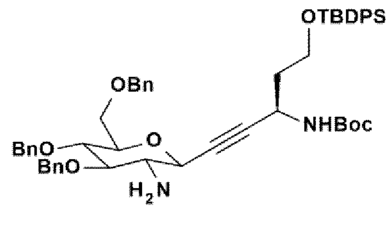 | 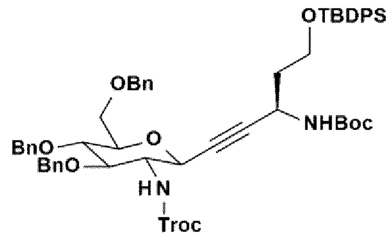 |
| 10(10e) | 10 (1 of) |
| 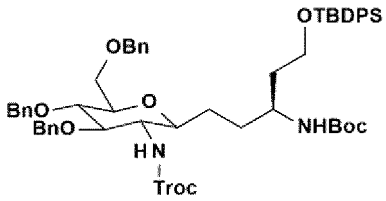 | 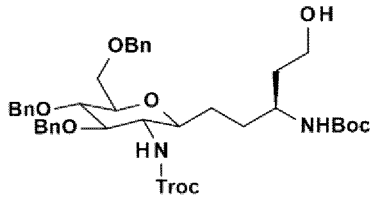 |
| 10(10g) | 10(10h) |
| 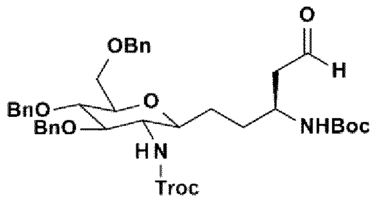 | 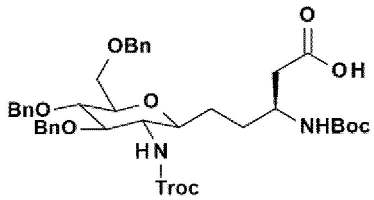 |
| 10(101) | 10(10j) |
| 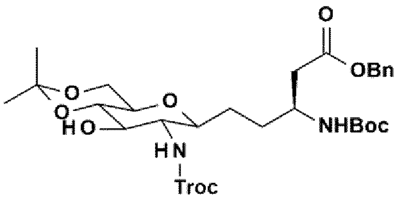 | 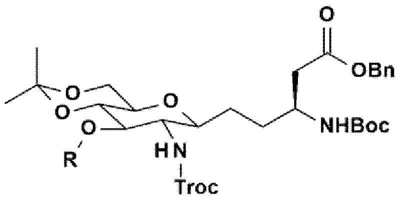 |

[Table 1-10]

| 10(10k) | 10(101) |
|---|---|
| 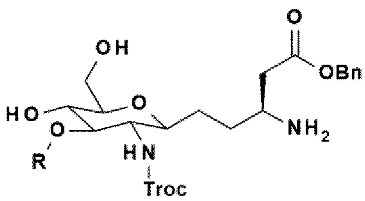 | 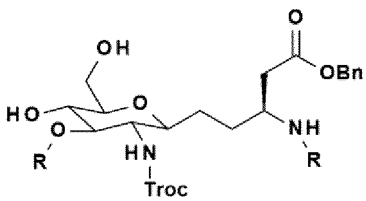 |
| 10(10m) | 10(10n) |
| 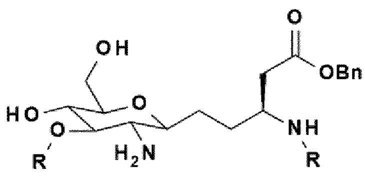 | 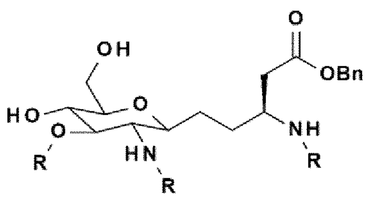 |
| 10(10o) | 10(10p) |

(continued)

| | |
|---|---|
| 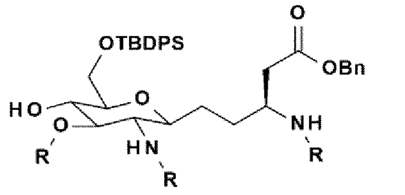 | 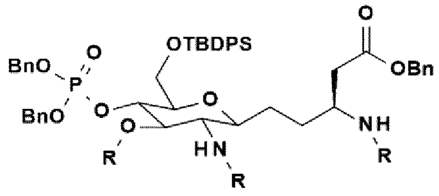 |
| 10(1 0q) | 10(10r) |
| 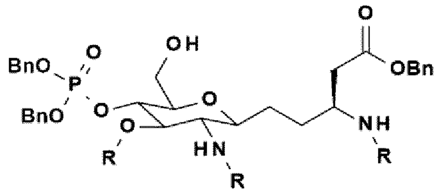 | 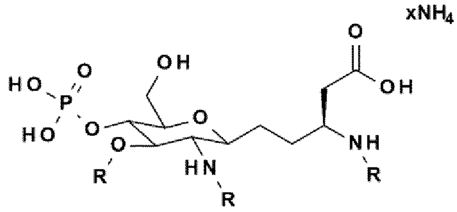 |
| 11(11a) | 11(11b) |
| 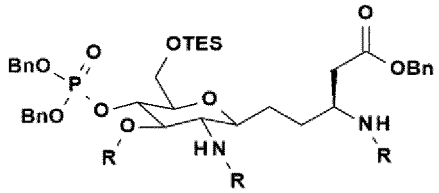 | 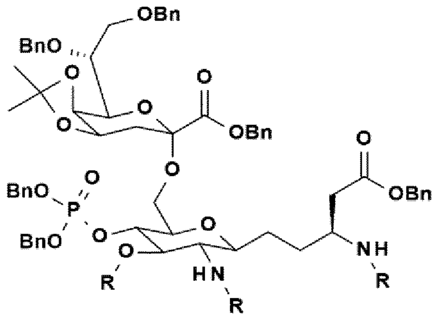 |

[Table 1-11]

| 11(11c) | 11(11d) |
|---|---|
| 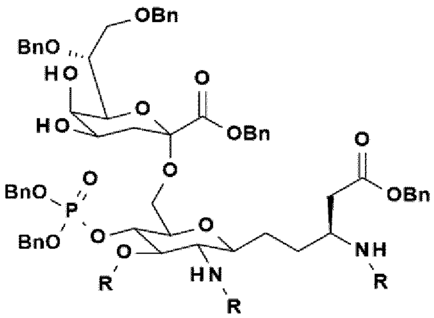 | 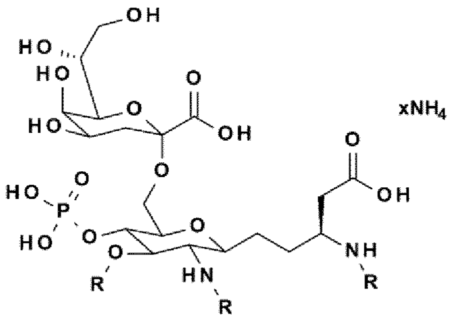 |
| 12(12a) | |
| 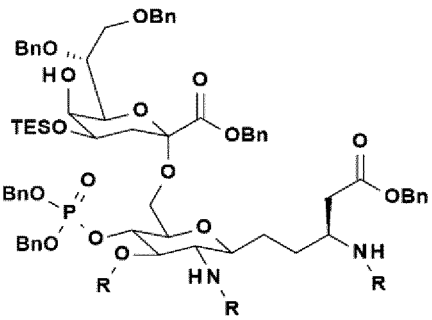 | |

(continued)

| 12(12b) |
| --- |
| 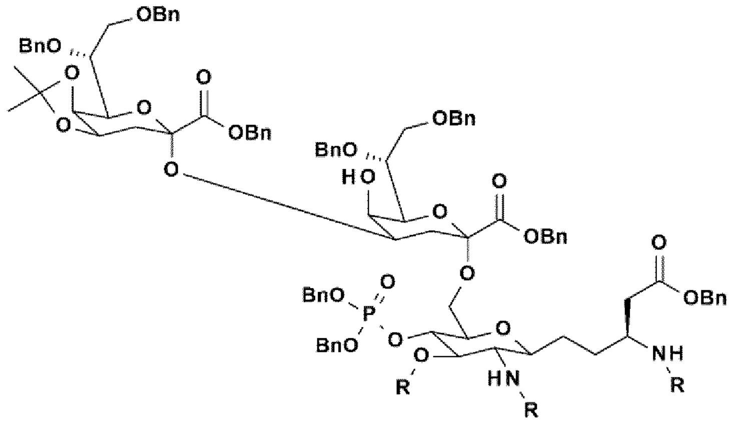 |

[Table 1-12]

| 12(12c) | |
| --- | --- |
| 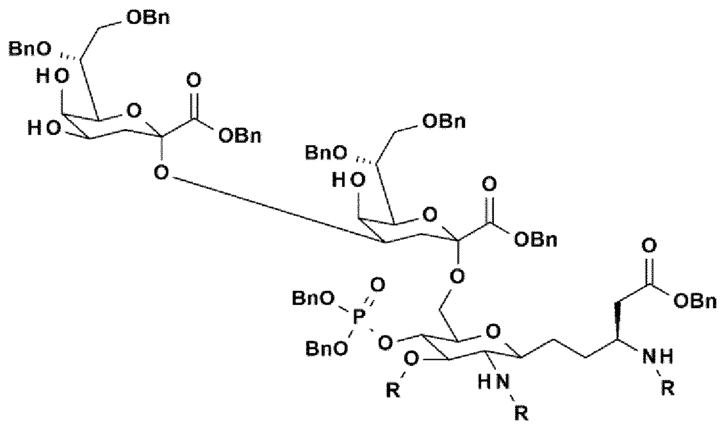 | |
| 12(12d) | |
| 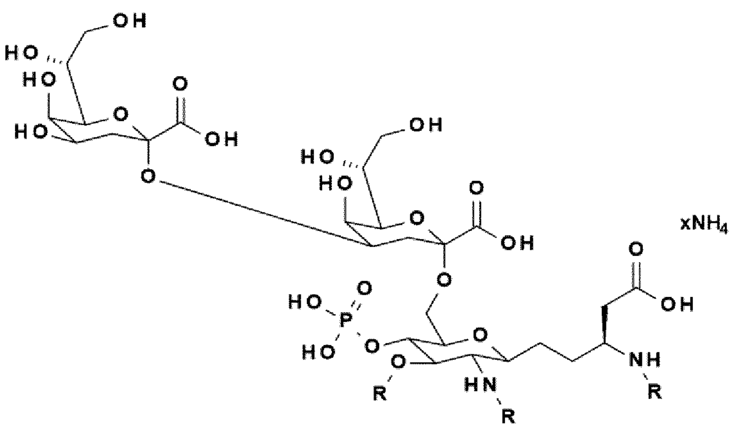 | |
| 13(13a) | 13(13b) |

(continued)

| | |
|---|---|
| 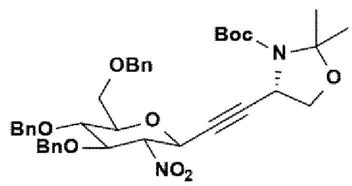 | 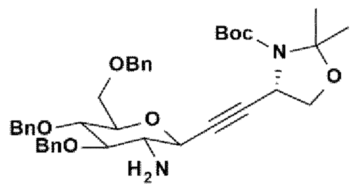 |

[Table 1-13]

| | |
|---|---|
| 13(13c) | 13(13d) |
| 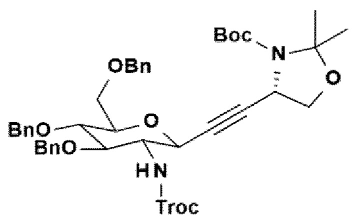 | 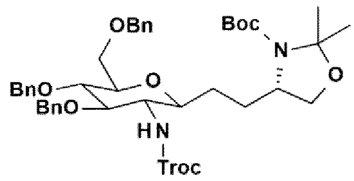 |
| 13(13e) | 13(13f) |
| 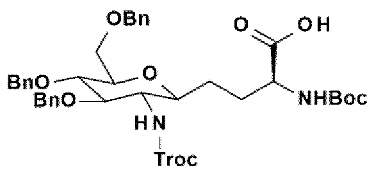 | 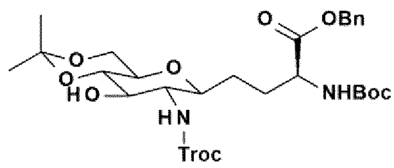 |
| 13(13g) | 13(13h) |
| 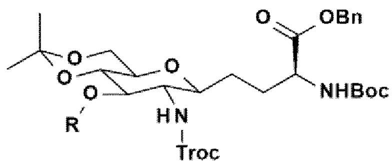 | 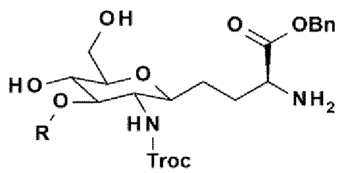 |
| 13(13i) | 13(13j) |
| 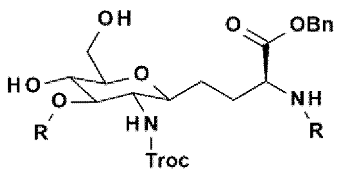 | 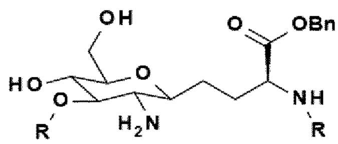 |
| 13(13k) | 13(131) |
| 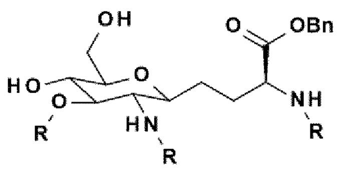 | 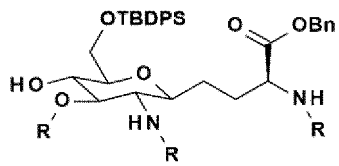 |
| 13(13m) | 13(13n) |

(continued)

| | |
|---|---|
| 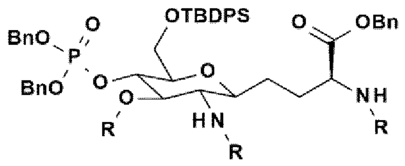 | 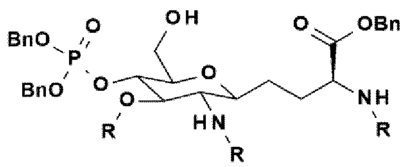 |

[Table 1-14]

| 13(13o) | 14(14a) |
|---|---|
| 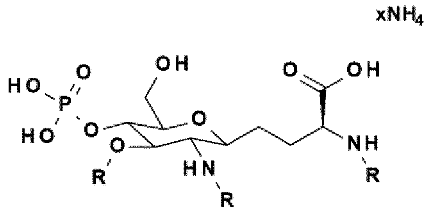 | 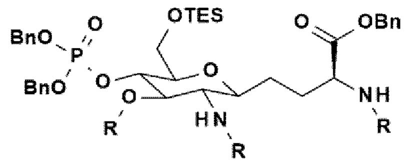 |
| 14(14b) | 14(14c) |
| 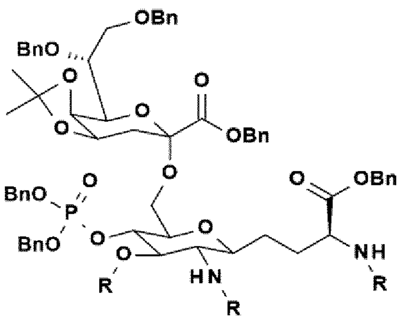 | 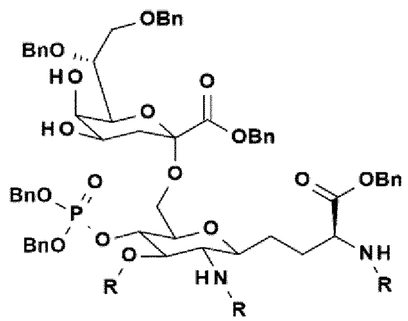 |
| 14(14d) | 15(15a) |
| 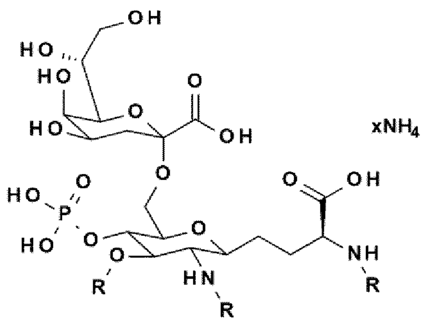 | 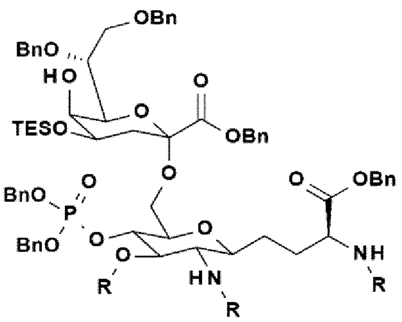 |

[Table 1-15]

| 15(15b) |
|---|

(continued)

| |
|---|
| 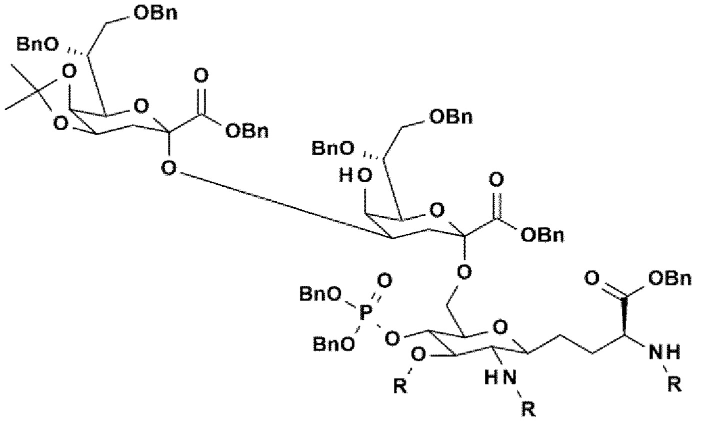 |
| 15(15c) |
| 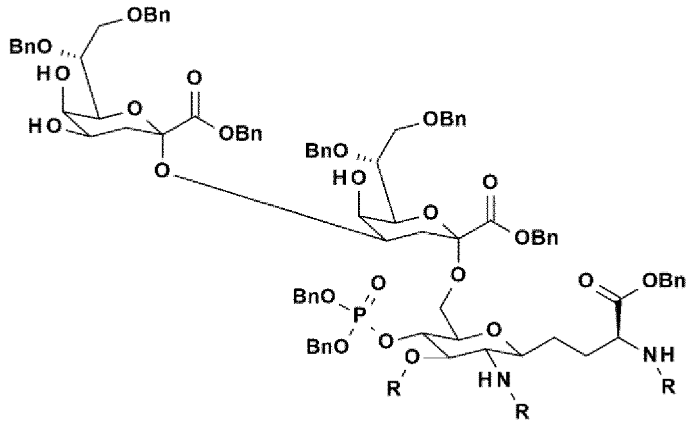 |
| 15(15d) |
| 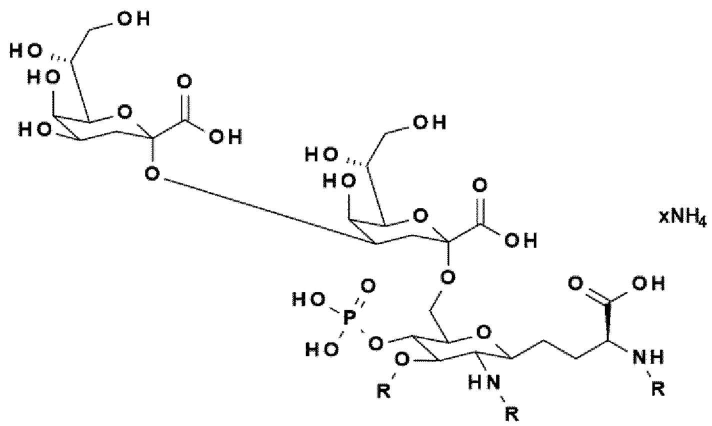 |

[Table 1-16]

| 16(16a) | 16(16b) |
|---|---|

(continued)

| | |
|---|---|
| 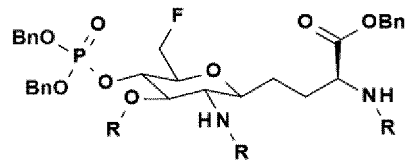 | 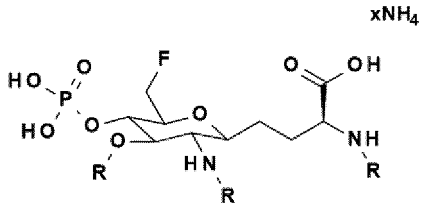 |
| 17(17a) | 17(17b) |
| 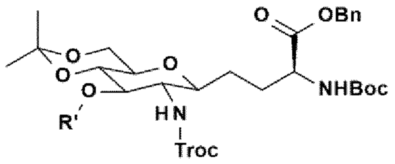 | 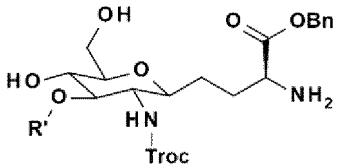 |
| 17(17c) | 17(17d) |
| 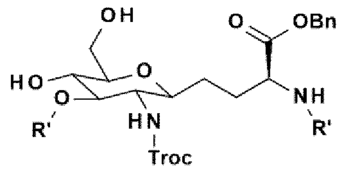 | 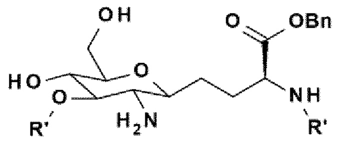 |
| 17(17e) | 17(17f) |
| 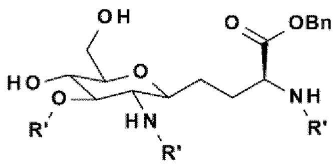 | 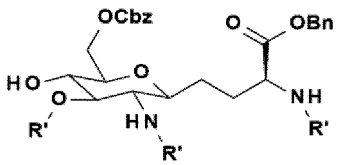 |
| 17(17g) | 17(17h) |
| 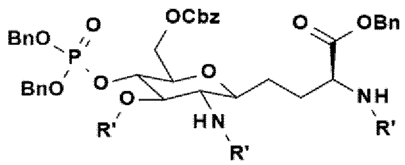 | 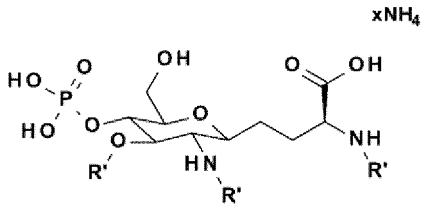 |

[Table 2-1]

| Analytical data |
|---|
| 1(1a) |
| $^1$H-NMR ($CDCl_3$) δ : 5.94-5.81 (1H, m), 5.41-5.11 (5H, m), 4.89 (1H, d, J = 3.9 Hz), 4.71 (2H, dd, J = 21.9, 11.7 Hz), 4.23-4.14 (1H, m), 4.03-3.93 (2H, m), 3.92-3.84 (1H, m), 3.81-3.69 (3H, m), 2.68-2.62 (1H, m), 2.50 (1H, dd, J = 15.5, 6.5 Hz), 2.27 (2H, t, J = 7.4 Hz), 1.62-1.57 (4H, m), 1.49 (3H, s), 1.39 (3H, s), 1.35-1.19 (30H, m), 0.92-0.84 (6H, m). |
| 1(1b) |
| $^1$H-NMR ($CDCl_3$) δ : 5.69 (1H, d, J = 9.8 Hz), 5.34-5.21 (2H, m), 5.20-5.11 (1H, m), 4.78-4.63 (2H, m), 4.04-3.92 (2H, m), 3.91-3.84 (1H, m), 3.80-3.69 (1H, m), 3.43-3.38 (1H, m), 2.70-2.44 (2H, m), 2.30-2.24 (2H, m), 1.64-1.54 (4H, m), 1.49 (3H, s), 1.39 (3H, s), 1.33-1.19 (30H, m), 0.91-0.84 (6H, m) |
| 1(1c) |

(continued)

| Analytical data |
|---|
| $^1$H-NMR ($CDCl_3$) δ : 7.43-7.32 (5H, m), 5.30-4.97 (6H, m), 4.77 (1H, d, J = 12.1 Hz), 4.63 (1H, d, J = 11.7 Hz), 4.39 (1H, d, J = 8.2 Hz), 4.14 (1H, brs), 3.97-3.86 (2H, m), 3.79-3.46 (4H, m), 3.24 (1H, td, J = 9.8, 5.5 Hz), 2.67-2.58 (3H, m), 2.52 (1H, dd, J = 15.3, 5.9 Hz), 2.28 (2H, t, J = 7.6 Hz), 1.65-1.53 (4H, m), 1.46 (3H, s), 1.43 (9H, s), 1.37 (3H, s), 1.34-1.20 (30H, m), 0.92-0.84 (6H, m). |
| 1(1d) |
| $^1$H-NMR ($CDCl_3$) δ : 7.41-7.30 (5H, m), 5.64 (1H, d, J = 8.6 Hz), 5.17-5.06 (3H, m), 4.96 (1H, t, J = 9.8 Hz), 4.72 (1H, d, J = 12.1 Hz), 4.65 (1H, d, J = 12.1 Hz), 4.48 (1H, d, J = 8.6 Hz), 3.92 (1H, dd, J = 11.9, 3.3 Hz), 3.82-3.73 (2H, m), 3.67-3.59 (2H, m), 3.53-3.47 (1H, m), 3.46-3.38 (2H, m), 2.60-2.51 (3H, m), 2.45-2.35 (1H, m), 2.33-2.26 (3H, m), 1.68-1.50 (4H, m), 1.35-1.19 (30H, m), 0.91-0.84 (6H, m). |
| 1(1e) |
| $^1$H-NMR ($CDCl_3$) δ : 7.41-7.31 (5H, m), 6.42 (1H, d, J = 8.6 Hz), 5.75 (1H, d, J = 7.4 Hz), 5.18-5.01 (4H, m), 4.96-4.88 (1H, m), 4.76 (1H, d, J = 11.7 Hz), 4.64 (1H, d, J = 11.7 Hz), 4.51-4.37 (2H, m), 3.95-3.83 (2H, m), 3.82-3.72 (1H, m), 3.68-3.53 (3H, m), 3.45 (1H, d, J = 3.9 Hz), 3.43-3.36 (1H, m), 2.68-2.47 (4H, m), 2.45-2.33 (2H, m), 2.33-2.26 (4H, m), 1.69-1.52 (8H, m), 1.37-1.15 (60H, m), 0.92-0.83 (12H, m). |
| 1(1f) |
| $^1$H-NMR ($CDCl_3$) δ : 7.40-7.30 (5H, m), 6.95 (1H, brs), 5.26-5.06 (6H m), 4.74 (1H, brs), 4.53 (1H, brs), 3.97-3.83 (2H, m), 3.82-3.69 (2H, m), 3.56-3.44 (2H, m), 2.97-2.88 (1H, m), 2.72-2.56 (4H, m), 2.49-2.42 (2H, m), 2.33-2.24 (4H, m), 1.66-1.51 (8H, m), 1.36-1.19 (60H, m), 0.91-0.84 (12H, m). |
| 1(1g) |
| $^1$H-NMR ($CDCl_3$) δ : 7.41-7.30 (5H, m), 6.61 (1H, d, J = 8.2 Hz), 6.09 (1H, d, J = 8.6 Hz), 5.14-5.02 (5H, m), 4.88 (1H, dd, J = 10.6, 9.0 Hz), 4.48-4.37 (2H, m), 3.93-3.70 (4H, m), 3.63-3.55 (2H, m), 3.43-3.34 (2H, m), 2.73-2.67 (1H, m), 2.64 (2H, d, J = 6.3 Hz), 2.58-2.35 (5H, m), 2.34-2.23 (7H, m), 1.68-1.50 (12H, m), 1.35-1.19 (90H, m), 0.92-0.84 (18H, m). |
| 1(1h) |
| $^1$H-NMR ($CDCl_3$) δ : 7.70-7.64 (4H, m), 7.46-7.28 (11H, m), 6.66 (1H, d, J = 8.6 Hz), 6.20 (1H, d, J = 8.2 Hz), 5.19-4.97 (5H, m), 4.91 (1H, dd, J = 10.6, 9.0 Hz), 4.47-4.35 (2H, m), 3.96-3.82 (4H, m), 3_75 (1H, td, J = 9.3, 2.9 Hz), 3.54 (1H, dd, J = 10.2, 4.3 Hz), 3.43-3.35 (2H, m), 2.66-2.22 (14H, m), 1.67-1.50 (12H, m), 1.37-1.18 (90H, m), 1.03 (9H, s), 0.91-0.83 (18H, m). |
| 1(1i) |
| $^1$H-NMR ($CDCl_3$) δ : 7.67-7.61 (4H, m), 7.42-7.09 (21H, m), 6.73 (1H, d, J = 8.2 Hz), 6.42 (1H, d, J = 7.8 Hz), 5.24 (1H, dd, J = 10.6, 9.0 Hz), 5.20-4.95 (5H, m), 4.88-4.66 (5H, m), 4.50-4.40 (2H, m), 3.99-3.79 (3H, m), 3.74-3.64 (1H, m), 3.61-3.48 (2H, m), 2.67-2.15 (14H, m), 1.69-1.49 (12H, m), 1.35-1.18 (90H, m), 1.03 (9H, s), 0.92-0.83 (18H, m). |
| 1(1j) |
| $^1$H-NMR ($CDCl_3$) δ : 7.39-7.27 (15H, m), 6.68 (1H, d, J = 8.2 Hz), 6.28 (1H, d, J = 7.8 Hz), 5.25 (1H, dd, J = 10.6, 9.0 Hz), 5.16-4.89 (9H, m), 4.67 (1H, d, J = 8.2 Hz), 4.46-4.36 (2H, m), 3.86 (1H, dd, J = 10.6, 4.3 Hz), 3.80-3.58 (5H, m), 3.41-3.35 (1H, m), 2.64 (2H, d, J = 6.3 Hz), 2.50-2.17 (12H, m), 1.68-1.49 (12H, m), 1.33-1.16 (90H, m), 0.92-0.83 (18H, m). |

[Table 2-2]

| I(lk) |
|---|
| $^1$H-NMR ($CD_3OD$-$CDCl_3$) δ : 7.60 (1H, d, J = 9.0 Hz), 5.26-5.13 (3H, m), 5.09 (1H, t, J = 9.8 Hz), 4.50-4.43 (1H, m), 4.33 (1H, brs), 4.24 (1H, q, J = 9.5 Hz), 3.97 (1H, d, J = 12.5 Hz), 3.89-3.79 (2H, m), 3.72 (1H, d, J = 12.9 Hz), 3.56 (1H, dd, J = 10.2, 5.1 Hz), 3.37-3.31 (1H, m), 2.70 (1H, dd, J = 16.6, 7.2 Hz), 2.61 (2H, dd, J = 16.4, 5.9 Hz), 2.53-2.26 (11H, m), 1.68-1.52 (12H, m), 1.38-1.21 (90H, m), 0.93-0.84 (18H, m).<br>HRMS (ESI Neg) : $C_{82}H_{153}N_2O_{19}P$ ($[M-2H]^{2-}$) Found 749.5328; Calcd for $C_{82}H_{151}N_2O_{19}P$ 749.5330. |

(continued)

| 2(2a) |
|---|
| $^1$H-NMR ($CDCl_3$) δ : 7.39-7.24 (15H, m), 6.68 (1H, d, J = 8.2 Hz), 6.34 (1H, d, J = 7.8 Hz), 5.23(1H, dd, J = 10.6, 8.6 Hz), 5.19-5.04 (5H, m), 4.98 (2H, d, J = 8.2 Hz), 4.92 (2H, d, J = 7.8 Hz), 4.67 (1H, d, J = 8.2 Hz), 4.46-4.32 (2H, m), 3.94-3.86 (2H, m), 3.75-3.56 (3H, m), 3.48-3.41 (1H, m), 2.61 (2H, d, J = 7.0 Hz), 2.57-2.17 (12H, m), 1.68-1.43 (12H, m), 1.35-1.16 (90H, m), 0.94-0.83 (27H, m), 0.58-0.48 (6H, m). |
| 2(2b) |
| $^1$H-NMR ($CDCl_3$) δ : 7.36-7.19 (30H, m), 6.64 (1H, d, J = 8.2 Hz), 6.33 (1H, d, J = 7.8 Hz), 5.19-5.00 (8H, m), 4.96-4.90 (2H, m), 4.87 (2H, d, J = 7.8 Hz), 4.73 (1H, d, J = 11.3 Hz), 4.64-4.46 (4H, m), 4.43-4.29 (3H, m), 4.11 (1H, q, J = 9.1 Hz), 4.00-3.94 (1H, m), 3.87-3.77 (4H, m), 3.68-3.48 (4H, m), 3.44-3.37 (1H, m), 2.61-2.16 (15H, m), 1.90 (1H, dd, J = 14.9, 3.9 Hz), 1.65-1.49 (12H, m), 1.37-1.17 (90H, m), 1.33 (3H, s), 1.29 (3H, s), 0.93-0.83 (18H, m). |
| 2(2c) |
| $^1$H-NMR ($CDCl_3$) δ : 7.38-7.19 (30H, m), 6.62 (1H, d, J = 8.2 Hz), 6.24 (1H, d, J = 7.8 Hz), 5.22-4.84 (12H, m), 4.68 (2H, s), 4.64 (1H, d, J = 8.2 Hz), 4.50 (2H, dd, J = 15.7, 12.1 Hz), 4.37-4.28 (1H, m), 4.08 (1H, brs), 4.04-3.88 (5H, m), 3.82-3.73 (2H, m), 3.70-3.39 (5H, m), 3.02 (1H, d, J = 7.0 Hz), 2.84 (1H, d, J = 2.3 Hz), 2.65 (1H, dd, J = 16.2, 6.1 Hz), 2.56-2.16 (13H, m), 2.11-1.96 (2H, m), 1.65-1.50 (12H, m), 1.35-1.18 (90H, m), 0.91-0.84 (18H, m). |
| 2 (2d) |
| $^1$H-NMR ($CD_3OD$-$CDCl_3$-$D_2O$) δ : 5.27-5.09 (4H, m), 4.52 (1H, d, J = 7.9 Hz), 4.38-4.27 (1H, m), 4.22-3.53 (13H, m), 2.75-2.23 (14H, m), 2.15-2.03 (1H, m), 1.96-1.82 (1H, m), 1.72-1.50 (12H, m), 1.41-1.16 (90H, m), 0.97-0.80 (18H, m).<br>HRMS (ESI Neg) : $C_{90}H_{165})N_2O_{26}P$ ([M-2H]$^{2-}$) Found 860.0622; Calcd for $C_{90}H_{163}N_2O_{26}P$ 860.0638 . EA : Anal. Calcd for $C_{90}H_{16S}N_2O_{26}P.4H_2O$ : C, 60.24; H, 9.72; N, 1.56; P, 1.73. Found : C, 60.50; H, 9.72; N, 1.77; P, 1.51. |
| 2(2e) |
| $^1$H-NMR ($CD_8OD$-$CDCl_3$-$D_2O$) δ : 5.24-5.04 (4H, m), 4.49 (1H, d, J = 7.9 Hz), 4.45-4.25 (1H, m), 4.05-3.46 (13H, m), 2.72-2.25 (14H, m), 2.03 (1H, brs), 1.75 (1H, brs), 1.67-1.48 (12H, m), 1.48-1.10 (90H, m), 0.94-0.83 (18H, m).<br>HRMS (ESI Neg) : $C_{90}H_{165}N_2O_{26}P$ ([M-3H]$^{3-}$) Found 572.7062; Calcd for $C_{90}H_{162}N_2O_{26}P$ 572.7057. |
| 3(3a) |
| $^1$H-NMR ($CDCl_3$) δ: 7.35-7.17 (30H, m), 6.62 (1H, d, J = 8.2 Hz), 6.35 (1H, d, J = 7.4 Hz), 5.24-4.98 (8H, m), 4.97-4.87 (2H, m), 4.85 (2H, d, J = 7.4 Hz), 4.75-4.63 (3H, m), 4.50 (2H, dd, J = 21.9, 12.1 Hz), 4.40-4.32 (1H, m), 4.19-4.12 (1H, m), 4.06-3.84 (6H, m), 3.79 (1H, dd, J = 10.8, 2.2 Hz), 3.71-3.49 (4H, m), 3.46-3.37 (1H, m), 2.51-2.16 (14H, m), 2.03-1.92 (2H, m), 1.67-1.34 (12H, m), 1.34-1.15 (90H, m), 0.98-0.83 (27H, m), 0.62-0.56 (6H, m). |
| 3(3b) |
| $^1$H-NMR ($CDCl_3$) δ: 7.35-7.12 (45H, m), 6.51 (1H, d, J = 8.6 Hz), 6.18 (1H, d, J = 7.8 Hz), 5.22-5.06 (5H, m), 5.04-4.85 (8H, m), 4.82 (1H, d, J = 12.5 Hz), 4.69-4.37 (13H, m), 4.05 (1H, d, J = 9.0 Hz), 3.98-3.78 (8H, m), 3.73-3.53 (6H, m), 3.49-3.40 (1H, m), 2.91 (1H, dd, J = 15.5, 3.3 Hz), 2.62-2.16 (15H, m), 2.15-2.06 (2H, m), 1.88-1.79 (1H, m), 1.65-1.41 (12H, m), 1.35-1.16 (96H, m), 0.92-0.83 (18H, m). |
| 3(3c) |
| $^1$H-NMR ($CDCl_3$) δ: 7.36-7.12 (45H, m), 6.62 (1H, d, J = 8.6 Hz), 6.23 (1H, d, J = 7.4 Hz), 5.20-4.85 (12H, m), 4.83 (2H, d, J = 7.8 Hz), 4.73-4.54 (6H, m), 4.51-4.42 (4H, m), 4.30-4.23 (1H, m), 4.05-3.81 (10H, m), 3.80-3.71 (2H, m), 3.70-3.59 (3H, m), 3.54 (1H, dd, J = 10.4, 4.1 Hz), 3.50-3.35 (3H, m), 2.69 (1H, d, J = 2.0 Hz), 2.52 (2H, d, J = 6.3 Hz), 2.50-2.06 (16H, m), 1.93 (1H, t, J = 12.3 Hz), 1.66-1.38 (12H, m), 1.35-1.17 (90H, m), 0.92-0.83 (18H, m). |

[Table 2-3]

| 3(3d) |
|---|

(continued)

| |
|---|
| $^1$H-NMR ($CD_3OD$-$CDCl_3$-DaO) δ : 5.25-5.13 (3H, m), 5.12-5.06 (1H, m), 4.54 (1H, d, J = 8.2 Hz), 4.31 (1H, brs), 4.13-3.56 (18H, m), 3.48 (1H, brs), 2.69 (1H, dd, J = 16.2, 6.5 Hz), 2.65-2.42 (6H, m), 2.39 (1H, dd, J = 14.9, 5.5 Hz), 2.34-2.25 (6H, m), 2.09 (1H, brs), 1.99 (1H, brs), 1.87 (1H, brs), 1.74 (1H, brs), 1.67-1.50 (12H, m), 1.38-1.20 (90H, m), 0.94-0.84 (18H, m). HRMS (ESI Neg) : $C_{98}H_{177}N_2O_{33}P$ ($[M-3H]^{3-}$) Found 646.3923; Calcd for $C_{98}H_{174}N_2O_{33}P$ 646.3929. |
| 4(4a) |
| $^1$H-NMR ($CDCl_3$) δ : 7.39-7.23 (15H, m), 7.16 (1H, d, J = 7.8 Hz), 6.41(1H, d, J = 7.0 Hz), 5.37-5.28 (2H, m), 5.24-5.14 (4H, m), 5.03-4.95 (3H, m), 4.94-4.88 (2H, m), 4.76-4.69 (1H, m), 4.42-4,31 (1H, m), 4,29-4,22 (1H, m), 3.91 (1H, d, J = 11.7 Hz), 3.83 (1H, d, J = 11.3 Hz), 3.71 (1H, dd, J = 11.2, 4.1 Hz), 3.50-3.39 (2H, m), 2.69 (1H, dd, J = 14.9, 6.3 Hz), 2.59-2.19 (11H, m), 1.72-1.44 (12H, m), 1.37-1.15 (90H, m), 0.95-0.81 (27H, m), 0.60-0.47 (6H, m). |
| 4(4b) |
| $^1$H-NMR ($CDCl_3$) δ = 7.36-7.19 (30H, m), 7.14 (1H, d, J = 8.2 Hz), 6.35 (1H, d, J = 7.4 Hz), 5.27-5.04 (7H, m), 5.03-4.82 (6H, m), 4.76-4.66 (2H, m), 4.61 (1H, d, J = 11.3 Hz), 4.53 (1H, d, J = 12.1 Hz), 4.46 (1H, d, J = 12.1 Hz), 4.41-4.35 (1H, m), 4.29 (1H, dd, J = 7.0, 2.0 Hz), 4.25-4.12 (2H, m), 3.99-3.93 (1H, m), 3.87-3.74 (4H, m), 3.68-3.57 (2H, m), 3.41-3.34 (1H, m), 3.29 (1H, dt, J = 13.8, 5.3 Hz), 2.68 (1H, dd, J = 14.9, 5.9 Hz), 2.56-2.46 (3H, m), 2.42-2.19 (9H, m), 1.84 (1H, dd, J = 14.9, 3.9 Hz), 1.70-1.41 (12H, m), 1.36-1.15 (90H, m), 1.32 (3H, s), 1.28 (3H. s), 0.92-0.83 (18H, m). |
| 4(4c) |
| $^1$H-NMR ($CDCl_3$) δ : 7.35-7.20 (30H, m), 7.11 (1H, d, J = 8.2 Hz), 6.35 (1H, d, J = 7.4 Hz), 5.26 (1H, dd, J = 10.4, 8.8 Hz), 5.22-5.13 (3H, m), 5.11 (2H, s), 5.08 (1H, d, J = 12.1 Hz), 5.01 (1H, d, J = 12.1 Hz), 4.97-4.88 (3H, m), 4.86 (2H, d, J = 8.2 Hz), 4.72-4.65 (3H, m), 4.49 (2H, dd, J = 14.1, 12.1 Hz), 4.18 (1H, dd, J = 11.3, 3.5 Hz), 4.10 (1H, q, J = 9.0 Hz), 4.05-3.87 (5H, m), 3.83-3.73 (2H, m), 3.68-3.50 (3H, m), 3.34-3.25 (1H, m), 2.89 (1H, d, J = 2.7 Hz), 2.70-2.63 (1H, m), 2.55-2.41 (3H, m), 2.38-2.17 (9H, m), 2.11 (1H, dd, J = 12.5, 5.1 Hz), 1.95 (1H, t, J = 11.9 Hz), 1.72-1.36 (12H, m), 1.35-1.17 (90H, m), 0.91-0.83 (18H, m). |
| 4(4d) |
| $^1$H-NMR ($CD_3OD$-$CDCl_3$-$D_2O$) δ : 5.27-5.21 (1H, m), 5.20-5.13 (2H, m), 5.10 (1H, t, J= 9.8 Hz), 4.48 (1H, d, J = 8.2 Hz), 4.14 (1H, brs), 4.0-3.92 (3H, m), 3.91-3.60 (9H, m), 3.52 (1H, brs), 2.70 (1H, dd, J = 16.4, 6.8 Hz), 2.63-2.42 (4H, m), 2.38 (1H, dd, J = 14.9, 5.2 Hz), 2.34-2.24 (6H, m), 1.98 (1H, brs), 1.75 (1H, brs), 1.67-1.49 (12H, m), 1.38-1.18 (90H, m), 0.94-0.82 (18H, m). HRMS (ESI Neg) : $Ca_{89}H_{163}N_2O_{26}P$ ($[M-3H]^{3-}$) Found 568.0344 ; Calcd for $C_{89}H_{160}N_2O_{26}P$ 568.0338. |
| 5(5a) |
| $^1$H-NMR ($CDCl_3$) δ : 7.32-7.20 (30H, m), 7.14 (1H, d, J = 8.2 Hz), 6.38 (1H, d, J = 7.0 Hz), 5.26 (1H, dd, J = 10.4, 8.8 Hz), 5.23-5.13 (3YH, m), 5.12-4.97 (5H, m), 4.97-4.85 (2H, m), 4.83 (2H, d, J = 7.4 Hz), 4.75-4.64 (3H, m), 4.50 (2H, q, J = 12.1 Hz), 4.30 (1H, dd, J = 11.2, 2.9 Hz), 4.19-4.12 (1H, m), 4.06-3.87 (5H, m), 3.80 (1H, dd, J = 10.4, 2.2 Hz), 3.74-3.53 (4H, m), 3.22-3.14 (1H, m), 2.69 (1H, dd, J = 14.9, 6.3 Hz), 2.55-2.43 (3H, m), 2.38-2.17 (9H, m), 2.06-1.94 (2H, m), 1.71-1.37 (12H, m), 1.36-1.18 (90H, m), 0.96-0.83 (27H, m), 0.62-0.53 (6H, m). |
| 5(5b) |
| $^1$H-NMR ($CDCl_3$) δ : 7.36-7.08 (46H, m), 6.29 (1H, d, J = 7.0 Hz), 5.29-4.99 (8H, m), 4.99-4.74 (8H, m), 4.73-4.36 (12H, m), 4.05 (1H, d, J = 9.0 Hz), 3.99-3.56 (13H, m), 3.16-3.07 (1H, m), 2.90 (1H, dd, J = 15.5, 3.7 Hz), 2.72 (1H, d, J = 1.6 Hz), 2.62 (1H, dd, J = 15.1, 6.1 Hz), 2.54-2.41 (2H, m), 2.40-2.17 (9H, m), 2.17-2.07 (2H, m), 1.87-1.80 (1H, m), 1.67-1.41 (12H, m), 1.35-1.16 (96H, m), 0.92-0.83 (18H, m). |
| 5(5c) |
| $^1$H-NMR ($CDCl_3$) δ : 7.38-7.11 (46H, m), 6.30 (1H, d, J = 7.4 Hz), 5.26-4.76 (16H, m), 4.70-4.56 (5H, m), 4.52-4.42 (3H, m), 4.36-4.25 (2H, m), 4.08-3.82 (9H, m), 3.80-3.72 (3H, m), 3.69-3.58 (3H, m), 3.45 (1H, t, J = 8.6 Hz), 3.34 (1H, brs), 3.15 (1H, dt, J = 13.7, 5.2 Hz), 2.76 (1H, d, J = 2.0 Hz), 2.65 (1H, dd, J = 15.3, 5.9 Hz), 2.52-2.43 (3H, m), 2.38-2.08 (12H, m), 1.90 (1H, t, J = 12.3 Hz), 1.66-1.42 (12H, m), 1.35-1.16 (90H, m), 0.92-0.82 (18H, m). |

[Table 2-4]

| |
|---|
| 5(5d) |
| $^1$H-NMR ($CD_3OD$-$CDCl_3$-$D_2O$) δ : 5.26 (1H, brs), 5.17 (2H, brs), 5.11 (1H, brs), 4.53 (1H, brs), 4.20 (1H, brs), 4.14-3.55 (18H, m), 3.47 (1H, brs), 2.75-2.44 (5H, m), 2.43-2.35 (1H, m), 2.35-2.22 (6H, m), 2.08 (1H, brs), 1.97 (1H, brs), 1.88 (1H, brs), 1.74 (1H, brs), 1.67-1.50 (12H, m), 1.39-1.18 (90H, m), 0.94-0.83 (18H, m).<br>HRMS (ESI Neg) : $C_{97}H_{175}N_2O_{33}P$ ($[M-3H]^{3-}$) Found 641.7202 ; Calcd for $C_{97}H_{172}N_2O_{33}P$ 641.7210. |
| 6(6a-2) |
| $^1$H-NMR ($CDCl_3$) δ : 7.37-7.14 (33H, m), 7.13-7.04 (3H, m), 6.40 (1H, d, J = 7.4 Hz), 5.31 (1H, dd, J = 10.6, 8.6 Hz), 5.21-5.04 (6H, m), 5.02-4.93 (5H, m), 4.90 (2H, d, J = 7.4 Hz), 4.85 (1H, d, J = 11.0 Hz), 4.72-4.62 (5H, m), 4.44-4.35 (3H, m), 4.30 (1H, dd, J = 11.5, 2.9 Hz), 3.95 (1H, t, J = 9.2 Hz), 3.91-3.85 (1H, m), 3.82-3.67 (3H, m), 3.67-3.61 (1H, m), 3.54 (1H, dd, J = 9.4, 3.5 Hz), 3.34 (1H, dt, J = 14.0, 5.3 Hz), 2.68 (1H, dd, J = 14.9, 5.9 Hz), 2.55-2.46 (2H, m), 2.45-2.19 (9H, m), 1.73-1.40 (12H, m), 1.37-1.15 (90H, m), 0.92-0.83 (18H, m). |
| 6(6b) |
| $^1$H-NMR ($CD_3OD$-$CDCl_3$-$D_2O$) δ : 5.32-4.87 (5H, m), 4.62-3.28 (13H, m), 2.72 (1H, dd, J = 15.9, 6.2 Hz), 2.66-2.42 (4H, m), 2.38 (1H, dd, J = 15.0, 4.8 Hz), 2.35-2.23 (6H, m), 1.71-1.46 (12H, m), 1.46-1.07 (90H, m), 1.04-0.72 (18H, m).<br>HRMS (ESI Neg) : $C_{87}H_{159}N_2O_{25}P$ ($[M-2H]^{2-}$) Found 830.5417 ; Calcd for $C_{87}H_{157}N_2O_{25}P$ 830.5412. |
| 7(7a) |
| $^1$H-NMR ($CDCl_3$) δ : 7.38-7.13 (30H, m), 7.03 (1H, d, J = 7.8 Hz), 6.72 (1H, d, J = 9.0 Hz), 6.25 (III, d, J = 7.4 Hz), 5.29 (1H, dd, J = 10.6, 9.0 Hz), 5.23-5.06 (5H, m), 5.04-4.93 (2H, m), 4.$7-4.68 (9H, m), 4.59 (1H, d, J = 12.1 Hz), 4.54-4.48 (2H, m), 4.41-4.23 (3H, m), 4.13 (1H, d, J = 11.0 Hz), 3.84 (1H, dd, J = 10.8, 2.5 Hz), 3.79-3.57 (5H, m), 3.49-3.41 (2H, m), 3.39-3.31 (2H, m), 2.67 (1H, dd, J = 15.1, 6.5 Hz), 2.54 (1H, dd, J = 14.9, 5.9 Hz), 2.37-2.18 (10H, m), 1.70-1.38 (12H, m), 1.35-1.16 (90H, m), 0.92-0.83 (18H, m). |
| 7(7b) |
| $^1$H-NMR ($CDCl_3$) δ : 7.39-7.08 (31H, m), 6.36 (1H, d, J = 7.0 Hz), 5.47 (1H, dd, J = 10.4, 8.8 Hz), 5.21-5.02 (6H, m), 4.95-4.66 (8H, m), 4.59-4.46 (4H, m), 4.30 (1H, d, J = 8.6 Hz), 4.22 (1H, dd, J = 11.3, 3.5 Hz), 4.09 (1H, d, J = 10.2 Hz), 3.99 (1H, q, J = 9.0 Hz), 3.82 (1H, dd, J = 11.3, 2.7 Hz), 3.70-3.50 (4H, m), 3.49-3.43 (2H, m), 3.35-3.28 (1H, m), 3.28-3.18 (2H, m), 2.67 (1H, dd, J = 14.9, 6.3 Hz), 2.54 (1H, dd, J = 14.9, 6.3 Hz), 2.37-2.15 (10H, m), 1.97 (3H, s), 1.72-1.36 (12H, m), 1.36-1.17 (90H, m), 0.92-0.83 18H, m). |
| 7(7c) |
| $^1$H-NMR ($CD_3OD$-$CDCl_3$) δ : 5.31-5.05 (411, m), 4.66 (1H, d, J = 8.2 Hz), 4.50 (1H, d, J = 7.9 Hz), 4.37 (1H, brs), 4.23-4.11 (2H, m), 4.10-3.99 (1H, m), 3.99-3.88 (1H, m), 3.88-3.65 (5H, m), 3.59-3.42 (3H, m), 3.38-3.25 (1H, m), 2.71 (1H, dd, J = 16.0, 7.2 Hz), 2.66-2.42 (4H, m), 2.38 (1H, dd, J = 14.7, 5.4 Hz), 2.35-2.26 (6H, m), 2.13 (3H, s), 1.72-1.48 (12H, m), 1.40-1.19 (90H, m), 0.95-0.83 (18H, m).<br>HRMS (ESI Neg) : $C_{89}H_{164}N_3O_{24}P$($[M-2H]^{2-}$) Found 844.5663 ; Calcd for $C_{89}H_{162}N_3O_{24}P$ 844.5665. |
| 8(8a) |
| $^1$H-NMR ($CDCl_3$) δ : 7.45-7.21 (10H, m), 5.96-5.78 (1H, m), 5.31-5.19 (2H, m), 5.18 (2H, s), 5.14 (2H, d, J = 9.8 Hz), 4.89-4.77 (3H, m), 4.72 (1H, d, J = 11.7 Hz), 4.65 (1H, d, J = 12.1 Hz), 4.47 (1H, dd, J = 11.9, 4.5 Hz), 4.36 (1H, dd, J = 11.9, 2.2 Hz), 4.15 (1H, ddt, J = 12.8, 3.4, 1.8 Hz), 4.03-3.93 (2H, m). 3.83-3.77 (1H, m), 3.66-3.56 (2H, m). |
| 8(8b) |
| $^1$H-NMR ($CDCl_3$) δ : 7.42-7.19 (18H, m), 7.19-7.12 (2H, m), 5.91-5.78 (1H, m), 5.29-5.19 (2H, m), 5.16 (2H, s), 5.06-4.99 (1H, m), 4.99-4.79 (6H, m), 4.76-4.60 (3H, m), 4.54-4.42 (2H, m), 4.38 (1H, dd, J = 11.9, 5.3 Hz), 4.17-4.08 (1H, m), 4.07-3.90 (311, m), 3.78 (1H, dd, J = 10.2, 9.0 Hz). |
| 8(8c) |
| $^1$H-NMR ($CDCl_3$) δ : 7.40-7.19 (18H, m), 7.19-7.14 (2H, m), 5.23 (1H, t, J = 3.7 Hz), 5.14 (2H, s), 5.11 (lll, d, J = 9.4 Hz), 4.98-4.79 (5H, m), 4.73-4.58 (3H, m), 4.53 (1H, dd, J = 11.7, 2.0 Hz), 4.45 (1H, q, J = 9.4 Hz), 4.33 (1H, dd, J = 12.1, 5.5 Hz), 4.20-4.13 (1H, m), 3.97 (1H, td, J = 10.1, 2.5 Hz), 3.82 (1H, t, J = 9.8 Hz), 3.31-3.27 (1H,m). |

[Table 2-5]

| |
|---|
| 8(8d) |
| $^1$H-NMR ($CDCl_3$) δ : 7.39-7.07 (35H, m), 7.03 (1H, d, J = 7.8 Hz), 6.73 (1H, d, J = 9.0 Hz), 6.30 (1H, d, J = 7.4 Hz), 5.32-5.25 (1H, m), 5.24-4.24 (28H, m), 4.06 (1H, d, J = 12.1 Hz), 3.86-3.72 (2H, m), 3.63 (1H, t, J = 9.2 Hz), 3.48-3.27 (3H, m), 2.66 (1H, dd, J = 14.7, 6.5 Hz), 2.54 (1H, dd, J = 14.7, 6.1 Hz), 2.39-2.17 (10H, m), 1.68-1.40 (12H, m), 1.35-1.16 (90H, m), 0.91-0.84 (18H, m). |
| 8(8e) |
| $^1$H-NMR ($CDCl_3$) δ : 7.38-7.14 (36H, m), 6.39 (1H, d, J = 7.4 Hz), 5.45 (1H, dd, J = 10.4, 8.8 Hz), 5.23-5.03 (8H, m), 4.96-4.76 (9H, m), 4.72-4.62 (2H, m), 4.50-4.18 (6H, m), 4.05-3.99 (1H, m), 3.94 (1H, dd, J = 18.6, 8.4 Hz), 3.81 (1H, dd, J = 11.2, 2.9 Hz), 3.65 (1H, t, J = 9.4 Hz), 3.50-3.40 (1H, m), 3.36-3.30 (1H, m), 3.28-3.19 (2H, m), 2.66 (1H, dd, J = 14.9, 6.3 Hz), 2.53 (1H, dd, J = 14.9, 6.3 Hz). 2.38-2.16 (10H, m), 1.93 (3H, s), 1.77-1.45 (12H. m). 1.35-1.16 (90H, m), 0.91-0.83 (18H, m). |
| 8(8f) |
| $^1$H-NMR ($CD_3OD$-$CDCl_3$-$D_2O$) δ : 5.28-5.09 (4H, m), 4.65 (1H, brs), 4.56-4.46 (2H, m), 4.24-4.09 (4H, m), 3.98 (1H, brs), 3.89-3.64 (6H, m), 3.62-3.55 (1H, m), 3.46 (1H, brs), 2.70 (1H, dd, J = 16.4, 6.8 Hz), 2.64-2.42 (4H, m), 2.37 (1H, dd, J = 14.7, 5.4 Hz), 2.34-2.24 (6H, m), 2.08 (3H, s), 1.69-1.50 (12H, m), 1.39-1.20 (90H, m), 0.93-0.84 (18H, m),<br>HRMS (ESI Neg) : $C_{89}H_{165}N_3O_{27}P_2$ ([M-2H]$^{2-}$) Found 884.5503 ; Calcd for $C_{89}H_{163}N_3O_{27}P_2$ 884.5497. |
| 9(9a) |
| $^1$H-NMR ($CDCl_3$) δ : 7.37-7.20 (20H, m), 5.88-5.76 (1H, m), 5.25-5.15 (2H, m), 5.08-4.99 (5H, m), 4.86-4.69 (5H, m), 4.66 (1H, d, J = 11.7 Hz), 4.56 (1H, d, J = 10.6 Hz), 4.24 (2H, dd, J = 5.7, 2.9 Hz), 4.12-4.06 (1H, m), 3.99-3.88 (2H, m), 3.80-3.69 (2H, m), 3.56 (1H, t, J = 9.4 Hz). |
| 9(9b) |
| $^1$H-NMR ($CDCl_3$) δ : 7.38-7.19 (20H, m), 5.18-5.12 (2H, m), 5.07-4.96 (4H, m), 4.84-4.68 (4H, m), 4.64 (1H, d, J = 12.1 Hz), 4.54 (1H, d, J = 11.0 Hz), 4.36 (lH, d, J = 2.7 Hz), 4.29-4.13 (2H, m), 4.06-3.97 (1H, m), 3.89 (1H, td, J = 10.0, 3.1 Hz), 3.77 (1H, t, J = 9.6 Hz), 3.53 (1H, t, J = 9.4 Hz). |
| 9(9c) |
| $^1$H-NMR ($CDCl_3$) δ : 7.38-7.15 (35H, m), 7.03 (1H, d, J = 7.8 Hz), 6.73 (1H, d, J = 9.0 Hz), 6.36 (1H, d, J = 7.4 Hz), 5.33-5.27 (1H, m), 5.21-5.08 (5H, m), 5.05-4.70 (14H, m), 4.67 (1H, d, J = 7.4 Hz), 4.51 (1H, d, J = 11.0 Hz), 4.39-4.31 (2H, m), 4.26-4.01 (4H, m), 3.80-3.68 (2H, m), 3.57 (1H, t, J = 9.4 Hz), 3.50-3.31 (4H, m), 3.27-3.21 (1H, m), 2.67 (1H, dd, J = 14.9, 6.3 Hz), 2.53 (1H, dd, J = 14.9, 6.3 Hz), 2.37-2.17 (10H, m), 1.69-1.38 (12H, m), 1.36-1.16 (90H, m), 0.91-0.84 (18H, m). |
| 9(9d) |
| $^1$H-NMR ($CDCl_3$) δ: 7.38-7.16 (35H, m), 7.11 (1H, d, J = 8.2 Hz), 6.46 (1H, d, J = 7.0 Hz), 5.47 (1H, dd, J = 10.4, 8.8 Hz), 5.23-4.97 (11H, m), 4.92-4.71 (8H, m), 4.68-4.62 (1H, m), 4.53-4.46 (2H, m), 4.27 (1H, d, J = 8.6 Hz), 4.24-4.17 (1H, m), 4.14-3.92 (4H, m), 3.73 (1H, dd, J = 11.3, 2.7 Hz), 3.55-3.42 (3H, m), 3.28-3.16 (2H, m), 2.67 (1H, dd, J = 14.9, 6.3 Hz), 2.53 (1H, dd, J = 14.9, 6.3 Hz), 2.38-2.16 (10H, m), 1.98 (3H, s), 1.72-1.39 (12H, m), 1.36-1.16 (90H, m), 0.92-0.83 (18H, m). |
| 9(9e) |
| $^1$H-NMR ($CD_3OD$-$CDCl_3$-$D_2O$) δ : 5.29-5.08 (4H, m), 4.71-4.64 (1H, m), 4.56-4.50 (2H, m), 4.24-4.04 (5H, m), 3.86-3.64 (4H, m), 3.64-3.46 (4H, m), 2.71 (1H, dd, J = 16.3, 6.9 Hz), 2.64-2.42 (4H, m), 2.37 (1H, dd, J = 14.9, 5.5 Hz), 2.34-2.25 (6H, m), 2.09 (3H, s), 1.69-1.49 (12H, m), 1.39-1.18 (90H, m), 0.95-0.82 (18H, m).<br>HRMS (ESI Neg) : $C_{89}H_{165}N_3O_{27}P_2$([M-2H]$^{2-}$) Found 884.5507 ; Calcd for $C_{89}H_{163}N_3O_{27}P_2$ 884.5497. |
| 10(10a) |
| $^1$H-NMR ($CDCl_3$) δ : 7.74-7.65 (4H, m), 7.48-7.36 (6H, m), 5.76 (1H, d, J = 6.7 Hz), 4.67 (1H, brs), 4.11-3.97 (1H, m), 3.77 (1H, dt, J = 10.7, 4.9 Hz), 2.27 (1H, d, J = 2.3 Hz), 2.03-1.92 (1H, m), 1.88-1.76 (1H, m), 1.45 (9H, s), 1.06 (9H, s). |
| 10(10b) |

(continued)

| |
|---|
| $^1$H-NMR ($CDCl_3$) δ : 7.72-7.64 (4H, m), 7.45-7.25 (17H, m), 7.24-7.19 (2H, m), 7.18-7.12 (2H, m), 5.59 (1H, d, J = 8.6 Hz), 4.80-4.71 (3H, m), 4.64-4.47 (6H, m), 4.23 (1H, t, J = 9.2 Hz), 3.93 (1H, brs), 3.77-3.68 (4H, m), 3.57-3.49 (1H, m), 1.99-1.86 (1H, m), 1.83-1.71 (1H, m), 1.44 (9H, s), 1.06 (9H, s). |

[Table 2-6]

| 10(10c) |
|---|
| $^1$H-NMR ($CDCl_3$) δ : 7.71-7.63 (4H, m), 7.43-7.23 (19H, m), 7.19-7.13 (2H, m), 5.58 (1H, d, J = 7.8 Hz), 4.95 (1H, d, J = 11.3 Hz), 4.82-4.65 (3H, m), 4.65-4.49 (3H, m), 3.96-3.88 (2H, m), 3.76-3.64 (4H, m), 3.49-3.37 (2H, m), 2.90 (1H, t, J = 9.8 Hz), 2.30 (2H, brs), 2.01-1.90 (1H, m), 1.89-1.76 (1H, m), 1.44 (9H, s), 1.05 (9H, s). |
| 10(10d) |
| $^1$H-NMR ($CDCl_3$) δ : 7.73-7.62 (4H, m), 7.43-7.23 (19H, m), 7.19-7.12 (2H, m), 5.48 (1H, d, J = 8.6 Hz), 4.90 (1H, brs), 4.81 (2H, dd, J = 11.0, 9.4 Hz), 4.75-4.45 (7H, m), 4.23 (1H, d, J = 9.8 Hz), 3.90 (1H, brs), 3.85-3.60 (5H, m), 3.50-3.40 (2H, m), 1.99-1.86 (1H, m), 1.86-1.75 (1H, m), 1.44 (9H, s), 1.05 (9H, s). |
| 10(10e) |
| $^1$H-NMR ($CDCl_3$) δ : 7.69-7.62 (4H, m), 7.45-7.22 (19H, m), 7.21-7.15 (2H, m), 5.04 (1H, d, J = 8.6 Hz), 4.87-4.67 (5H, m), 4.63-4.48 (4H, m), 3.82-3.47 (8H, m), 3.46-3.34 (2H, m), 1.87-1.48 (6H, m), 1.41 (9H, s), 1.05 (9H, s). |
| 10(10f) |
| $^1$H-NMR ($CDCl_3$) δ : 7.37-7.25 (13H, m), 7.23-7.18 (2H, m), 4.89-4.48 (10H, m), 3.76-3.36 (9H, m), 3.32-3.24 (1H, m), 1.85-1.52 (6H, m), 1.44 (9H, s). |
| 10(10g) |
| $^1$H-NMR ($CDCl_3$) δ : 9.74-9.69 (1H, m), 7.37-7.25 (13H, m), 7.22-7.17 (2H, m), 4.93-4.51 (10H, m), 3.97 (1H, d, J = 6.3 Hz), 3.74-3.44 (5H, m), 3.44-3.37 (1H, m), 3.35-3.27 (1H, m), 2.56 (2H, d, J = 4.7 Hz), 1.84-1.51 (4H, m), 1.42 (9H, s). |
| 10(10h) |
| $^1$H-NMR ($CDCl_3$) δ : 7.36-7.24 (13H, m), 7.21-7.15 (2H, m), 5.04 (1H, d, J = 8.2 Hz), 4.88-4.49 (9H, m), 3.87 (1H, brs), 3.74-3.45 (5H, m), 3.43-3.37 (1H, m), 3.36-3.29 (1H, m), 2.63-2.43 (2H, m), 1.81-1.49 (4H, m), 1.43 (9H, s). |
| 10(10i) |
| $^1$H-NMR ($CDCl_3$) δ : 7.41-7.30 (5H, m), 5.19-5.06 (3H, m), 5.03 (1H, d, J = 9.0 Hz), 4.83-4.64 (2H, m), 3.94-3.84 (2H, m), 3.68 (1H, t, J = 10.6 Hz), 3.64-3.40 (4H, m), 3.26-3.17 (1H, m), 2.71 (1H, brs), 2.56 (2H, d, J = 5.5 Hz), 1.79-1.68 (1H, m), 1.66-1.46 (3H, m), 1.51 (6H, s), 1.43 (9H, s). |
| 10(10j) |
| $^1$H-NMR ($CDCl_3$) δ : 7.42-7.32 (5H, m), 5.22-5.06 (4H, m), 5.04-4.91 (2H, m), 4.79-4.63 (2H, m), 3.94-3.81 (2H, m), 3.73-3.56 (3H, m), 3.41 (1H, brs), 3.32-3.22 (1H, m), 2.65 (1H, dd, J = 15.3, 6.7 Hz), 2.60-2.47 (3H, m), 2.28 (2H, t, J = 7.4 Hz), 1.76-1.52 (8H, m), 1.47 (3H, s), 1.43 (9H, s), 1.37 (3H, s), 1.35-1.20 (30H, m), 0.93-0.85 (6H, m). |
| 10(10k) |
| $^1$H-NMR ($CDCl_3$) δ : 7.41-7.30 (5H, m), 5.57 (1H, d, J = 9.0 Hz), 5.16-5.06 (3H, m), 4.88 (1H, dd, J = 10.4, 9.2 Hz), 4.71 (2H, dd, J = 14.9, 12.1 Hz), 3.92 (1H, dd, J = 11.7, 3.1 Hz), 3.76-3.68 (1H, m), 3.61-3.51 (2H, m), 3.42-3.30 (2H, m), 3.28-3.20 (1H, m), 2.60-2.47 (3H, m), 2.41-2.25 (3H, m), 1.82-1.71 (1H, m), 1.68-1.50 (7H, m), 1.36-1.19 (30H, m), 0.92-0.83 (6H, m). |
| 10(101) |
| $^1$H-NMR ($CDCl_3$) δ : 7.41-7.32 (5H, m), 6.36 (1H, d, J = 9.0 Hz), 5.45 (1H, d, J = 9.4 Hz), 5.18-5.04 (4H, m), 4.84 (1H, t, J = 9.6 Hz), 4.75 (1H, d, J = 12.1 Hz), 4.67 (1H, d, J = 12.1 Hz), 4.29 (1H, brs), 3.93-3.85 (1H, m), 3.78-3.70 (1H, m), 3.62-3.48 (2H, m), 3.44-3.31 (2H, m), 2.69 (1H, t, J = 6.7 Hz), 2.59-2.34 (6H, m), 2.33-2.24 (4H, m), 1.75-1.46 (12H, m), 1.37-1.18 (60H, m), 0.93-0.83 (12H, m). |
| 10(10m) |

(continued)

| |
|---|
| $^1$H-NMR ($CDCl_3$) δ : 7.41-7.30 (5H, m), 6.32 (1H, d, J = 9.4 Hz), 5.24-5.05 (5H, m), 4.68 (1H, t, J = 9.4 Hz), 4.34 (1H, td, J = 9.1, 5.2 Hz), 3.89-3.82 (1H, m), 3.76-3.68 (1H, m), 3.50 (1H, t, J = 9.2 Hz), 3.34-3.28 (1H, m), 3.19-3.12 (1H, m), 2.97 (1H, brs), 2.78 (1H, brs), 2.66-2.50 (6H, m), 2.47-2.33 (2H, m), 2.32-2.24 (4H, m), 1.93-1.82 (1H, m), 1.74-1.39 (11H, m), 1.36-1.18 (60H, m), 0.92-0.83 (12H, m). |

[Table 2-7]

| |
|---|
| 10(10n) |
| $^1$H-NMR ($CDCl_3$) δ : 7.40-7.30 (5H, m), 6.49 (1H, d, J = 8.6 Hz), 5.96 (1H, d, J = 9.0 Hz), 5.19-5.05 (5H, m), 4.80 (1H, dd, J = 10.2, 9.4 Hz), 4.31-4.22 (1H, m), 3.94-3.83 (2H, m), 3.77-3.68 (1H, m), 3.59-3.51 (1H, m), 3.39-3.30 (3H, m), 2.73-2.66 (1H, m), 2.60-2.51 (4H, m), 2.51-2.35 (3H, m), 2.35-2.21 (7H, m), 1.76-1.37 (16H, m), 1.36-1.17 (90H, m), 0.92-0.83 (18H, m). |
| 10($10_o$) |
| $^1$H-NMR ($CDCl_3$) δ : 7.71-7.65 (4H, m), 7.44-7.27 (11H, m), 6.57 (1H, d, J = 8.2 Hz), 5.98 (1H, d, J = 8.6 Hz), 5.20-5.00 (5H, m), 4.83 (1H, t, J = 9.6 Hz), 4.19-4.08 (1H, m), 3.94-3.83 (3H, m), 3.72 (1H, td, J= 9.2, 3.1 Hz), 3.38-3.30 (3H, m), 2.66-2.16 (14H, m), 1.74-1.35 (16H, m), 1.35-1.17 (90H, m), 1.02 (9H, s), 0.93-0.82 (18H, m). |
| 10(10p) |
| $^1$H-NMR ($CDCl_3$) δ : 7.68-7.61 (4H, m), 7.38-7.17 (19H, m), 7.16-7.09 (2H, m), 6.58 (1H, d, J = 8.2 Hz), 6.01 (1H, d, J = 9.0 Hz), 5.21-4.98 (6H, m), 4.89-4.81 (3H, m), 4.74 (1H, dd, J = 11.7, 9.4 Hz), 4.45 (1H, q, J = 9.3 Hz), 4.23-4.13 (1H, m), 3.99-3.78 (3H, m), 3.48-3.39 (2H, m), 2.53-2.12 (14H, m), 1.74-1.36 (16H, m), 1.35-1.18 (90H, m), 1.02 (9H, s), 0.91-0.83 (18H, m). |
| 10(10q) |
| $^1$H-NMR ($CDCl_3$) δ : 7.40-7.27 (15H, m), 6.49 (1H, d, J = 8.6 Hz), 6.00 (1H, d, J = 8.6 Hz), 5.19-4.91 (10H, m), 4.39 (1H, q, J = 9.4 Hz), 4.27-4.18 (1H, m), 3.92-3.73 (3H, m), 3.41 (1H, t, J = 8.4 Hz), 3.30 (1H, d, J = 9.8 Hz), 2.57 (2H, d, J = 5.5 Hz), 2.49-2.14 (12H, m), 1.76-1.34 (16H, m), 1.34-1.15 (90H, m), 0.92-0.83 (18H, m). |
| 10(10r) |
| $^1$H-NMR ($CD_3OD$-$CDCl_3$) δ : 7.43 (1H, d, J = 9.4 Hz), 5.24-5.13 (3H, m), 5.00 (1H, t, J = 9.8 Hz), 4.24-4.12 (2H, m), 3.94 (1H, d, J = 12.9 Hz), 3.81 (1H, t, J = 9.2 Hz), 3.71 (1H, d, J = 12.5 Hz), 3.40 (1H, d, J = 9.0 Hz), 3.26 (1H, d, J = 9.4 Hz), 2.71 (1H, dd, J = 16.4, 7.0 Hz), 2.58 (1H, dd, J = 16.2, 5.7 Hz), 2.52-2.24 (12H, m), 1.73 (1H, brs), 1.67-1.50 (1311, m), 1.36-1.21 (2H, m), 1.37-1.19 (90H, m), 0.93-0.84 (18H, m).<br>HRMS (ESI Neg) : $C_{83}H_{155}N_2O_{18}P$ ($[M-2H]^{2-}$) Found 748.5433 ; Calcd for $C_{83}H_{153}N_2O_{18}P$ 748.5433. |
| 11(11a) |
| $^1$H-NMR ($CDCl_3$) δ : 7.40-7.24 (15H, m), 6.57 (1H, d, J = 8.2 Hz), 6.00 (1H, d, J = 8.6 Hz), 5.21-5.00 (6H, m), 4.97 (2H, d, J = 7.8 Hz), 4.94 (2H, d, J = 7.8 Hz), 4.30 (1H, q, J = 9.3 Hz), 4.19-4.10 (1H, m), 3.95-3.89 (1H, m), 3.83 (1H, q, J = 9.7 Hz), 3.67 (1H, dd, J = 11.7, 5.9 Hz), 3.42-3.34 (2H, m), 2.63-2.13 (14H, m), 1.72-1.35 (16H, m), 1.35-1.15 (90H, m), 0.94-0.83 (27H, m), 0.57-0.47 (6H, m). |
| 11(11b) |
| $^1$H-NMR ($CDCl_3$) δ : 7.35-7.21 (30H, m), 6.47 (1H, d, J = 8.6 Hz), 5.96 (1H, d, J = 9.0 Hz), 5.19-4.88 (12H, m), 4.74 (1H, d, J = 11.3 Hz), 4.63 (1H, d, J = 11.3 Hz), 4.52 (1H, d, J = 12.1 Hz), 4.46 (1H, d, J = 12.1 Hz), 4.42-4.36 (1H, m), 4.32 (1H, dd, J = 6.8, 1.8 Hz), 4.17-4.08 (1H, m), 4.04-3.94 (2H, m), 3.91-3.81 (2H, m), 3.81-3.75 (1H, m), 3.70-3.50 (3H, m), 3.35-3.22 (2H, m), 2.51-2.14 (15H, m), 1.92 (1H, dd, J = 14.7, 4.1 Hz), 1.66-1.39 (16H, m), 1.35-1.15 (90H, m), 1.34 (3H, s), 1.29 (3H, s), 0.92-0.83 (18H, m). |
| 11(11c) |
| $^1$H-NMR ($CDCl_3$) δ : 7.38-7.19 (30H, m), 6.43 (1H, d, J = 8.6 Hz), 5.92 (1H, d, J = 8.6 Hz), 5.17-4.87 (12H, m), 4.70 (2H, s), 4.49 (2H, dd, J = 19.0, 11.9 Hz), 4.12 (1H, brs), 4.04-3.88 (6H, m), 3.73 (1H, dd, J = 10.6, 2.7 Hz), 3.65-3.46 (4H, m), 3.29 (1H, t, J = 9.2 Hz), 2.77-2.70 (2H, m), 2.51 (2H, d, J = 5.9 Hz), 2.45-2.22 (10H, m), 2.18 (2H, t, J = 7.6 Hz), 2.06 (1H, dd, J = 12.5, 5.1 Hz), 1.97 (1H, t, J = 12.1 Hz), 1.66-1.35 (16H, m), 1.34-1.17 (90H, m), 0.92-0.83(1811, m). |

(continued)

| 11(11d) |
|---|
| $^1$H-NMR ($CD_3OD$-$CDCl_3$-$D_2O$) δ : 5.23-5.10 (3H, m), 5.01 (1H, t, J = 9.5 Hz), 4.07 (1H, brs), 4.02-3.64 (9H, m), 3.63-3.54 (1H, m), 3.46-3.36 (2H, m), 2.70 (1H, dd, J = 16.3, 6.7 Hz), 2.61-2.23 (13H, m), 2.03 (1H, brs), 1.75 (1H, brs), 1.69-1.46 (14H, m), 1.43-1.19 (92H, m), 0.93-0.85 (18H, m). HRMS (ESI Neg) : $C_{91}H_{167}N_2O_{25}P$ ([M-2H]$^{2-}$) Found 858.5728 ; Calcd for $C_9H_{165}N_2O_{25}P$ 858.5725. |

[Table 2-8]

| 12(12a) |
|---|
| $^1$H-NMR ($CDCl_3$) δ : 7.37-7.20 (30H, m), 6.51 (1H, d, J = 8.6 Hz), 6.04 (1H, d, J = 7.8 Hz), 5.21-5.10 (3H, m), 5_09-4.85 (9H, m), 4.76 (1H, d, J = 11.3 Hz), 4.70 (1H, d, J = 11.3 Hz), 4.50 (1H, d, J = 11.7 Hz), 4.45 (1H, d, J = 12.1 Hz), 4.21-4.09 (2H, m), 4.09-4.02 (2H, m), 4.01-3.87 (4H, m), 3.80-3.74 (1H, m), 3.63 (1H, dd, J = 10.6, 5.5 Hz), 3.58-3.41 (4H, m), 2.47-2.14 (14H, m), 2.02-1.92 (2H, m), 1.66-1.38 (16H, m), 1.35-1.16 (90H, m), 0.97-0.83 (27H, m), 0.58 (6H, q, J = 8.0 Hz). |
| 12(12b) |
| $^1$H-NMR ($CDCl_3$) δ : 7.38-7.12 (45H, m), 6.44 (1H, d, J = 8.6 Hz), 5.88 (1H, d, J = 8.6 Hz), 5.22-5.11 (4H, m), 5.07-4.82 (10H, m), 4.71 (1H, d, J = 11.7 Hz), 4.66 (1H, d, J = 11.7 Hz), 4.62-4.34 (9H, m), 4.19 (1H, brs), 4.04-3.76 (8H, m), 3.74-3.47 (6H, m), 3.28-3.21 (1H, m), 2.90 (1H, dd, J = 15.3, 3.5 Hz), 2.66 (1H, dd, J = 15.1, 4.9 Hz), 2.51-2.04 (16H, m), 1.87-1.79 (1H, m), 1.67-1.39 (16H, m), 1.36-1.17 (96H, m), 0.92-0.83 (18H, m). |
| 12(12c) |
| $^1$H-NMR ($CDCl_3$) δ : 7.38-7.12 (45H, m), 6.53 (1H, d, J = 8.6 Hz), 5.89 (1H, d, J = 8.2 Hz), 5.20-5_10 (3H, m), 5.09-4.82 (11H, m), 4.75-4.58 (4H, m), 4.56-4.37 (4H, m), 4.28-4.10 (2H, m), 4.05-3.79 (9H, m), 3.76-3.69 (2H, m), 3.68-3.48 (4H, m), 3.40 (1H, t, J = 9.2 Hz), 3.26 (2H, d, J = 2.3 Hz), 2.65-2.34 (8H, m), 2.33-2.08 (11H, m), 1.91 (1H, t, J = 12.1 Hz), 1.64-1.39 (16H, m), 1.34-1.15 (90H, m), 0.92-0.83 (18H, m). |
| 12(12d) |
| $^1$H-NMR ($CD_3OD$-$CDCl_3$-$D_2O$) δ : 5.24-5.10 (3H, m), 5.07-4.98 (1H, m), 4.12-3.53 (17H, m), 3.51-3.35 (2H, m), 2.70 (1H, dd, J = 16.4, 6.2 Hz), 2.63-2.23 (13H, m), 2.12-1.97 (2H, m), 1.87 (1H, brs), 1.80-1.48 (15H, m), 1.47-1.09 (92H, m), 0.93-0.83 (18H, m). HRMS (ESI Neg) : $C_{99}H_{179}N_2O_{32}P$ ([M-3H]$^{3-}$) Found 645.3993 ; Calcd for $C_{99}H_{176}N_2O_{32}P$ 645.3987. |
| 13(13a) |
| $^1$H-NMR ($CDCl_3$) δ : 7.45-7.05 (15H, m), 4.86-4.68 (2H, m), 4.68-4.43 (7H, m), 4.23 (1H, t, J = 9.5 Hz), 4.06-3.95 (2H, m), 3.78-3.67 (3H, m), 3.58-3.48 (1H, m), 1.62 (3H, s), 1.54-1.42 (12H, m). |
| 13(13b) |
| $^1$H-NMR ($CDCl_3$) δ : 7.41-7.23 (13H, m), 7.16 (2H, brs), 4.96 (1H, d, J = 11.0 Hz), 4.84-4.70 (2H, m), 4.69-4.50 (4H, m), 4.07-3.96 (2H, m), 3.89 (1H, d, J = 8.5 Hz), 3.78-3.62 (3H, m), 3.45 (1H, brs), 3.39 (1H, t, J = 9.2 Hz), 2.95 (1H, t, J = 9.8 Hz), 1.68-1.40 (15H, m). |
| 13(13c) |
| $^1$H-NMR ($CDCl_3$) δ : 7.47-7.05 (15H, m), 5.39-4.45 (10H, m), 4.26 (1H, brs), 4.06-3.34 (8H, m), 1.73-1.35 (15H, m). |
| 13(13d) |
| $^1$H-NMR ($CDCl_3$) δ : 7.43-7.14 (15H, m), 4.98-4.46 (9H, m), 4.02-3.16 (10H, m), 2.04-1.87 (2H, m), 1.81-1.31 (17H, m). |
| 13(13e) |
| $^1$H-NMR ($CDCl_3$) δ : 7.38-7.23 (13H, m), 7.21-7.15 (2H, m), 5.36 (1H, d, J = 7.9 Hz), 4.92-4.46 (9H, m), 4.30-4.17 (1H, m), 3.74-3.39 (6H, m), 3.39-3.29 (1H, m), 2.08-1.97 (1H, m), 1.96-1.74 (2H, m), 1.66-1.51 (1H, m), 1.44 (9H, s). |
| 13(13f) |

(continued)

| |
|---|
| $^{1}$H-NMR ($CDCl_3$) δ : 7.41-7.30 (5H, m), 5.32-5.08 (4H, m), 4.79 (1H, d, J = 12.1 Hz), 4.70 (1H, d, J = 12.1 Hz), 4.30-4.20 (1H, m), 3.87 (1H, dd, J = 10.6, 5.5 Hz), 3.68 (1H, t, J = 10.6 Hz), 3.60 (1H, dd, J = 8.6, 2.3 Hz), 3.56-3.40 (3H, m), 3.26-3.16 (1H, m), 2.85 (1H, d, J = 2.3 Hz), 2.07-1.71 (4H, m), 1.51 (3H, s), 1.44 (9H, s), 1.42 (3H, s). |
| 13(13g) |
| $^{1}$H-NMR ($CDCl_3$) δ : 7.40-7.31 (5H, m), 5.26 (1H, d, J = 9.0 Hz), 5.23-5.09 (4H, m), 4.96 (1H, t, J = 9.8 Hz), 4.70 (2H, s), 4.28-4.19 (1H, m), 3.89 (1H, dd, J = 11.0, 5.5 Hz), 3.72-3.54 (3H, m), 3.47-3.39 (1H, m), 3.26 (1H, td, J = 9.8, 5.3 Hz), 2.63 (1H, dd, J = 15.3, 6.7 Hz), 2.51 (1H, dd, J = 15.5, 6.1 Hz), 2.27 (2H, t, J = 7.4 Hz), 1.94 (1H, brs), 1.86-1.70 (2H, m), 1.66-1.53 (5H, m), 1.47 (3H, s), 1.43 (9H, s), 1.36 (3H, s), 1.34-1.20 (30H, m), 0.91-0.85 (6H, m). |

[Table 2-9]

| 13(13h) |
|---|
| $^{1}$H-NMR ($CDCl_3$) δ : 7.42-7.30 (5H, m), 5.65 (1H, d, J = 9.4 Hz), 5.17-5.06 (3H, m), 4.93-4.85 (1H, m), 4.76-4.60 (2H, m), 3.88 (1H, dd, J = 11.7, 2.7 Hz), 3.71 (1H, dd, J = 11.9, 5.3 Hz), 3.60-3.46 (3H, m), 3.40-3.30 (2H, m), 2.73 (2H, brs), 2.61-2.46 (2H, m), 2.29 (2H, t, J = 7.6 Hz), 1.92-1.74 (2H, m), 1.67-1.50 (6H, m), 1.35-1.18 (30H, m), 0.93-0.84 (6H, m). |
| 13(13i) |
| $^{1}$H-NMR ($CDCl_3$) δ : 7.41-7.31 (5H, m), 6.43 (1H, d, J = 7.4 Hz), 5.44 (1H, d, J = 9.0 Hz), 5.22-5.04 (4H, m), 4.83 (1H, t, J = 9.6 Hz), 4.75 (1H, d, J = 12.1 Hz), 4.66 (1H, d, J = 12.1 Hz), 4.64-4.56 (1H, m), 3.93-3.85 (1H, m), 3.77-3.71 (1H, m), 3.61-3.51 (2H, m), 3.43-3.29 (3H, m), 2.57-2.42 (4H, m), 2.33-2.24 (4H, m), 2.08-1.98 (1H, m), 1.84-1.68 (2H, m), 1.67-1.49 (9H, m), 1.35-1.18 (60H, m), 0.92-0.84 (12H, m). |
| 13(13j) |
| $^{1}$H-NMR ($CDCl_3$) δ : 7.40-7.30 (5H, m), 6.67 (1H, brs), 5.28-5.05 (5H, m), 4.70-4.61 (1H, m), 3.97-3.83 (1H, m), 3.77-3.70 (1H, m), 3.61 (1H, brs), 3.56-3.39 (2H, m), 2.86 (1H, brs), 2.76-2.45 (4H, m), 2.34-2.22 (4H, m), 2.12-1.99 (1H, m), 1.96-1.76 (2H, m), 1.67-1.50 (9H, m), 1.35-1.21 (60H, m), 0.92-0.83 (12H, m). |
| 13(13k) |
| $^{1}$H-NMR ($CDCl_3$) δ : 7.40-7.30 (5H, m), 6.64 (1H, d, J = 7.4 Hz), 5.97 (1H, d, J = 9.0 Hz), 5.21-5.03 (5H, m), 4.78 (1H, dd, J = 10.2, 9.4 Hz), 4.56-4.49 (1H, m), 3.92-3.82 (2H, m), 3.76-3.68 (1H, m), 3.55 (1H, td, J = 9.2, 3.9 Hz), 3.38 (1H, d, J = 3.9 Hz), 3.36-3.29 (2H, m), 2.59-2.38 (6H, m), 2.34-2.22 (6H, m), 2.07-1.95 (1H, m), 1.83-1.47 (15H, m), 1.36-117 (90H, m), 0.92-0.84 (18H, m). |
| 13(131) |
| $^{1}$H-NMR ($CDCl_3$) δ : 7.71-7.64 (4H, m), 7.46-7.32 (6H, m), 7.26-7.23 (5H, m), 6.70 (1H, d, J = 7.0 Hz), 6.01 (1H, d, J = 8.6 Hz), 5.25-5.07 (4H, m), 5.02 (1H, d, J = 12.1 Hz), 4.82 (1H, dd, J = 10.2, 9.4 Hz), 4.44-4.36 (1H, m), 3.95-3.81 (3H, m), 3.74 (1H, td, J = 9.3, 3.0 Hz), 3.39-3.27 (3H, m), 2.66-2.39 (6H, m), 2.36-2.20 (6H, m), 2.06-1.95 (1H, m), 1.86-1.74 (1H, m), 1.73-1.48 (14H, m), 1.36-1.18 (90H, m), 1.02 (9H, s), 0.92-0.82 (18H, m). |
| 13(13m) |
| $^{1}$H-NMR ($CDCl_3$) δ : 7.68-7.61 (4H, m), 7.37-7.21 (17H, m), 7.21-7.16 (2H, m), 7.15-7.11 (2H, m), 6.71 (1H, d, J = 7.4 Hz), 6.06 (1H, d, J = 8.6 Hz), 5.26-4.97 (6H, m), 4.91-4.81 (3H, m), 4.75 (1H, dd, J = 11.7, 9.8 Hz), 4.52-4.42 (2H, m), 3.94 (1H, d, J = 10.2 Hz), 3.86-3.76 (2H, m), 3.48-3.38 (2H, m), 2.60-2.30 (6H, m), 2.30-2.23 (4H, m), 2.19 (2H, dd, J = 10.4, 4.9 Hz), 2.09-1.96 (1H, m), 1.93-1.80 (1H, m), 1.75-1.40 (14H, m), 1.36-1.16 (90H, m), 1.01 (9H, s), 0.92-0.83 (18H, m). |
| 13(13n) |
| $^{1}$H-NMR ($CDCl_3$) δ : 7.40-7.27 (15H, m), 6.60 (1H, d, J = 7.4 Hz), 6.01 (1H, d, J = 8.6 Hz), 5.21-4.91 (10H, m), 4.51 (1H, dt, J = 12.5, 4.5 Hz), 4.38 (1H, q, J = 9.5 Hz), 3.88-3.71 (4H, m), 3.44-3.36 (1H, m), 3.30-3.24 (1H, m), 2.60-2.45 (2H, m), 2.43-2.14 (10H, m), 2.09-1.97 (1H, m), 1.83-1.44 (15H, m), 1.35-1.12 (90H, m), 0.93-0.81 (18H, m). |
| 13(13o) |

(continued)

| |
|---|
| 1H-NMR ($CD_3OD$-$CDCl_3$) δ : 7.80 (1H, d, J = 7.4 Hz), 7.62 (1H, d, J = 7.4 Hz), 5.29-5.15 (3H, m), 5.05 (1H, t, J = 9.6 Hz), 4.30 (1H, dd, J = 9.6, 4.1 Hz), 4.19 (1H, q, J = 9.8 Hz), 3.98 (1H, d, J = 11.3 Hz), 3.85-3.66 (3H, m), 3.28 (1H, d, J = 9.4 Hz), 2.71 (1H, dd, J = 16.4, 7.4 Hz), 2.65-2.25 (11H, m), 1.98 (1H, brs), 1.89 (1H, brs), 1.70-1.53 (12H, m), 1.52-1.40 (2H, m), 1.39-1.19 (90H, m), 0.94-0.84 (18H, m).<br>HRMS (ESI Neg) : $C_{82}H_{153}N_2O_{18}P$ ($[M-2H]^{2-}$) Found 741.5343 ; Calcd for $C_{82}H_{151}N_2O_{18}P$ 741.5355. |
| 14(14a) |
| 1H-NMR ($CDCl_3$) δ : 7.37-7.25 (15H, m), 6.71 (1H, d, J = 7.0 Hz), 6.02 (1H, d, J = 8.6 Hz), 5.25-5.09 (5H, m), 5.05 (1H, dd, J = 10.2, 9.4 Hz), 4.98 (2H, d, J = 8.2 Hz), 4.93 (2H, d, J = 7.8 Hz), 4.42-4.37 (1H, m), 4.29 (1H, q, J = 9.3 Hz), 3.90 (1H, d, J = 10.6 Hz), 3.78 (1H, q, J = 9.5 Hz), 3.66 (1H, dd, J = 11.9, 5.7 Hz), 3.43-3.35 (2H, m), 2.60-2.29 (6H, m), 2.29-2.22 (4H, m), 2.19 (2H, t, J = 7.6 Hz), 2.06-1.94 (1H, m), 1.89-1.74 (1H, m), 1.69-1.39 (14H, m), 1.36-1.12 (90H, m), 0.93-0.82 (27H, m), 0.55-0.45 (6H, m). |

[Table 2-10]

| |
|---|
| 14(14b) |
| 1H-NMR ($CDCl_3$) δ : 7.39-7.21 (30H, m), 6.67 (1H, d, J = 7.8 Hz), 5.98 (1H, d, J = 8.6 Hz), 5.21-4.86 (12H, m), 4.76 (1H, d, J = 11.3 Hz), 4.67 (1H, d, J = 11.3 Hz), 4.54-4.39 (3H, m), 4.36-4.27 (2H, m), 4.04-3.94 (2H, m), 3.90-3.74 (3H, m), 3.65-3.50 (3H, m), 3.32 (2H, dd, J = 19.0, 9.6 Hz), 2.53-2.15 (13H, m), 1.97-1.82 (2H, m), 1.63-1.40 (15H, m), 1.37-1.17 (90H, m), 1.34 (3H, s), 1.31 (3H, s), 0.92-0.83 (18H, m). |
| 14(14c) |
| 1H-NMR ($CDCl_3$) δ : 7.37-7.21 (30H, m), 6.63 (1H, d, J = 7.4 Hz), 6.00 (1H, d, J = 8.2 Hz), 5.21 (1H, d, J = 12.1 Hz), 5.18-5.08 (4H, m), 5.08-4.99 (3H, m), 4.96-4.87 (4H, m), 4.71 (2H, s), 4.53-4.41 (3H, m), 4.06-3.88 (6H, m), 3.76-3.71 (1H, m), 3.65-3.47 (4H, m), 3.38 (1H, t, J = 9.2 Hz), 2.85 (1H, d, J = 3.1 Hz), 2.54-2.15 (13H, m), 2.10-2.03 (1H, m), 1.99-1.82 (2H, m), 1.73-1.34 (15H, m), 1.34-1.17 (90H, m), 0.93-0.83 (18H, m). |
| 14(14d) |
| 1H-NMR ($CD_3OD$-$CDCl_3$-$D_2O$) δ : 5.24-5.09 (3H, m), 5.02 (1H, t, J = 9.5 Hz), 4.20 (1H, brs), 4.05-3.56 (10H, m), 3.47-3.36 (2H, m), 2.70 (1H, dd, J = 16.0, 6.4 Hz), 2.61-2.52 (2H, m), 2.51-2.42 (2H, m), 2.36 (1H, dd, J = 15.0, 5.1 Hz), 2.33-2.25 (6H, m), 2.05-1.97 (1H, m), 1.87 (1H, brs), 1.75 (2H, brs), 1.67-1.48 (12H, m), 1.37-1.18 (92H, m), 0.93-0.84 (18H, m).<br>HRMS (ESI Neg) - $C_{90}H_{165}N_2O_{25}P$ ($[M-2H]^{2-}$) Found 852.0658 ; Calcd for $C_{90}H_{163}N_2O_{25}P$ 852.0664. |
| 15(15a) |
| 1H-NMR ($CDCl_3$) δ : 7.37-7.20 (30H, m), 6.84 (1H, d, J = 7.8 Hz), 6.03 (1H, d, J = 7.4 Hz), 5.21-5.01 (8H, m), 4.99-4.84 (5H, m), 4.74 (2H, brs), 4.51-4.43 (3H, m), 4.20-4.14 (1H, m), 4.07-4.02 (1H, m), 4.02-3.92 (3H, m), 3.89-3.85 (1H, m), 3.78 (1H, dd, J = 10.4, 2.2 Hz), 3.65 (1H, dd, J = 10.6, 5.1 Hz), 3.55 (1H, dd, J = 11.0, 7.8 Hz), 3.48-3.36 (3H, m), 2.50 (1H, dd, J = 14.9, 7.0 Hz), 2.45-2.16 (11H, m), 2.01-1.95 (2H, m), 1.87-1.73 (2H, m), 1.64-1.37 (14H, m), 1.34-1.17 (90H, m), 0.94 (9H, t, J = 8.0 Hz), 0.91-0.84 (18H, m), 0.60 (6H, q, J = 8.0 Hz). |
| 15(15b) |
| 1H-NMR ($CDCl_3$) δ : 7.39-7.14 (45H, m), 6.57 (1H, d, J = 7.4 Hz), 5.99 (1H, d, J = 8.6 Hz), 5.20-4.82 (13H, m), 4.69-4.25 (11H, m), 4.13 (1H, brs), 4.07-3.73 (10H, m), 3.68-3.51 (4H, m), 3.42 (1H, t, J = 8.8 Hz), 3.32 (1H, t, J = 8.2 Hz), 3.03 (1H, dd, J = 15.7, 3.1 Hz), 2.49 (1H, dd, J = 14.7, 6.5 Hz), 2.44-2.14 (12H, m), 2.12-2.01 (2H, m), 1.91 (1H, brs), 1.65-1.39 (16H, m), 1.35-1.09 (96H, m), 0.91-0.83 (18H, m). |
| 15(15c) |
| 1H-NMR ($CDCl_3$) δ : 7.35-7.17 (45H, m), 6.94 (1H, d, J = 7.4 Hz), 5.77 (1H, d, J = 8.6 Hz), 5.19-5.02 (7H, m), 5.01-4.88 (6H, m), 4.86 (1H, s), 4.84 (1H, s), 4.72-4.52 (5H, m), 4.50-4.35 (4H, m), 4.29-4.22 (1H, m), 4.05 (1H, brs), 4.02-3.88 (7H, m), 3.84 (1H, d, J = 5.5 Hz), 3.76-3.68 (2H, m), 3.67-3.52 (3H, m), 3.46-3.34 (2H, m), 3.26 (1H, d, J = 2.3 Hz), 3.13 (1H, brs), 2.75 (1H, d, J = 2.0 Hz), 2.48 (1H, dd, J = 15.1, 6.8 Hz), 2.42-2.12 (12H, m), 1.93 (1H, t, J = 12.1 Hz), 1.84-1.74 (2H, m), 1.64-1.37 (16H, m), 1.34-1.15 (90H, m), 0.92-0.83 (18H, m). |
| 15(15d) |

(continued)

| |
|---|
| $^1$H-NMR ($CD_3OD$-$CDCl_3$-$D_2O$) δ : 5.24-5.10 (3H, m), 5.07-4.99 (1H, m), 4.22-4.14 (1H, m), 4.12-3.53 (16H, m), 3.51-3.38 (2H, m), 2.70 (1H, dd, J = 16.3, 6.4 Hz), 2.63-2.54 (2H, m), 2.54-2.42 (2H, m), 2.41-2.24 (7H, m), 2.13-2.04 (1H, m), 2.03-1.84 (3H, m), 1.81-1.69 (2H, m), 1.68-1.49 (12H, m), 1.48-1.20 (92H, m), 0.93-0.84 (18H, m).<br>HRMS (ESI Neg) : $C_{98}H_{177}N_2O_{32}P$ ($[M-3H]^{3-}$) Found 641.0612 ; Calcd for $C_{98}H_{174}N_2O_{32}P$ 641.0613. |
| 16(16a) |
| $^1$H-NMR ($CDCl_3$) δ : 7.40-7.25 (15H, m), 6.63 (1H, d, J = 7.3 Hz), 6.07 (1H, d, J = 8.5 Hz), 5.24-5.04 (6H, m), 5.01-4.90 (4H, m), 4.64 (0.5H, d, J = 9.8 Hz), 4.57-4.38 (2.5H, m), 4.30 (1H, q, J = 9.4 Hz), 3.75 (1H, q, J = 9.4 Hz), 3.55-3.40 (2H, m), 2.59-2.16 (12H, m), 2.05-1.94 (1H, m), 1.85-1.77 (1H, m), 1.73-1.39 (14H, m), 1.35-1.18 (90H, m), 0.92-0.84 (18H, m). |

[Table 2-11]

| 16(16b) |
|---|
| $^1$H-NMR ($CD_3OD$-$CDCl_3$) δ : 5.28-5.15 (3H, m), 5.07 (1H, t, J = 9.8 Hz), 4.87 (0.5H, d, J = 9.0 Hz), 4.79-4.70 (0.5H, m), 4.54 (1H, brs), 4.26 (1H, dd, J = 9.6, 4.1 Hz), 4.08 (1H, q, J = 9.3 Hz), 3.79 (1H, t, J = 9.6 Hz), 3.69-3.66 (1H, m), 3.64-3.54 (1H, m), 3.45-3.38 (1H, m), 2.75 (1H, dd, J = 16.2, 7.2 Hz), 2.66-2.42 (4H, m), 2.41-2.26 (7H, m), 1.99 (1H, brs), 1.93-1.84 (1H, m), 1.73-1.52 (12H, m), 1.52-1.18 (92H, m), 0.93-0.85 (18H, m).<br>HRMS (ESI Neg) : $C_{82}H_{152}FN_2O_{17}P$ ($[M-2H]^{2-}$) Found 742.5323 ; Calcd for $C_{82}H_{150}FN_2O_{17}P$ 742.5334. |
| 17(17a) |
| $^1$H-NMR (CDCla) δ : 7.41-7.31 (5H, m), 5.23-5.07 (3H, m), 5.03-4.90 (2H, m), 4.81-4.57 (2H, m), 4.33-4.20 (1H, m), 3.94-3.85 (1H, m), 3.74-3.53 (4H, m), 3.53-3.19 (4H, m), 2.60 (1H, dd, J = 14.9, 6.3 Hz), 2.36 (1H, dd, J = 15.5, 6.1 Hz), 2.08-1.34 (8H, m), 1.46 (3H, s), 1.43 (9H, s), 1.36 (3H, s), 1.34-1.19 (32H, m), 0.92-0.84 (6H, m). |
| 17(17b) |
| $^1$H-NMR ($CDCl_3$) δ : 7.42-7.31 (5H, m), 5.47 (1H, d, J = 9.4 Hz), 5.14 (2H, s), 4.89 (1H, t, J = 9.8 Hz), 4.73-4.64 (2H, m), 3.86 (1H, dd, J = 11.9, 2.9 Hz), 3.77-3.29 (9H, m), 2.58-2.44 (2H, m), 1.91-1.73 (3H, m), 1.68-1.53 (2H, m), 1.53-1.40 (3H, m), 1.35-1.20 (32H, m), 0.92-0.84 (6H, m). |
| 17(17c) |
| $^1$H-NMR ($CDCl_3$) δ : 7.41-7.30 (5H, m), 7.01 (1H, d, J = 8.2 Hz), 5.24 (1H, d, J = 9.4 Hz), 5.19 (1H, d, J = 12.5 Hz), 5.14 (1H, d, J = 12.1 Hz), 4.84 (1H, t, J = 9.6 Hz), 4.74-4.65 (3H, m), 3.92-3.84 (1H, m), 3.78-3.53 (5H, m), 3.49-3.38 (5H, m), 3.37-3.29 (1H, m), 3.06 (1H, d, J = 3.1 Hz), 2.62-2.31 (5H, m), 2.12-2.01 (1H, m), 1.83-1.70 (2H, m), 1.67-1.39 (9H, m), 1.36-1.19 (64H, m), 0.93-0.83 (12H, m). |
| 17(17d) |
| $^1$H-NMR ($CDCl_3$) δ : 7.39-7.30 (5H, m), 6.97 (1H, d, J = 8.3 Hz), 5.21 (1H, d, J = 12.2 Hz), 5.13 (1H, d, J = 12.2 Hz), 4.79-4.73 (1H, m), 4.67 (1H, t, J = 9.3 Hz), 3.85 (1H, d, J = 11.7 Hz), 3.76-3.68 (2H, m), 3.65-3.59 (1H, m), 3.54 (1H, t, J = 9.3 Hz), 3.49-3.38 (4H, m), 3.32-3.26 (1H, m), 3.18-3.12 (1H, m), 2.98 (1H, brs), 2.71-2.31 (6H, m), 2.03-1.90 (2H, m), 1.90-1.79 (1H, m), 1.70-1.40 (9H, m), 1.36-1.20 (64H, m), 0.92-0.83 (12H, m). |
| 17(17e) |
| $^1$H-NMR ($CDCl_3$) δ : 7.39-7.30 (5H, m), 7.09 (1H, d, J = 7.3 Hz), 6.32 (1H, d, J = 9.3 Hz), 5.18 (1H, d, J = 12.2 Hz), 5.14 (1H, d, J = 12.2 Hz), 4.82 (1H, t, J = 9.8 Hz), 4.63-4.54 (1H, m), 4.05-3.83 (2H, m), 3.78-3.53 (5H, m), 3.46-3.29 (8H, m), 3.08 (1H, d, J = 2.9 Hz), 2.59-2.21 (7H, m), 2.11-2.00 (1H, m), 1.83-1.70 (2H, m), 1.68-1.35 (13H, m), 1.34-1.19 (96H, m), 0.91-0.84 (18H, m). |
| 17(17f) |
| $^1$H-NMR ($CDCl_3$) δ : 7.40-7.27 (10H, m), 7.07 (1H, d, J = 7.4 Hz), 6.38 (1H, d, J = 9.0 Hz), 5.15 (2H, s), 5.13 (2H, s), 4.82 (1H, dd, J = 10.4, 8.8 Hz), 4.49-4.41 (2H, m), 4.33 (1H, dd, J = 11.7, 5.1 Hz), 3.94 (1H, q, J = 9.8 Hz), 3.73-3.30 (12H, m), 3.17 (1H, d, J = 3.1 Hz), 2.58-2.21 (6H, m), 2.11-1.95 (1H, m), 1.84-1.57 (2H, m), 1.56-1.34 (13H. m), 1.34-1.20 (96H, m), 0.92-0.84 (18H, m). |
| 17(17g) |

(continued)

| |
|---|
| $^1$H-NMR ($CDCl_3$) δ : 7.41-7.22 (20H, m), 7.16 (1H, d, J = 7.4 Hz), 6.40 (1H, d, J = 9.0 Hz), 5.13 (2H, s), 5.10 (2H, s), 5.10-4.81 (5H, m), 4.51-4.42 (2H, m), 4.34 (1H, q, J = 9.3 Hz), 4.24 (1H, dd, J = 11.9, 5.3 Hz), 3.92 (1H, q, J = 9.9 Hz), 3.68-3.50 (4H, m), 3.47-3.27 (6H, m), 3.23-3.14 (1H, m), 2.55-2.18 (6H, m), 2.09-1.94 (1H, m), 1.80-1.63 (2H, m), 1.57-1.34 (13H, m), 1.33-1.19 (96H, m), 0.92-0.84 (18H, m). |
| 17(17h) |
| $^1$H-NMR ($CD_3OD$-$CDCl_3$) δ : 7.47 (1H, d, J = 9.0 Hz), 5.07 (1H, t, J = 9.8 Hz), 4.35 (1H, dd, J = 9.4, 3.9 Hz), 4.21 (1H, q, J = 9.8 Hz), 4.00 (1H, d, J = 11.3 Hz), 3.88 (1H, t, J = 10.0 Hz), 3.78-3.63 (4H, m), 3.62-3.26 (8H, m), 2.68 (1H, dd, J = 15.8, 6.8 Hz), 2.54-2.31 (4H, m), 2.22 (1H, dd, J = 13.9, 4.5 Hz), 2.02 (1H, brs), 1.87 (1H, brs), 1.68 (1H, brs), 1.62-1.40 (13H, m), 1.40-1.20 (96H, m), 0.94-0.85 (18H, m).<br>HRMS (ESI Neg) : $C_{82}H_{159}N_2O_{15}P$ ([M-2H]$^{2-}$) Found 720.5665 ; Calcd for $C_{82}H_{159}N_2O_{15}P$ 720.5666. |

[Table 2-12]

| |
|---|
| 18 |
| $^1$H-NMR ($CD_3OD$-$CDCl_3$-$D_2O$) δ : 5.27-5.07 (4H, m), 4.49 (1H, d, J = 8.2 Hz), 4.37-4.25 (2H, m), 4.13-3.54 (12H, m), 2.77-2.58 (3H, m), 2.57-2.27 (12H, m), 2.11-2.00 (1H, m), 1.85-1.72 (1H, m), 1.70-1.50 (12H, m), 1.40-1.08 (90H, m), 0.93-0.86 (18H, m).<br>HRMS (ESI Neg) : $C_{90}H_{164}N_2O_{26}PNa$ ([M-2H]$^{2-}$) Found 871.0535; Calcd for $C_{90}H_{162}N_2O_{26}PNa$ 871.0548.<br>EA : Anal. Calcd for $C_{90}H_{163}N_2O_{26}P.2Na.4H_2O$ : C, 58.80; H, 9.38; N, 1.52; Na, 2.50; P, 1.69.<br>Found : C, 58.55; H, 9.49; N, 1.48; Na, 2.23; P, 1.54. |
| 19 |
| $^1$H-NMR ($CD_3OD$-$CDCl_3$-$D_2O$) δ : 5.27-5.07 (4H, m), 4.49 (1H, d, J = 8.2 Hz), 4.37-4.25 (2H, m), 4.13-3.54 (12H, m), 2.77-2.58 (3H, m), 2.57-2.27 (12H, m), 2.11-2.00 (1H, m), 1.85-1.72 (1H, m), 1.70-1.50 (12H, m), 1.40-1.08 (90H, m), 0.93-0.86 (18H, m).<br>HRMS (ESI Neg) : $C_{90}H_{164}N_2O_{26}PK$ ([M-2H]$^{2-}$) Found 879.0425; Calcd for $C_{90}H_{162}N_2O_{26}PK$ 879.0418. |
| 20 |
| $^1$H-NMR ($CD_3OD$-$CDCl_3$-$D_2O$) δ : 5.29-5.07 (4H, m), 4.97-4.91 (1H, m), 4.35-4.24 (1H, m), 4.08-3.45 (37H, m), 3.30-3.10 (24H, m), 2.84-2.24 (14H, m), 2.08-1.96 (1H, m), 1.86-1.72 (1H, m), 1.71-1.52 (12H, m), 1.50-1.09 (90H, m), 0.99-0.79 (18H, m).<br>HRMS (ESI Neg) : $C_{90}H_{165}N_2O_{26}P$ ([M-2H]$^{2-}$) Found 860.0629; Calcd for $C_{90}H_{163}N_2O_{26}P$ 860.0638 .<br>EA : Anal. Calcd for $C_{90}H_{165}N_2O_{26}P.4C_6H_{15}NO_3.8H_2O$ : C, 55.59; H, 9.86; N, 3.41; P, 1.26.<br>Found : C, 55.35; H, 9.57; N, 3.64; P, 1.11. |
| 21 |
| $^1$H-NMR ($CD_3OD$-$CDCl_3$-$D_2O$) δ : 5.29-5.08 (4H, m), 4.50 (1H, d, J = 7.9 Hz), 4.38-4.27 (1H, m), 4.14-3.52 (19H, m), 3.25-3.07 (2H, m), 2.80-2.25 (14H, m), 2.74 (3H,s), 2.13-2.02 (1H, m), 1.89-1.76 (1H, m), 1.71-1.51 (12H, m), 1.49-1.08 (90H, m), 0.95-0.83 (18H, m).<br>HRMS (ESI Neg) : $C_{90}H_{165}N_2O_{26}P$ ([M-2H]$^{2-}$) Found 860.0652; Calcd for $C_{90}H_{163}N_2O_{26}P$ 860.0638<br>EA : Anal. Calcd for $C_{90}H_{165}N_2O_{26}P.C_7H_{17}NO_5.7H_2O$ : C, 57.01; H, 9.67; N, 2.06; P, 1.52.<br>Found : C, 57.25; H, 9.56; N, 2.31; P, 1.23. |
| 22 |
| $^1$H-NMR ($CD_3OD$-$CDCl_3$-$D_2O$) δ : 5.28-5.07 (4H, m), 4.83-4.37 (1H, m), 4.35-4.24 (1H, m), 4.15-3.42 (25H, m), 3.25-3.06 (4H, m), 2.82-2.24 (14H, m), 2.73 (6H,s), 2.09-1.97 (1H, m), 1.85-1.71 (1H, m), 1.70-1.50 (12H, m), 1.49-1.09 (90H, m), 0.95-0.80 (18H, m).<br>HRMS (ESI Neg) - $C_{90}H_{165}N_2O_{26}P$ ([M-2H]$^{2-}$) Found 860.0630; Calcd for $C_{90}H_{163}N_2O_{26}P$ 860.0638<br>EA : Anal. Calcd for $C_{90}H_{165}N_2O_2eP.2C_7H_{17}NO_5.9H_2O$ : C, 54.91; H, 9.61; N, 2.46; P, 1.36.<br>Found : C, 54.75; H, 9.47; N, 2.49; P, 1.09. |
| 23 |

(continued)

| |
|---|
| $^1$H-NMR ($CD_3OD$-$CDCl_3$-$D_2O$) $\delta$ : 5.28-5.08 (4H, m), 4.55 (1H, d, J = 7.8 Hz), 4.32-4.22 (1H, m), 4.16-3.54 (30H, m), 3.53-3.41 (1H, m), 3.23-3.04 (6H, m), 2.81-2.24 (14H, m), 2.72 (9H,s), 2.06-1.95 (1H, m), 1.86-1.73 (1H, m), 1.71-1.49 (12H, m), 1.48-1.08 (90H, m), 0.96-0.82 (18H, m).<br>HRMS (ESI Neg) : $C_{90}H_{165}N_2O_{26}P$ ([M-2H]$^{2-}$) Found 860.0640; Calcd for $C_{90}H_{163}N_2O_{26}P$ 860.0638<br>EA : Anal. Calcd for $C_{90}H_{165}N_2O_{26}P.3C_7H_{17}NO_5.11H_2O$ : C, 53.20; H, 9.57; N, 2.79; P, 1.24.<br>Found : C, 53.15; H, 9.41; N, 3.05; P, 1.07. |
| 24 |
| $^1$H-NMR ($CD_3OD$-$CDCl_3$-$D_2O$) $\delta$ : 5.30-5.03 (4H, m), 4.55 (1H, d, J = 7.4 Hz), 4.34-4.21 (1H, m), 4.15-3.53 (36H, m), 3.51-3.40 (1H, m), 3.21-3.00 (8H, m), 2.85-2.25 (14H, m), 2.70 (12H, s), 2.05-1.93 (1H, m), 1.87-1.73 (1H, m), 1.70-1.51 (12H, m), 1.49-1.08 (90H, m), 0.98-0.83 (18H, m).<br>HRMS (ESI Neg) : $C_{90}H_{165}N_2O_{26}P$ ([M-2H]$^{2-}$) Found 860.0638; Calcd for $C_{90}H_{163}N_2O_{26}P$ 860.0638<br>EA : Anal. Calcd for $C_{90}H_{165}N_2O_{26}P.4C_7H_{17}NO_58H_2O$ : C, 53.54; H, 9.48; N, 3.17; P, 1.17.<br>Found : C, 53.50; H, 9.22; N, 3.13; P, 1.00. |

(Test Example 1) Human TLR4 activating effect

The human TLR4 activating effect was studied using the compounds described in Examples 1 to 17 and monophosphoryl lipid A as Comparative Example A.

Triethanolamine was dissolved at 0.5% (v/v) in injectable distilled water to prepare an aqueous triethanolamine solution. 1 mg of each test drug was dissolved in 0.98 mL of the aqueous triethanolamine solution, and pH of the solution was then adjusted to 7.2 to 7.4 by the addition of 20 $\mu$L of 1 M HCl to prepare a 1 mg/mL solution. A dilution series of the test drug was prepared using a medium [DMEM (Nacalai Tesque, Inc.), 10% FBS (Sigma-Aldrich Co. LLC), 50 U/mL penicillin-50 $\mu$g/mL streptomycin (Thermo Fisher Scientific Inc.), 1 $\times$ HEK-Blue Selection (InvivoGen), and 100 $\mu$g/mL Normocin (InvivoGen)] . 2 $\times$ $10^5$ cells/mL HEK-Blue (TM) Human TLR4 Cells (InvivoGen) were inoculated to each well of a 96-well microplate for tissue culture (Iwaki), then treated with the test drug, and cultured at 37°C for 24 hours under 5% $CO_2$. The cells thus cultured were centrifuged at 400 $\times$ g at 4°C for 3 minutes. Recombinant SEAP Protein (InvivoGen) was used for a calibration curve. After addition of 180 $\mu$L of QUANTI-Blue (InvivoGen) to each well of 96 Well TC-Treated Microplates (Corning, Inc.), 20 $\mu$L each of the cell culture supernatant and a standard diluting solution was added to each well, and the plate was left standing at 37°C for 4 hours. After reaction, absorbance (wavelength: 655 nm) was measured using a microplate reader (PerkinElmer, Inc., EnSpire), and a SEAP Protein concentration was calculated from the calibration curve and used as an index for TLR4 agonist activity. A drug concentration that induced reaction corresponding to 50% of the maximum reaction was calculated as EC50 (ng/mL) from a linear expression using two drug concentrations between which the drug concentration that induced reaction corresponding to 50% of the maximum reaction in each experiment was positioned, and measured values thereof. The results are shown in Table 3.

[Table 3]

| Test drug | EC50 (ng/mL) |
|---|---|
| Comparative Example A | >10 |
| Example 1(1k) | 0.92 |
| Example 2(2e) | 0.086 |
| Example 3 (3d) | 0.82 |
| Example 4(4d) | 0.56 |
| Example 5(5d) | 0.36 |
| Example 6(6b) | 0.11 |
| Example 7(7c) | 0.67 |
| Example 8(8f) | 0.62 |
| Example 9(9e) | 0.51 |
| Example 10(10r) | 1.0 |

(continued)

| Test drug | EC50 (ng/mL) |
|---|---|
| Comparative Example A | >10 |
| Example 11(11d) | 0.50 |
| Example 12(12d) | 1.3 |
| Example 13(13o) | 2.4 |
| Example 14(14d) | 0.32 |
| Example 15(15d) | 0.77 |
| Example 16(16b) | 1.5 |
| Example 17(17h) | 1.4 |

(Test Example 2) Immunostimulatory effect on ovalbumin (OVA) antigen by sublingual administration

(Test drug preparation)

5 μg of ovalbumin (OVA, Hyglos GmbH, endotoxin free) and 0.01, 0.1, or 1 μg of the compound described in Example 2(2e) were dissolved in 2 μL of distilled water, and the solution was used as a vaccine preparation to study its immunostimulatory effect on the ovalbumin (OVA) antigen by sublingual administration.

(Sublingual administration test)

Each mouse (BALB/c mouse, female, 6 weeks old, Charles River Laboratories Japan, Inc.) was allowed to refrain from eating and drinking from 1 hour before sublingual administration. Then, the mouse was anesthetized with 1 to 4% vaporized isoflurane (Pfizer Inc.). 2 μL of the test drug was sublingually administered thereto, and the test drug was then sublingually maintained by continuous anesthesia for 10 minutes. After awakening, the second administration was performed 1 hour after the initial administration. The mouse was continuously allowed to refrain from eating and drinking for 1 hour after the completion of sublingual administration. The administration described above was performed for 4 weeks at 1-week intervals. Blood was continuously collected from the tail vein at 1-week intervals from 2 weeks after the start of administration, and serum was cryopreserved (-20°C).

(Measurement of anti-OVA IgG and IgA in blood)

50 μL of OVA (Sigma-Aldrich Co. LLC, 1 μg OVA/mL, PBS) was added to each well of 96-well Half Area Clear Flat Bottom Polystyrene High Bind Microplate (Corning, Inc.), which was then left standing overnight at 4°C and then washed three times with a washing solution (0.05% Tween 20 and PBS). 120 μL of an ELISA solution (1% BSA, 0.05% Tween 20, and PBS) was added to each well, and the plate was left standing at room temperature for 1 hour and then washed three times. The serum sample was diluted using an ELISA solution. Anti-OVA Mouse IgG (Chondrex, Inc.) and Anti-OVA Mouse IgA (Chondrex, Inc.) were used as specimens for calibration curves. 50 μL each of the sample and each specimen for a calibration curve was added to each well, and the plate was left standing at room temperature for 1 hour and then washed three times. 50 μL of HRP-labeled anti-mouse IgG (Southern Biotech, 1/4000 diluted) or IgA (Southern Biotech, 1/4000 diluted) was added to each well, and the plate was left standing at room temperature for 1 hour. After washing three times with a washing solution, 50 μL of a TMB substrate (SERACARE Life Sciences Inc.) was added to each well, and the plate was left standing for 10 minutes. The reaction was terminated by the addition of 50 μL/well of a TMB stop solution (SERACARE Life Sciences Inc.). Absorbance at 450 nm was measured using a microplate reader (PerkinElmer, Inc., EnSpire). The amounts of anti-OVA IgG and IgA in blood were calculated from the calibration curves. The detection lower limit values were 0.1 and 0.01 μg/mL for anti-OVA IgG and IgA, respectively. These values were adopted for values of the sample less than the detection limit. The results are shown in Figures 1 and 2.

(Test Example 3) Immunostimulatory effect on various allergens by sublingual administration

The compound described in Example 2(2e) was used to study its immunostimulatory effect on various allergens by sublingual administration.

Japanese cedar pollen antigen extracts were prepared by treating Japanese cedar pollen (Yamizo Pollen Study

Group) with a solution containing 0.125 M NaHCO3 and 0.5 M NaCl for 24 hours (4°C), and then insoluble matter was removed by filtration. The concentration of Cry j 1 contained in the extracts was measured, and the extracts were diluted into 12.5 μg/mL (10000 JAU/mL). Allergen scratch extracts (Torii Pharmaceutical Co., Ltd.) were used as mite, ragweed, timothy, peanut, and milk. Allergen sensitization was performed by intramuscularly administering the allergen (20 μL) to the mouse femoral region 1 week before the start of sublingual administration. The allergen scratch extracts of mite, ragweed, timothy, peanut, or milk were diluted 10-fold with PBS and used in allergen sensitization. Sublingual administration was started from 1 week after sensitization. Each allergen and distilled water or the compound described in Example 2(2e) dissolved at 1 mg/mL in distilled water were mixed in equal amounts immediately before administration to prepare a prototype vaccine preparation. Sublingual administration was performed once a day and three to five times a week for 12 weeks using 2 μL of the prototype vaccine preparation. Blood was collected from the tail vain 4, 8, and 12 weeks after the start of sublingual administration, and separated serum was preserved at -20°C.

(Measurement of allergen-specific IgG)

Each allergen was added at 25 μL/well (Japanese cedar pollen, 1 μg Cry j 1/mL; mite, ragweed, timothy, peanut, and milk, 1:1000 diluted) to a plate for ELISA, which was then left standing overnight at 4°C. 16 to 24 hours later, the plate was washed three times with a washing solution. Then, 100 μL of an ELISA solution was added to each well, and the plate was left standing at room temperature for 1 hour and then washed three times. A dilution series of the serum sample was prepared as 8 serial dilutions at a dilution ratio of 1/2 with 1/128 diluted serum as the highest concentration using an ELISA solution. 25 μL of the sample was added to each well of the plate, and the plate was left standing at room temperature for 1 hour and then washed three times. HRP-labeled anti-mouse IgG was diluted at 1:10000 with an ELISA solution and added at 25 μL/well, and the plate was left standing at room temperature for 1 hour and then washed three times. 30 μL of a TMB substrate was added to each well, and the plate was left standing for 10 minutes. Then, 30 μL of a TMB stop solution was added to each well, and absorbance at 450 nm was measured. An appropriate OD value was set for each allergen, and the dilution ratio of serum that reached the OD value is shown as an allergen-specific IgG titer. The results are shown in Figures 3 to 8.

No marked increase in allergen-specific IgG titer was observed up to 12 weeks after the start of sublingual administration in any group without sublingual administration (control) of mice sensitized with the allergen. For all the allergens, a markedly higher allergen-specific IgG titer was induced in the groups sublingually given the compound described in Example 2(2e) than in the case of sublingually administering the allergen alone.

(Test Example 4) Immunostimulatory effect on Japanese cedar pollen antigen by sublingual administration

The compound described in Example 24 was used to study its immunostimulatory effect on a Japanese cedar pollen antigen by sublingual administration.

(Allergen immunotherapy model)

Allergen sensitization was performed by subcutaneously administering 50 μL of Japanese cedar pollen antigen extracts (allergen scratch extracts "Torii" Japanese cedar pollen, Torii Pharmaceutical Co., Ltd.) to the mouse tail root 3 and 1 weeks before the start of sublingual administration. Blood was collected 4 days after the second sensitization, and anti-Cry j 1 IgG was measured in accordance with the subsequent section. Mice were grouped such that their measured values were evenly distributed among the groups. Sublingual administration (sublingual allergen immunotherapy model) was started from 3 days thereafter.

The antigen used was a Japanese cedar pollen antigen (5000 JAU CEDARCURE[R], Torii Pharmaceutical Co., Ltd.) dissolved in 100 μL of PBS. The compound of Example 24 was dissolved in injectable water (Otsuka Pharmaceutical Co., Ltd.) to prepare a 5 mg/mL solution. The antigen and the compound of Example 24 were mixed in equal amounts immediately before administration.

Each mouse was allowed to refrain from eating and drinking from 1 hour before sublingual administration. 2 μL of the prototype vaccine preparation was sublingually administered thereto twice at a 5-minute interval under isoflurane anesthesia and further awakened 5 minutes later. The sublingual administration described above was performed twice at a 1-hour interval, and feeding with food and water was further resumed from 1 hour thereafter. This administration was performed three times a week.

Blood was collected from the tail vein 3 and 4 weeks after the start of sublingual administration. Blood, nasal wash, and neck lymph node were collected 5 weeks after the start of sublingual administration.

(Measurement of anti-Cry j 1 IgG or IgA)

25 μL of 10 μg/mL ImmunoPure Streptavidin (Thermo Fisher Scientific Inc., Cat No. 21125) was added to each well of 96 Well Micro Plate (Corning, Inc., Cat No. 3690), which was then left standing overnight at 4°C. After washing three times with a washing solution, 100 μL of an ELISA solution was added to each well, and the plate was left standing at room temperature for 1 hour. After washing three times, 25 μL of 1 μg/mL biotinylated Cry j 1 (BioDynamics Laboratory Inc., Cat No. HBL-BC-1) was added to each well, and the plate was left standing at room temperature for 1 hour. After washing three times, 25 μL of the sample was added to each well, and the plate was left standing at room temperature for 1 hour. After washing three times, 25 μL of HRP-labeled anti-mouse IgG (1/8000) or HRP-labeled anti-mouse IgA (1/4000) was added to each well, and the plate was left standing at room temperature for 1 hour. After washing three times, 30 μL of TMB Microwell Peroxidase Substrate System was added to each well. The plate was left standing at room temperature for 10 minutes, and 30 μL of TMB Stop Solution was then added to each well, followed by the measurement of absorbance at 450 nm.

Positive serum was used as a standard specimen of serum, and anti-Cry j 1 IgG or IgA contained therein was set to 1000 units/mL. Six serial dilutions were prepared by 4-fold dilution from 100-fold dilution of this standard specimen, and a calibration curve was prepared. An evaluation specimen was diluted 1000-fold with an ELISA solution and measured, and the unit value of anti-Cry j 1 IgG or IgA in the evaluation specimen was determined using the calibration curve. The results are shown in Figures 9 and 10.

Nasal washes collected from four individuals in a positive group were mixed in equal amounts, and a pooled specimen thereof was used as a standard specimen of nasal wash. Anti-Cry j 1 IgA contained therein was set to 1000 units/mL. Twelve serial dilutions were prepared by 2-fold dilution from undiluted solution of this standard specimen, and a calibration curve was prepared. An evaluation specimen was diluted 8-fold with an ELISA solution and measured, and the unit value of anti-Cry j 1 IgA in the evaluation specimen was determined using the calibration curve. The results are shown in Figure 11.

Anti-Cry j 1 IgG (Figure 9) and anti-Cry j 1 IgA (Figure 10) in serum were induced at a higher level in the mice sublingually given the Japanese cedar pollen antigen and the compound of Example 24 (Japanese Cedar Pollen + Compound 24 SLIT group) than in mice without sublingual administration (No-SLIT group) and mice sublingually given the Japanese cedar pollen antigen alone (Japanese Cedar Pollen SLIT group). Anti-Cry j 1 IgA in nasal wash was induced at a high level in the Japanese Cedar Pollen + Compound 24 SLIT group (Figure 11) .

(Cry j 1-specific cell-mediated immunity assay)

Cervical lymph node was collected, then pooled for each group, and ground in PBS (1% BSA). The resultant was passed through a 70 μm strainer. After addition of 5 mL of RPMI/FBS/ps (RPMI 1640, 10% FBS, and 100 units/mL penicillin and streptomycin), the mixture was centrifuged at 400 g for 3 minutes. Cells were recovered with RPMI/FBS/ps, and the number of cells was prepared to $2 \times 5 \times 10^6$ cells/mL. After inoculation at 40 μL/well to a 96-well U-plate, 40 μL of RPMI or 40 μL of RPMI/FBS/ps containing $2 \times 5$ μg/mL Cry j 1 was added to each well, and the cells were cultured at 37°C for 44 hours. IL-10, IFN-γ, and IL-4 contained in the culture supernatant were measured using Mouse Th1/Th2/Th17 Cytokine Kit (BD Cytometric Bead Array). The results are shown in Figures 12 to 14.

IL-10 was strongly induced in the Japanese Cedar Pollen + Compound 24 SLIT group by Cry j 1 stimulation (Figure 12). On the other hand, both IFN-γ (Figure 13) and IL-4 (Figure 14) were at or below the detection limit (9.77 pg/mL).

(Measurement of mast cell degranulation by Cry j 1, and suppression of degranulation by immune serum-derived IgG)

Each mouse (C57BL/6J, 8 months old, male, Charles River Laboratories Japan, Inc.) was euthanized by exsanguination under isoflurane anesthesia. 10 mL of ice-cold RPMI/BSA/hep (RPMI 1640, 1 mg/mL BSA, and 10 units/mL heparin) was intraperitoneally injected thereto and mildly massaged for approximately 60 minutes, and peritoneal wash was recovered. The peritoneal washes from 4 individuals were pooled and centrifuged at 400 g for 3 minutes. Then, the supernatant was discarded, and peritoneal cells were suspended in 1 mL of RPMI/BSA/hep. The RPMI/BSA/hep was heated to 37°C, and 0.235 g/mL Histodenz was dissolved therein to prepare a Histodenz solution. 2 mL of the Histodenz solution was added to a 15 mL tube, and 1 mL of the peritoneal cell suspension was further layered thereon, followed by centrifugation at 400 g for 15 minutes. A cell group contained in the intermediate layer was discarded, and peritoneal mast cells at the bottom of the tube was recovered with 15 mL of RPMI/FBS/ps (RPMI 1640, 10% FBS, and 100 units/mL penicillin-streptomycin). The cells were centrifuged at 400 g for 3 minutes, then suspended in 5 mL of RPMI/FBS/ps, and inoculated at 0.5 to 1 mL/well to a 24-well plate, and immediately thereafter, 5 μg/mL Cry j 1-01-F11 mouse monoclonal IgE (mIgE) or 5 μg/mL Cry j 1-02-F02 mIgE, or both, were added to each well. The cells were cultured at 37°C for 24 hours.

10 mL of Tyrode's solution (Sigma Aldrich Co. LLC, T2145-10X1L) was added to the mast cells, and the mixture was centrifuged at 400 g for 3 minutes. The cells were suspended at approximately $10^6$ cells/mL in Tyrode's solution,

and 10 μL of the suspension was added to a 1.5 mL tube (Eppendorf) and left standing at room temperature until test substance treatment. A test substance (Cry j 1 [Hayashibara Co., Ltd., HBL-C-1]) was prepared with Tyrode's solution such that its concentration was twice the final concentration. Also, a 2 × 1% Triton-X100 solution was prepared with Tyrode's solution. 10 μL of the test substance or Triton-X100 was added to the cells, and the tube was immediately left standing at 37°C. 10 minutes later, the reaction was stopped by immediate ice cooling. After centrifugation at 400 g for 3 minutes, 10 μL of the supernatant was added to 50 μL of 4-nitrophenyl N-acetyl-b-D-glucosaminide (Sigma Aldrich Co. LLC, beta-N-Acetylglucosaminidase Assay Kit). The sample for background measurement used was 4-nitrophenyl N-acetyl-b-D-glucosaminide supplemented with 10 μL of Tyrode's solution alone. The resultant was left standing at 37°C for 1 hour, and the reaction was then terminated by the addition of 100 μL of sodium carbonate, followed by the measurement of absorbance at 405 nm. A microplate reader (EnSpire [PerkinElmer, Inc.]) was used in measurement.

The amount of the mast cell degranulation (%) was calculated according to the following expression.

$$\text{Amount of degranulation (\%)} = [\text{OD (Sample)} - \text{OD (Background)}]/\ \text{OD (Triton-X100)} - \text{OD (Background)}]$$

Mast cells unsensitized with IgE or sensitized with only one of two monoclonal IgEs (Cryj1-01-F11 mIgE or Cryj1-02-F02 mIgE) were not degranulated by Cry j 1 (Figure 15). This was presumably because the crosslink between IgE and FcεRI mediated by Cry j 1 was not formed. On the other hand, the mast cells sensitized with two clones were degranulated in a Cry j 1 concentration-dependent manner by Cry j 1 stimulation (Figures 15 and 16) .

IgG was purified from 200 μL of serum of each mouse individual of the allergen immunotherapy model in accordance with the protocol of Protein G HP spin trap (Cytiva, 28-9031-34). The solvent in the purified IgG was replaced with 50 μL of Tyrode's solution using Vivaspin 500, 10 kDa MWCO Polyethersulfone (Cytiva, 28-9322-25). Cry j 1 (2 × 10 × 200 ng/mL, 1.5 μL, Tyrode's solution) was added to the purified IgG (13.5 μL, Tyrode's solution), and the mixture was left standing at room temperature for 1 hour in the dark. The mast cells sensitized with anti-Cry j 1 IgE were treated with 10 μL of this specimen as a test substance, and the amount of the mast cell degranulation was measured (final concentration: 200 ng/mL, Cry j 1 stimulation).

The IgG purified from the mouse serum of the Japanese Cedar Pollen + Compound 24 SLIT group suppressed mast cell degranulation reaction mediated by anti-Cry j 1 IgE at a significantly low level as compared with the No-SLIT group and the Japanese Cedar Pollen SLIT group (Figure 17).

(Test Example 5) Immunostimulatory effect on SAPS-CoV-2 RBD by sublingual administration

The antigen used was recombinant RBD (receptor binding domain) protein (Recombinant 2019-nCoV RBD protein, Sino Biological Inc., Cat. No. 40592-V08H) of novel coronavirus (severe acute respiratory syndrome coronavirus 2: SARS-CoV-2) dissolved at 1 mg/mL. The compound described in Example 2(2e) was dissolved in a 0.5% aqueous triethanolamine solution and neutralized with HCl to prepare a 1 mg/mL solution. The antigen and the compound of Example 2(2e) were mixed in equal amounts immediately before administration. Sublingual administration (2 μL) was performed at twice/day/week. Blood and nasal wash were collected 6 weeks after the initial administration. Nasal washing was performed with 200 μL of PBS.

(Measurement of anti-RBD IgG and IgA in blood)

25 μL of 10 μg/mL streptavidin (Thermo Fisher Scientific Inc., Cat# 21125, dissolved in PBS) was added to each well of an ELISA plate, which was then left standing overnight at 4°C and then washed three times. 100 μL of an ELISA solution was added to each well, and the plate was left standing at room temperature for 1 hour and then washed three times. 25 μL of 0.2 μg/mL recombinant RBD protein (Aero Biosystems, Cat. No. SPD-C82E9) prepared with an ELISA solution was added to each well, and the plate was left standing at room temperature for 1 hour and then washed three times. 25 μL of serum diluted with an ELISA solution was added to each well, and the plate was left standing at room temperature for 1 hour and then washed three times. 25 μL of HRP-labeled anti-mouse IgG (1/10000 diluted) or HRP-labeled anti-mouse IgA (1/4000 diluted) diluted with an ELISA solution was added to each well, and the plate was left standing at room temperature for 1 hour and then washed three times. 30 μL of a TMB substrate was added to each well, and the plate was left standing for 10 minutes. Then, the reaction was terminated by the addition of 30 μL of a TMB stop solution. Absorbance at 450 nm was measured. Serum of a mouse given the RBD and compound of Example 2(2e) was used as a standard specimen, and anti-RED IgG and IgA contained in the standard serum were each set to 1000 units/mL to prepare calibration curves. Anti-RBD IgG and IgA in each individual sample were calculated. Values of samples equal to or lower than the lower limit value in the calibration curves were regarded as the lower limits 0.78

units/mL (anti-RED IgG) and 15.6 units/mL (anti-RED IgA) in the calibration curves.

(Measurement of anti-RED IgA in nasal wash)

0.2 μg/mL recombinant RBD protein (Aero Biosystems, SPD-C82E9) was immobilized on a solid phase in the same manner as in the preceding paragraph, and 25 μL of nasal washes diluted at 1/2 with an ELISA solution was added thereto. Subsequent procedures were performed in the same manner as in the preceding paragraph. Absorbance at 450 nm is shown in results.

(Inhibitory activity against RBD-hACE2 binding in nasal wash)

25 μL of 10 μg/mL streptavidin (Thermo Fisher Scientific Inc., Cat# 21125, dissolved in PBS) was added to each well of an ELISA plate, which was then left standing overnight at 4°C and then washed three times. 100 μL of an ELISA solution was added to each well, and the plate was left standing at room temperature for 1 hour and then washed three times. 0.04 μg/mL recombinant RBD protein (Aero Biosystems, SPD-C82E9) was immobilized on the solid phase, and the plate was washed three times. 25 μL of nasal wash was added to each well, and the plate was left standing at room temperature for 1 hour and then washed three times. 25 μL of 0.1 μg/mL recombinant hACE2 protein (Aero Biosystems, Cat. No. AC2-H5257) diluted with an ELISA solution was added to each well, and the plate was left standing at room temperature for 1 hour and then washed three times. 25 μL of HRP-labeled antihuman IgG1 (Cygnus Technologies Inc., Cat. No. IM50) diluted 500-fold with an ELISA solution was added to each well, and the plate was left standing at room temperature for 1 hour and then washed three times. 30 μL of a TMB substrate was added to each well, and the plate was left standing for 10 minutes. Then, the reaction was terminated by the addition of 30 μL of a TMB stop solution. Absorbance at 450 nm is shown in results.

(Results)

The results of the tests are shown in Figures 18 to 21. Anti-RBD IgG or IgA in blood was at or below the detection limit when the recombinant RBD protein alone was sublingually administered. By contrast, the amounts of anti-RBD IgG (Figure 18) and IgA (Figure 19) in blood were markedly high in the mice sublingually given the compound described in Example 2(2e) at the same time therewith. Also, anti-RBD IgA was induced at a high level in the nasal wash of the mice sublingually given the compound described in Example 2(2e) and the recombinant RBD protein at the same time (Figure 20). The interaction of novel coronavirus surface RBD with hACE2 of host cells is important for viral infection. The nasal washes of the mice sublingually given the compound described in Example 2(2e) and the recombinant RBD protein at the same time inhibited the binding between RBD and hACE2 (Figure 21). These results suggested the possibility that the sublingual administration of the compound described in Example 2(2e) at the same time with the recombinant RBD protein induces RBD-specific IgA in the nasal cavity and efficiently inhibits the binding between RBD and hACE2 in the nasal cavity serving as a viral entrance door.

## Claims

**1.** A compound represented by the general formula (I):

[Formula 1]

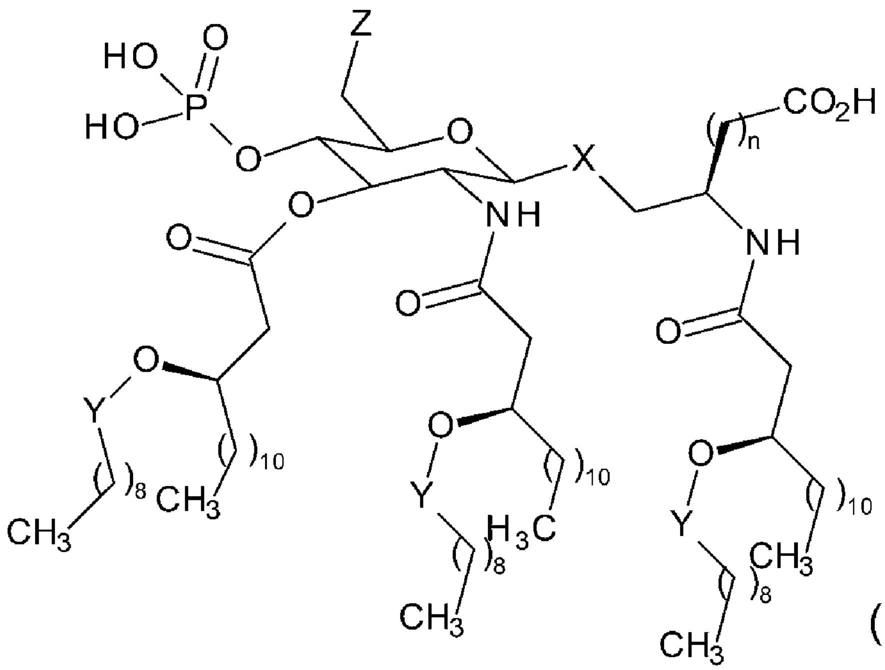

(I)

wherein
X represents an oxygen atom or $CH_2$,
Y represents $CH_2$ or C=O,
Z represents a halogen atom or $OR^1$,
$R^1$ represents a hydrogen atom or the following formula (II) :

[Formula 2]

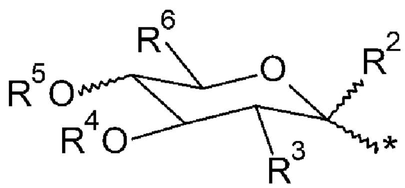

(II)

Wherein
$R^2$ represents a hydrogen atom or a carboxy group,
$R^3$ represents a hydrogen atom, a hydroxy group, or an acetylamino group,
$R^4$ represents a hydrogen atom or the following formula (III) :

[Formula 3]

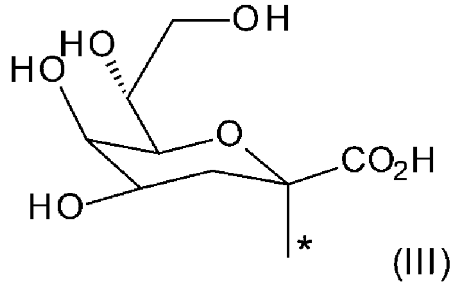

(III)

$R^5$ represents a hydrogen atom or a phosphoric acid group, and
$R^6$ represents a hydroxymethyl group, a methyl phosphate group, a carboxy group, or a (1S)-1,2-dihydroxyethyl group, and

n represents 0 or 1,
or a pharmaceutically acceptable salt thereof, with the proviso that a compound is excluded wherein X represents an oxygen atom, n represents 0, Z represents $OR^1$, and $R^1$ represents a hydrogen atom.

**2.** The compound according to claim 1 or a pharmaceutically acceptable salt thereof, wherein

in the formula (I),
X represents an oxygen atom or $CH_2$,
Y represents C=O,
Z represents the following formula (IV):

[Formula 4]

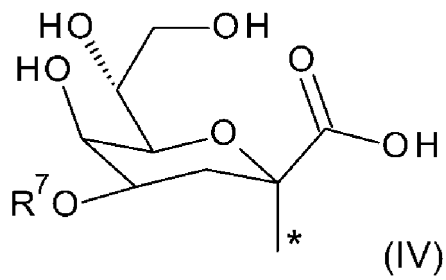

(IV)

wherein
$R^7$ represents a hydrogen atom or the formula (III), and
n represents 0 or 1,
with the proviso that a compound is excluded wherein X represents $CH_2$, n represents 1, and $R^7$ represents the formula (III).

**3.** The compound according to claim 1 or a pharmaceutically acceptable salt thereof, wherein in the formula (I),

X represents an oxygen atom,
Y represents C=O,
Z represents the following formula (V),

[Formula 5]

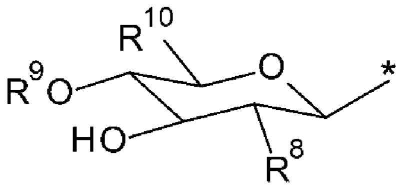

(V)

wherein
$R^8$ represents a hydroxy group or an acetylamino group,
$R^9$ represents a hydrogen atom or a phosphoric acid group, and
$R^{10}$ represents a hydroxymethyl group, a methyl phosphate group, or a carboxy group, and

n represents 0.

4. Any one compound selected from the following group:

(3R)-3-{[(3R)-3-(decanoyloxy)tetradecanoyl]amino}-4-({3-O-[(3R)-3-(decanoyloxy)tetradecanoyl]-2-{[(3R)-3-(decanoyloxy)tetradecanoyl]amino}-2-deoxy-6-O-(3-deoxy-α-D-manno-oct-2-ulopyranonosyl)-4-O-phosphono-β-D-glucopyranosyl}oxy)butanoic acid,
(2S)-2-{[(3R)-3-(decanoyloxy)tetradecanoyl]amino}-3-{[3-deoxy-α-D-manno-oct-2-ulopyranonosyl-(2→4)-3-deoxy-α-D-manno-oct-2-ulopyranonosyl-(2→6)-3-O-[(3R)-3-(decanoyloxy)tetradecanoyl]-2-{[(3R)-3-(decanoyloxy)tetradecanoyl]amino}-2-deoxy-4-O-phosphono-P-D-glucopyranosyl]oxy}propanoic acid,
(2S)-2-{[(3R)-3-(decanoyloxy)tetradecanoyl]amino}-3-({3-O-[(3R)-3-(decanoyloxy)tetradecanoyl]-2-{[(3R)-3-(decanoyloxy)tetradecanoyl]amino}-2-deoxy-6-O-D-glucopyranuronosyl-4-O-phosphono-(3-D-glucopyranosyl}oxy)propanoic acid,
6,10-anhydro-8-O-[(3R)-3-(decanoyloxy)tetradecanoyl]-3,7-bis{[(3R)-3-(decanoyloxy)tetradecanoyl]amino}-2,3,4,5,7-pentadeoxy-11-O-(3-deoxy-α-D-manno-oct-2-ulopyranonosyl)-9-O-phosphono-D-erythro-L-galacto-undecanoic acid, and
5,9-anhydro-7-O-[(3R)-3-(decanoyloxy)tetradecanoyl]-2,6-bis{[(3R)-3-(decanoyloxy)tetradecanoyl]amino}-2,3,4,6-tetradeoxy-10-O-(3-deoxy-α-D-manno-oct-2-ulopyranonosyl)-8-O-phosphono-D-erythro-L-galacto-decanoic acid,
or a pharmaceutically acceptable salt thereof.

5. (3R)-3-{[(3R)-3-(Decanoyloxy)tetradecanoyl]amino}-4-({3-O-[(3R)-3-(decanoyloxy)tetradecanoyl]-2-{[(3R)-3-(decanoyloxy)tetradecanoyl]amino}-2-deoxy-6-O-(3-deoxy-a-D-manno-oct-2-ulopyranonosyl)-4-O-phosphono-(3-D-glucopyranosyl}oxy)butanoic acid or a pharmaceutically acceptable salt.

6. A pharmaceutical composition comprising a compound according to any one of claims 1 to 5 or a pharmaceutically acceptable salt thereof.

7. A pharmaceutical composition comprising a compound according to any one of claims 1 to 5 or a pharmaceutically acceptable salt thereof and an antigen.

8. A pharmaceutical composition wherein a compound according to any one of claims 1 to 5 or a pharmaceutically acceptable salt thereof and an antigen are administered in combination at the same time or at different times.

9. The pharmaceutical composition according to claim 7 or 8, wherein the antigen is one or more selected from the group consisting of an attenuated virus, an inactivated virus, and a recombinant protein of a viral structural protein of influenza virus, adenovirus, rubella virus, mumps virus, RS virus, enterovirus, rotavirus, norovirus, or coronavirus, Japanese cedar pollen, cypress pollen, birch pollen, ragweed pollen, goldenrod pollen, Japanese hop pollen, orchard grass pollen, spinach pollen, black pine pollen, narrow leaf cattail pollen, red pine pollen, chrysanthemum pollen, artemisia pollen, timothy pollen, Bermuda grass pollen, Kentucky grass pollen, meadow fescue grass pollen, orchard

grass pollen, redtop grass pollen, perennial ryegrass pollen, sweet vernal grass pollen, fat hen pollen, mite, cat hair, chicken egg, milk, peanut, wheat, and buckwheat.

**10.** The pharmaceutical composition according to any one of claims 6 to 9 for the prevention or treatment of viral infection, allergy disease, bacterial infection and bacterium-derived toxin, cancer, or intracellular parasitic protozoa.

**11.** The pharmaceutical composition according to any one of claims 6 to 9 for the prevention or treatment of influenza virus, coronavirus, RS virus, norovirus, or rotavirus infection.

**12.** The pharmaceutical composition according to any one of claims 6 to 9 for the prevention or treatment of allergy disease caused by Japanese cedar pollen, cypress pollen, birch pollen, ragweed pollen, goldenrod pollen, Japanese hop pollen, orchard grass pollen, spinach pollen, black pine pollen, narrow leaf cattail pollen, red pine pollen, chrysanthemum pollen, artemisia pollen, timothy pollen, Bermuda grass pollen, Kentucky grass pollen, meadow fescue grass pollen, orchard grass pollen, redtop grass pollen, perennial ryegrass pollen, sweet vernal grass pollen, fat hen pollen (*Chenopodium album* [white goosefoot or lamb's quarters]), mite, cat hair, chicken egg, milk, peanut, wheat, or buckwheat.

**13.** A TLR4 activator comprising a compound according to any one of claims 1 to 5 or a pharmaceutically acceptable salt thereof.

**14.** An immunostimulant comprising a compound according to any one of claims 1 to 5 or a pharmaceutically acceptable salt thereof.

**15.** The immunostimulant according to claim 14, wherein the immunostimulant is a vaccine adjuvant.

FIG. 1

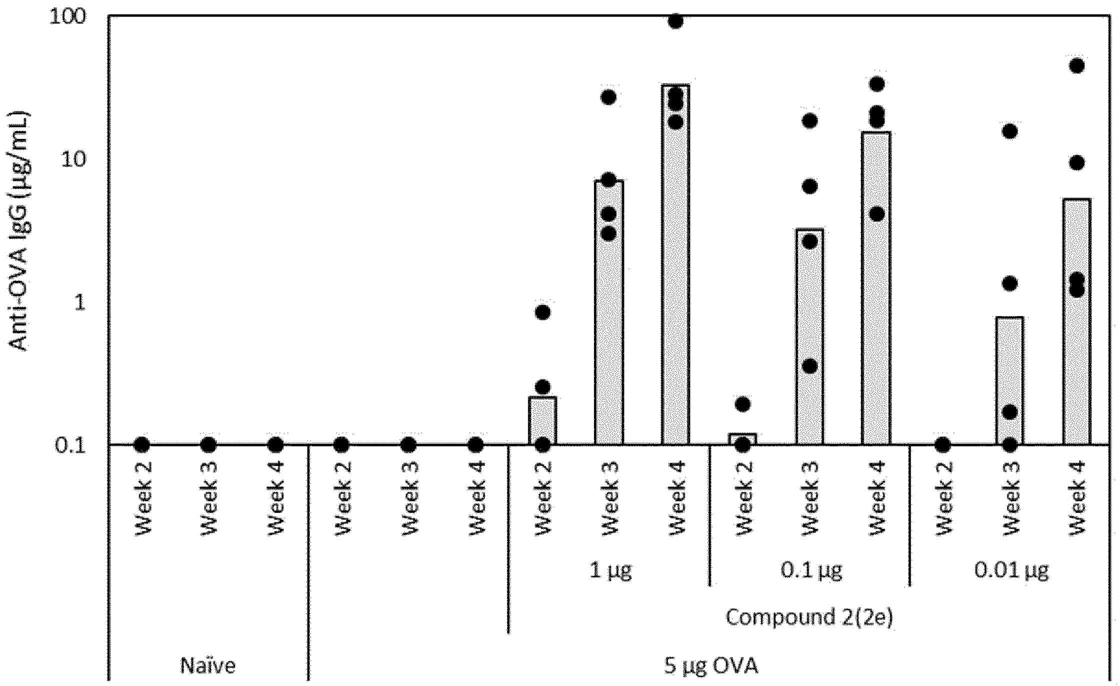
100
10
1
0.1
Anti-OVA IgG (µg/mL)
Week 2
Week 3
Week 4
Week 2
Week 3
Week 4
Week 2
Week 3
Week 4
Week 2
Week 3
Week 4
Week 2
Week 3
Week 4
1 µg
0.1 µg
0.01 µg
Compound 2(2e)
Naïve
5 µg OVA

FIG. 2

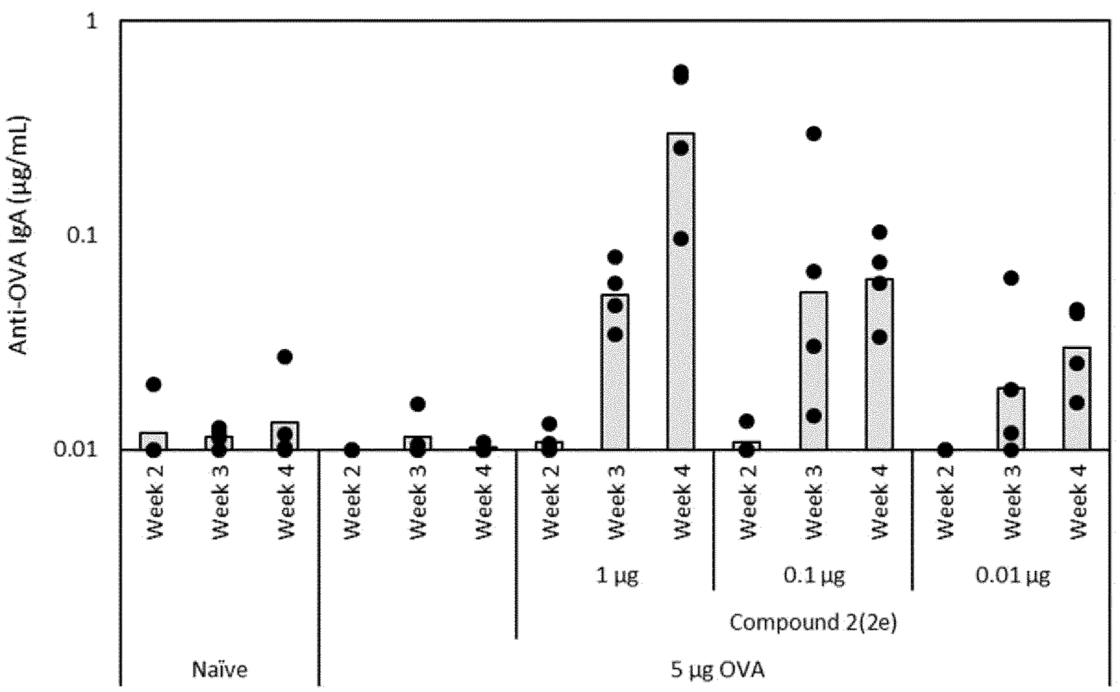
1
0.1
0.01
Anti-OVA IgA (µg/mL)
Week 2
Week 3
Week 4
Week 2
Week 3
Week 4
Week 2
Week 3
Week 4
Week 2
Week 3
Week 4
Week 2
Week 3
Week 4
1 µg
0.1 µg
0.01 µg
Compound 2(2e)
Naïve
5 µg OVA

FIG. 3

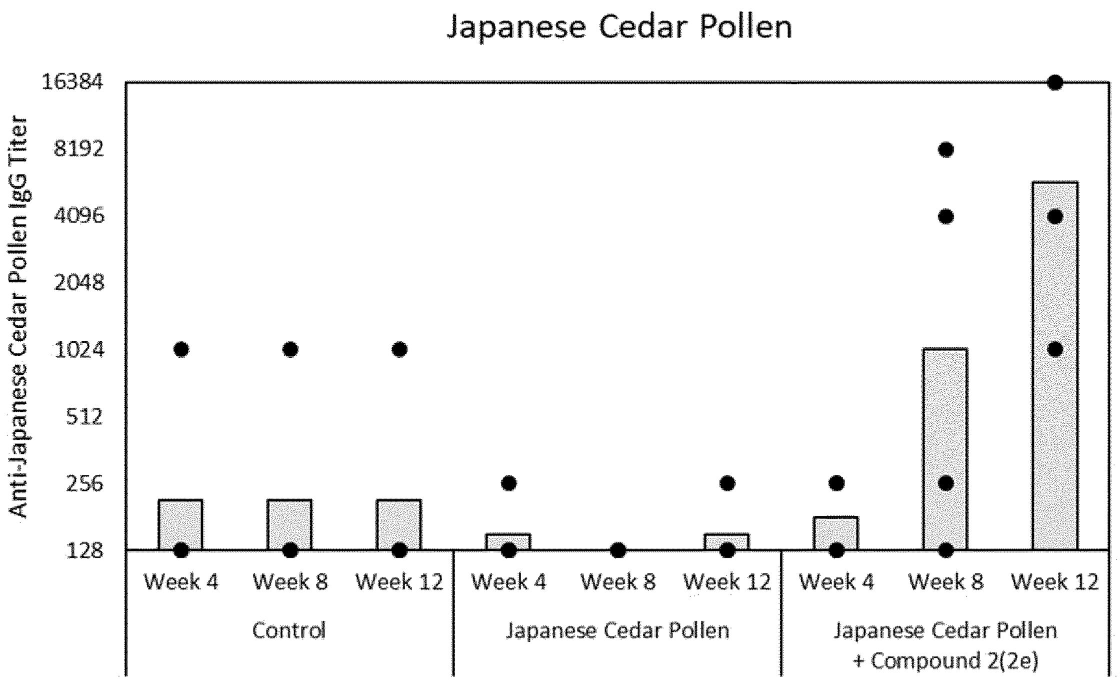
Japanese Cedar Pollen
Anti-Japanese Cedar Pollen IgG Titer
16384
8192
4096
2048
1024
512
256
128
Week 4
Week 8
Week 12
Week 4
Week 8
Week 12
Week 4
Week 8
Week 12
Control
Japanese Cedar Pollen
Japanese Cedar Pollen
+ Compound 2(2e)

FIG. 4

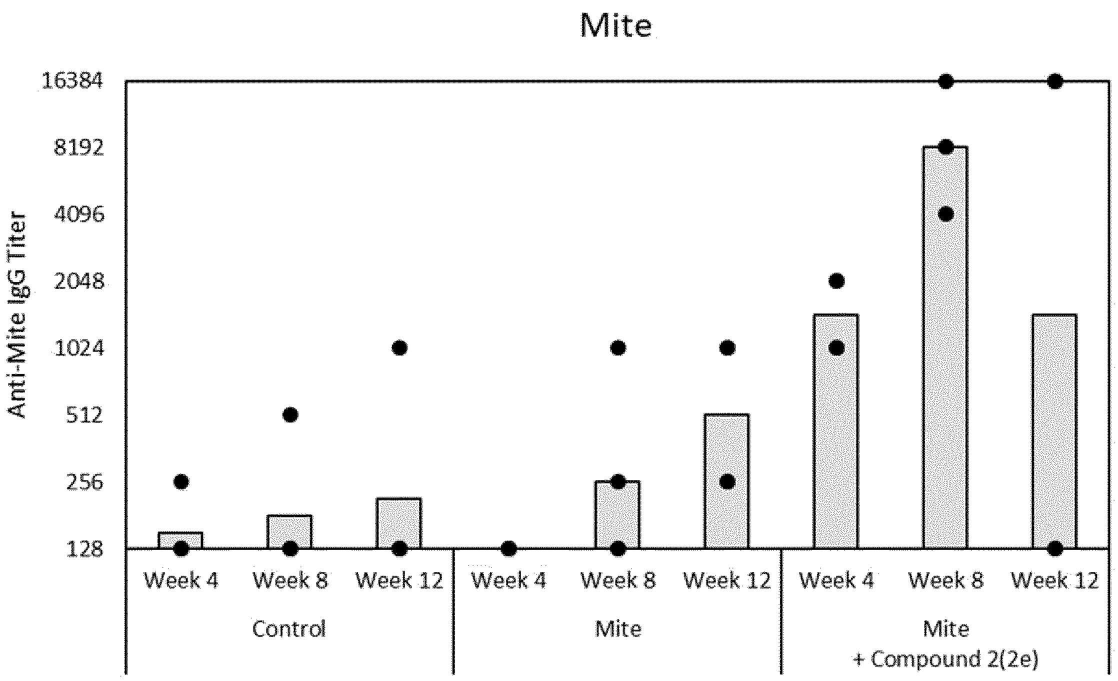
Mite
Anti-Mite IgG Titer
16384
8192
4096
2048
1024
512
256
128
Week 4
Week 8
Week 12
Week 4
Week 8
Week 12
Week 4
Week 8
Week 12
Control
Mite
Mite
+ Compound 2(2e)

FIG. 5

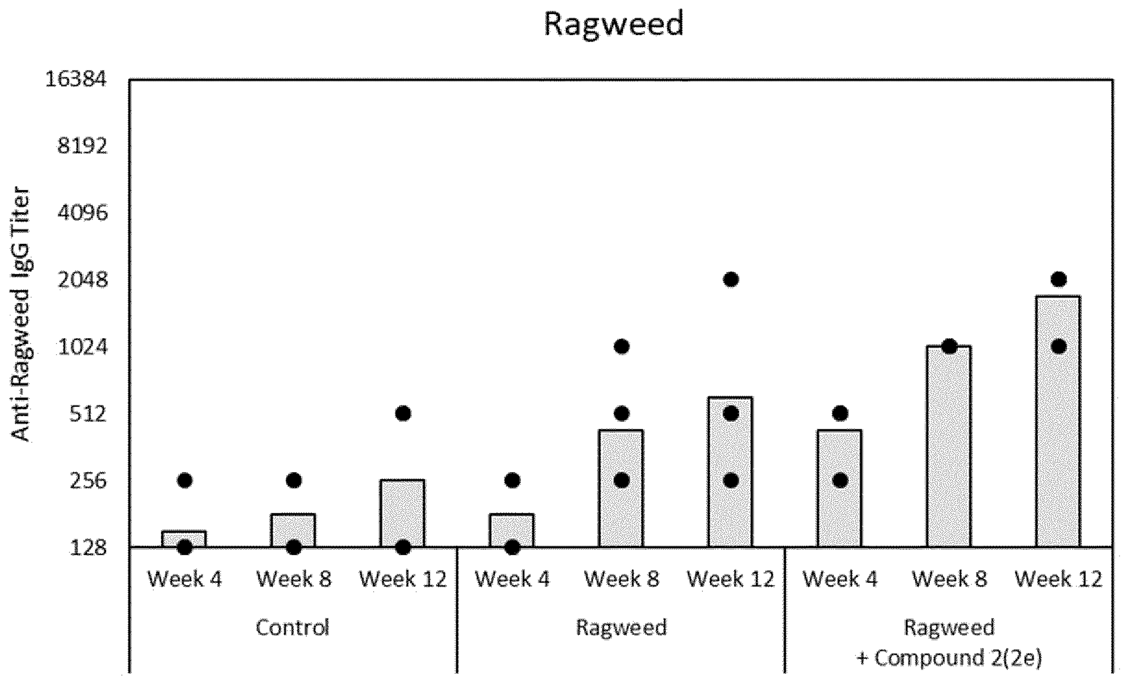
Ragweed
Anti-Ragweed IgG Titer
16384
8192
4096
2048
1024
512
256
128
Week 4
Week 8
Week 12
Week 4
Week 8
Week 12
Week 4
Week 8
Week 12
Control
Ragweed
Ragweed
+ Compound 2(2e)

FIG. 6

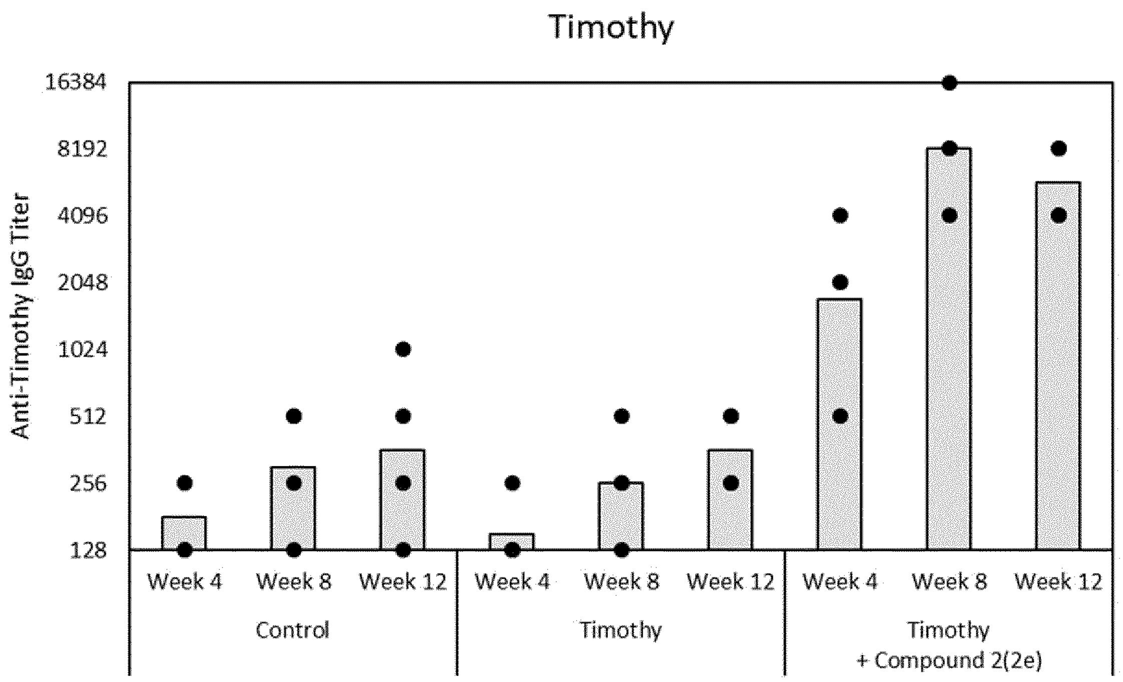
Timothy
Anti-Timothy IgG Titer
16384
8192
4096
2048
1024
512
256
128
Week 4
Week 8
Week 12
Week 4
Week 8
Week 12
Week 4
Week 8
Week 12
Control
Timothy
Timothy
+ Compound 2(2e)

FIG. 7

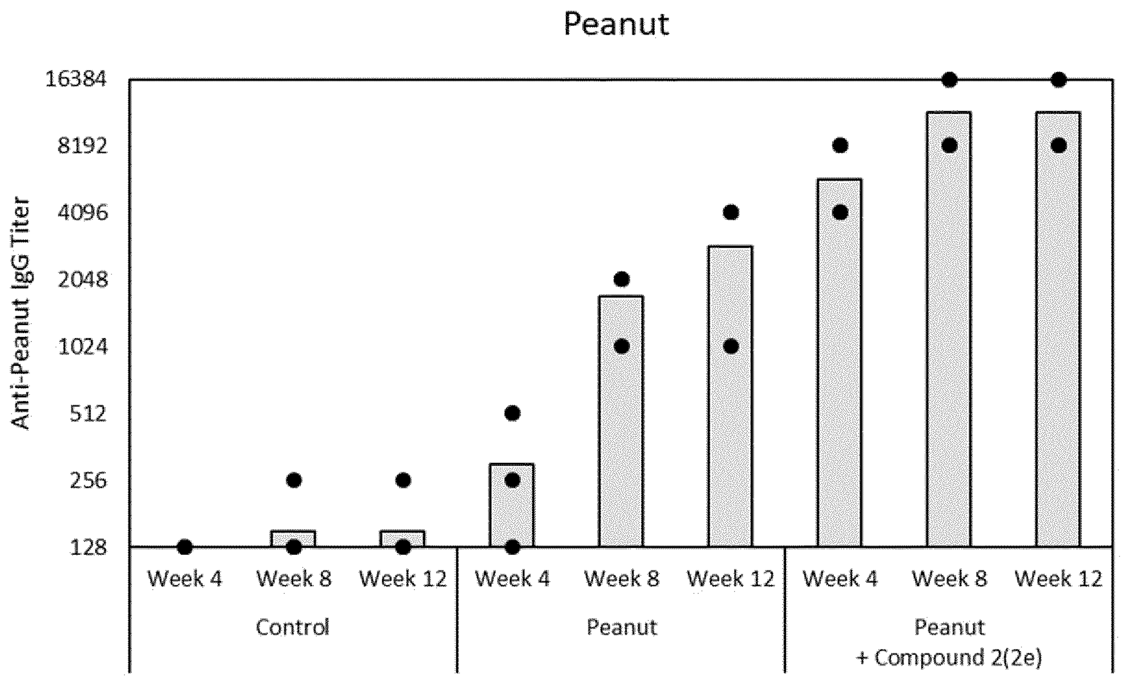
Peanut
Anti-Peanut IgG Titer
16384
8192
4096
2048
1024
512
256
128
Week 4
Week 8
Week 12
Week 4
Week 8
Week 12
Week 4
Week 8
Week 12
Control
Peanut
Peanut + Compound 2(2e)

FIG. 8

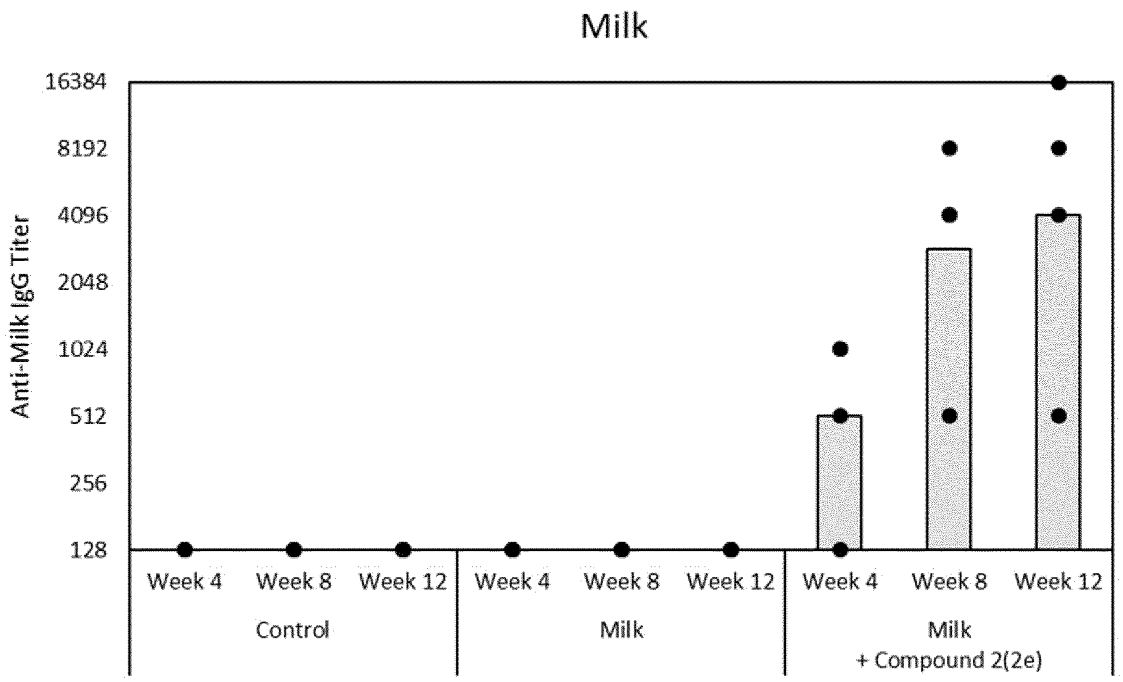
Milk
Anti-Milk IgG Titer
16384
8192
4096
2048
1024
512
256
128
Week 4
Week 8
Week 12
Week 4
Week 8
Week 12
Week 4
Week 8
Week 12
Control
Milk
Milk + Compound 2(2e)

FIG. 9

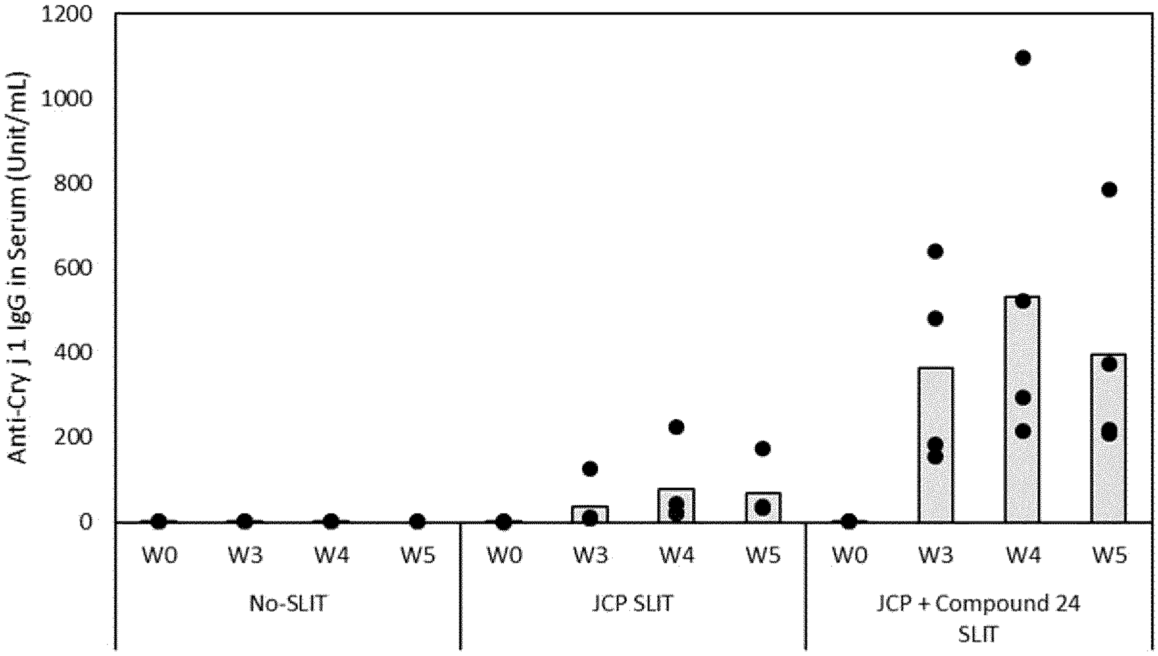
Anti-Cry j 1 IgG in Serum (Unit/mL)
1200
1000
800
600
400
200
0
W0
W3
W4
W5
W0
W3
W4
W5
W0
W3
W4
W5
No-SLIT
JCP SLIT
JCP + Compound 24 SLIT

FIG. 10

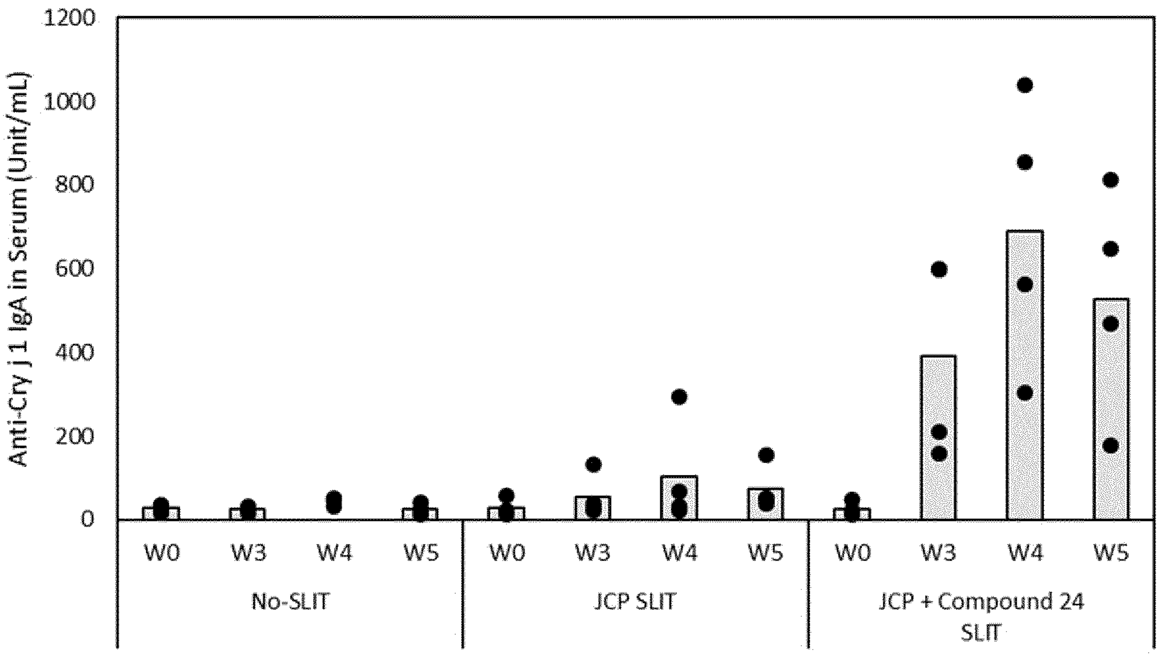
Anti-Cry j 1 IgA in Serum (Unit/mL)
1200
1000
800
600
400
200
0
W0
W3
W4
W5
W0
W3
W4
W5
W0
W3
W4
W5
No-SLIT
JCP SLIT
JCP + Compound 24 SLIT

FIG. 11

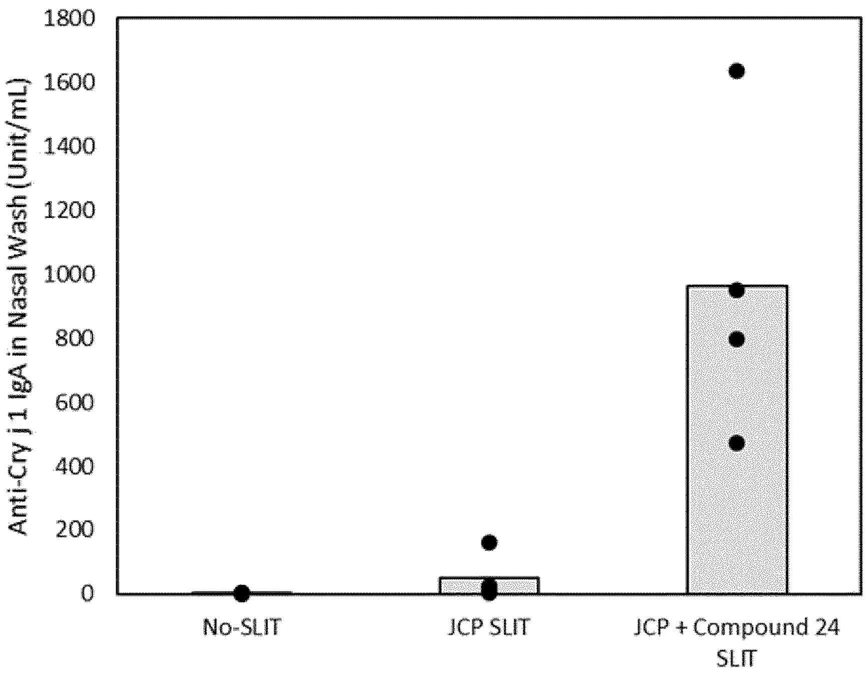
1800
1600
1400
1200
1000
800
600
400
200
0
Anti-Cry j 1 IgA in Nasal Wash (Unit/mL)
No-SLIT
JCP SLIT
JCP + Compound 24 SLIT

FIG. 12

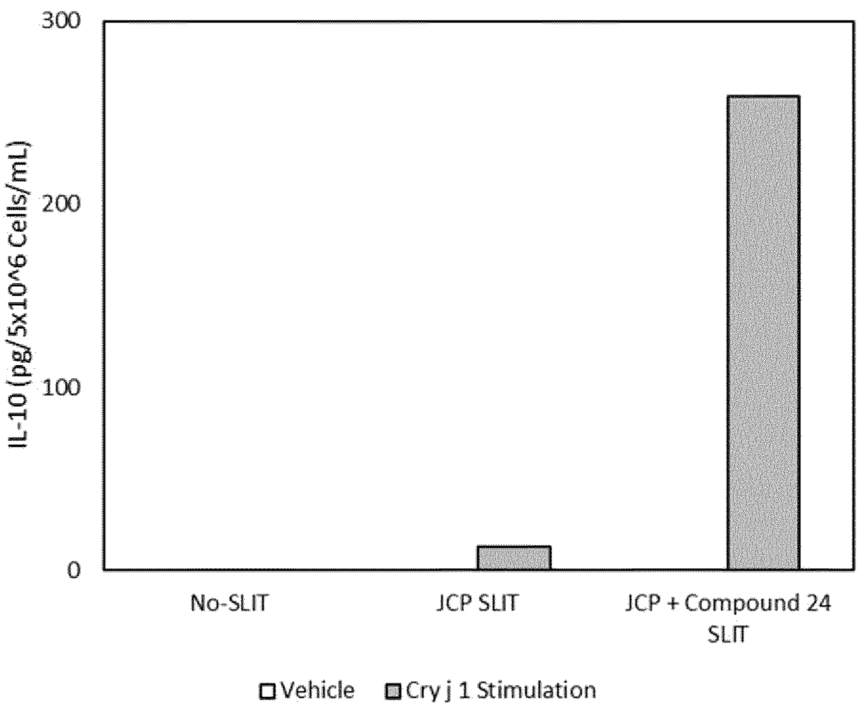
300
200
100
0
IL-10 (pg/5x10^6 Cells/mL)
No-SLIT
JCP SLIT
JCP + Compound 24 SLIT
Vehicle
Cry j 1 Stimulation

FIG. 13

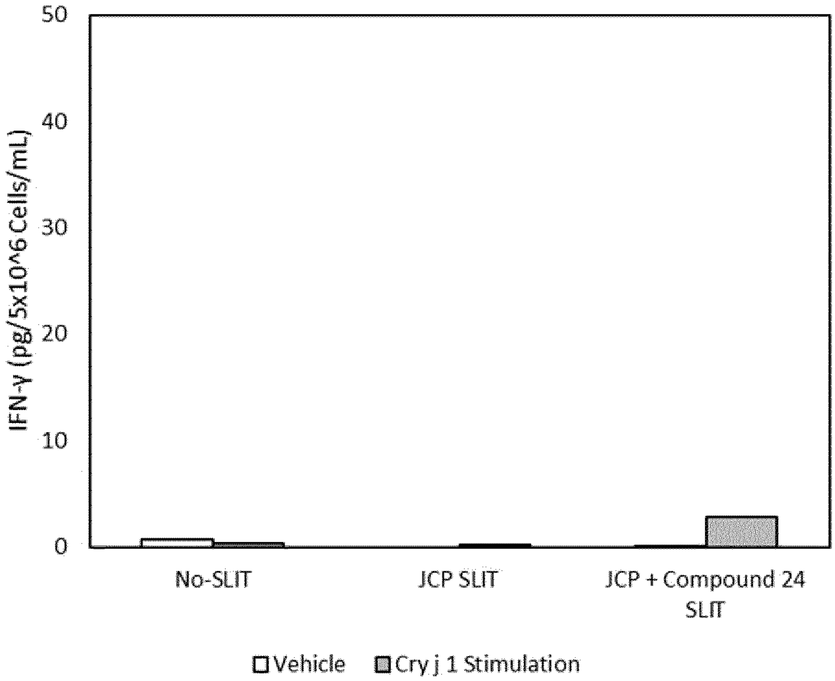
50
40
30
20
10
0
IFN-γ (pg/5x10^6 Cells/mL)
No-SLIT
JCP SLIT
JCP + Compound 24 SLIT
Vehicle
Cry j 1 Stimulation

FIG. 14

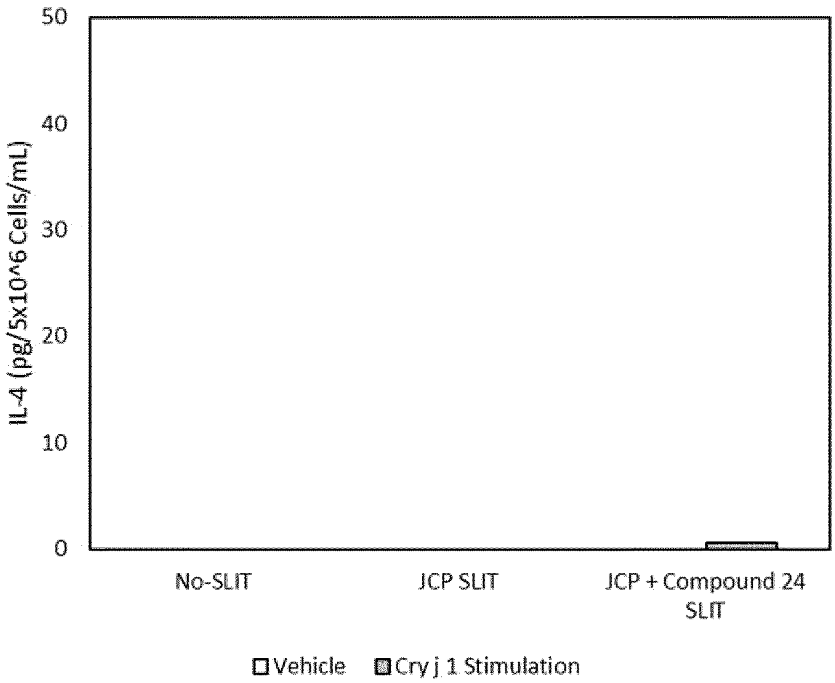
50
40
30
20
10
0
IL-4 (pg/5x10^6 Cells/mL)
No-SLIT
JCP SLIT
JCP + Compound 24 SLIT
Vehicle
Cry j 1 Stimulation

FIG. 15

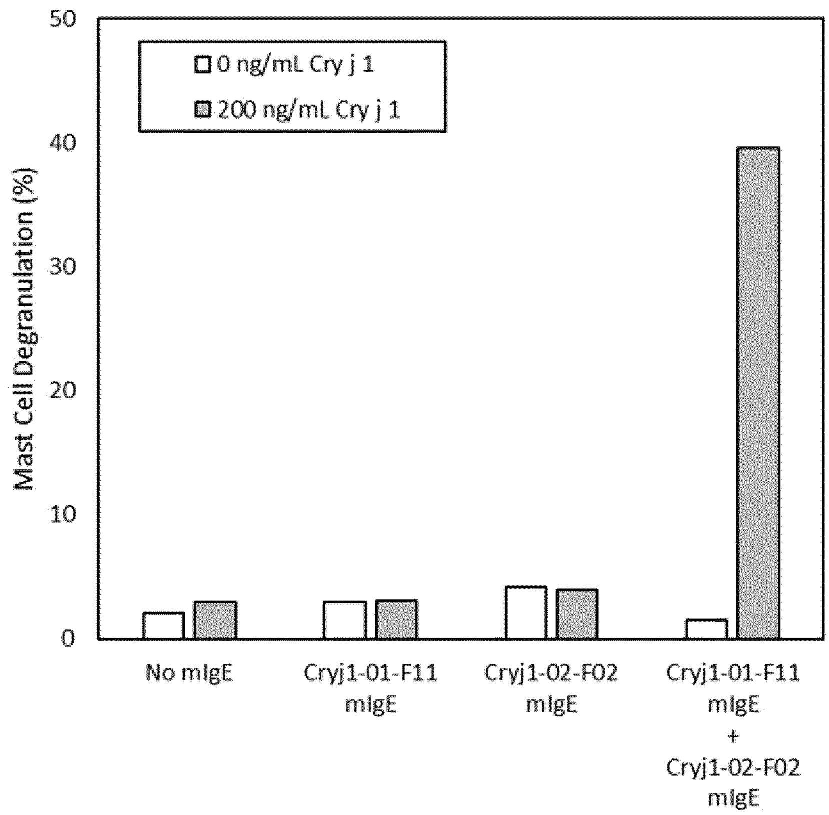

50
40
30
20
10
0
Mast Cell Degranulation (%)
0 ng/mL Cry j 1
200 ng/mL Cry j 1
No mIgE
Cryj1-01-F11 mIgE
Cryj1-02-F02 mIgE
Cryj1-01-F11 mIgE + Cryj1-02-F02 mIgE

FIG. 16

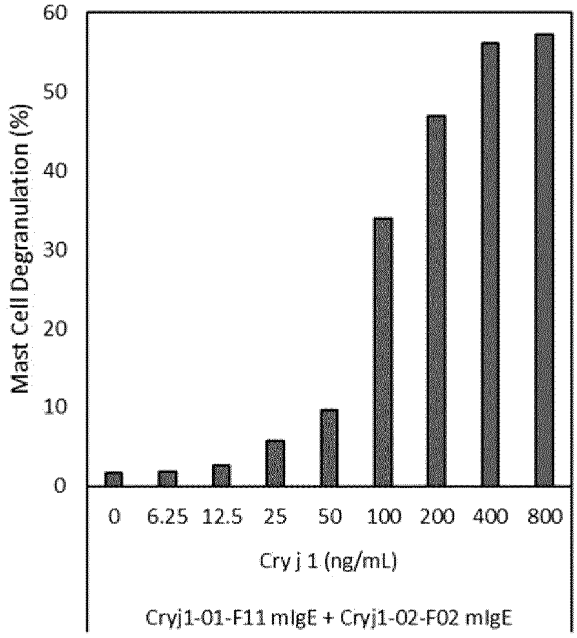
60
50
40
30
20
10
0
Mast Cell Degranulation (%)
0
6.25
12.5
25
50
100
200
400
800
Cry j 1 (ng/mL)
Cryj1-01-F11 mIgE + Cryj1-02-F02 mIgE

FIG. 17

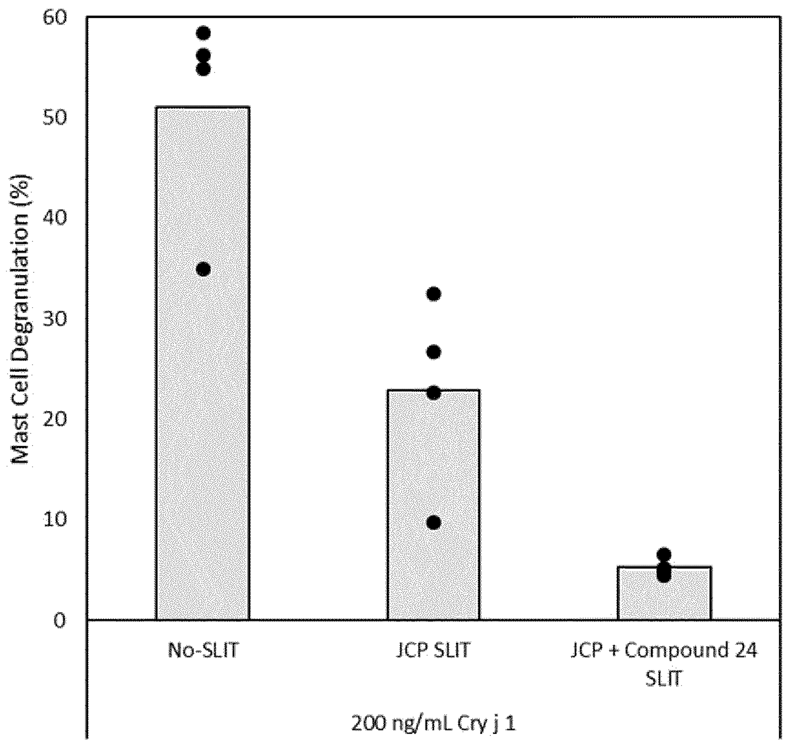
60
50
40
30
20
10
0
Mast Cell Degranulation (%)
No-SLIT
JCP SLIT
JCP + Compound 24 SLIT
200 ng/mL Cry j 1

FIG. 18

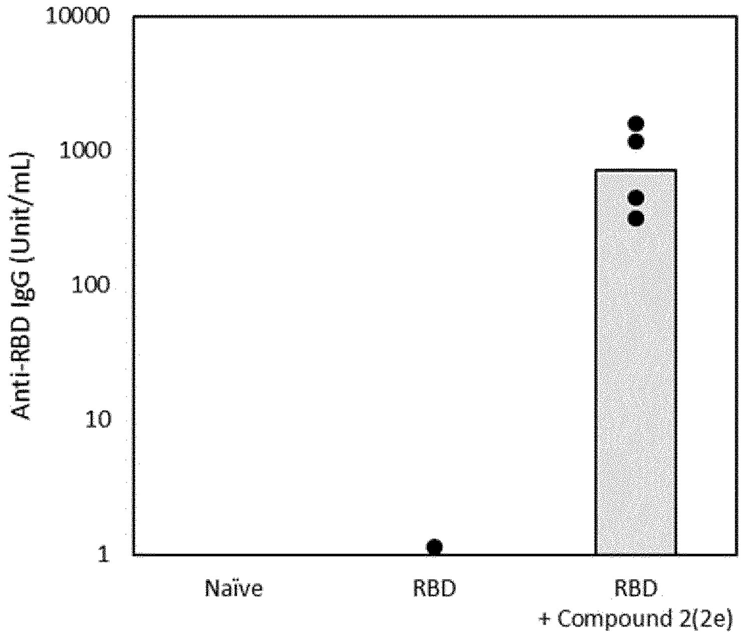
10000
1000
100
10
1
Anti-RBD IgG (Unit/mL)
Naïve
RBD
RBD
+ Compound 2(2e)

FIG. 19

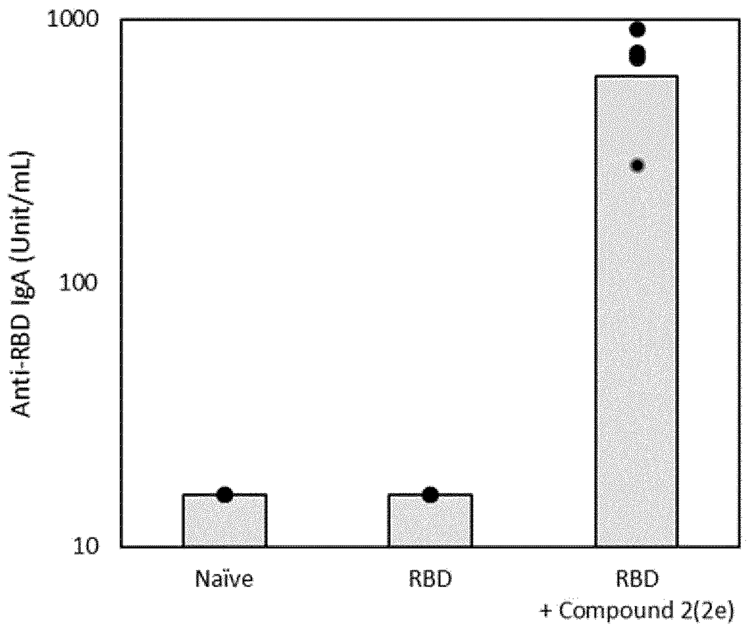
1000
100
10
Anti-RBD IgA (Unit/mL)
Naïve
RBD
RBD
+ Compound 2(2e)

FIG. 20

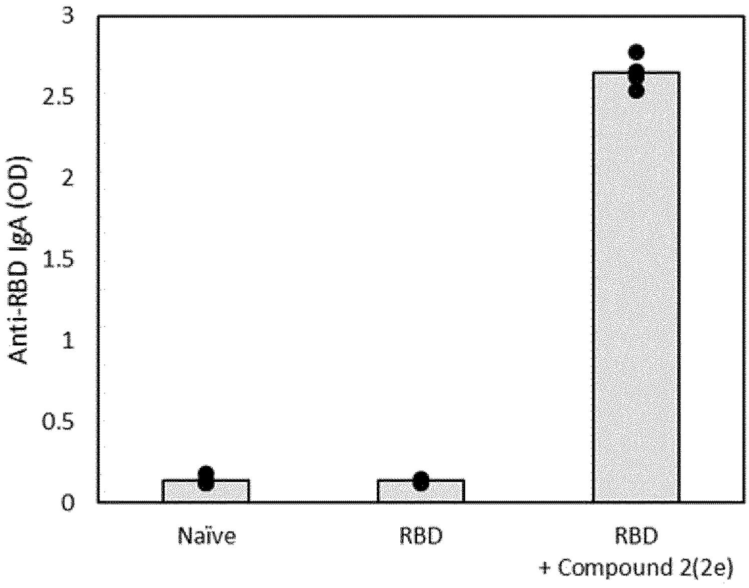
Anti-RBD IgA (OD)
3
2.5
2
1.5
1
0.5
0
Naïve
RBD
RBD + Compound 2(2e)

FIG. 21

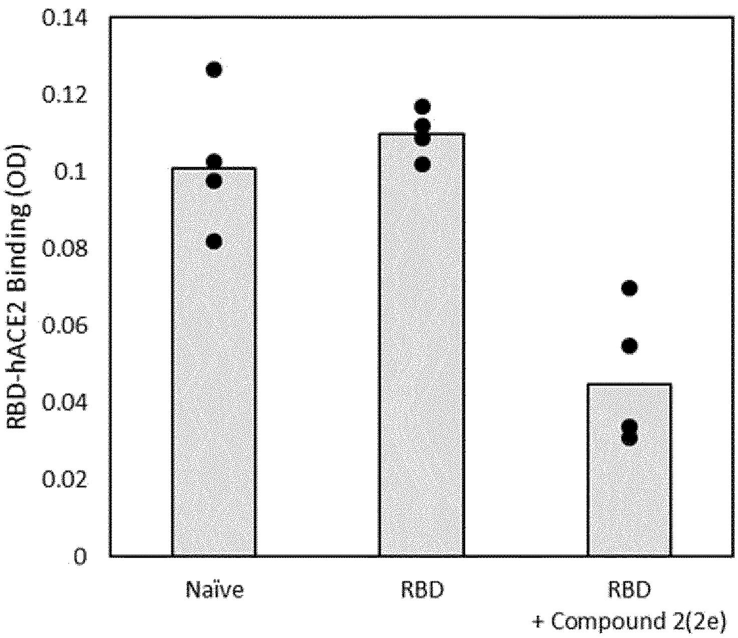
RBD-hACE2 Binding (OD)
0.14
0.12
0.1
0.08
0.06
0.04
0.02
0
Naïve
RBD
RBD + Compound 2(2e)

## INTERNATIONAL SEARCH REPORT

International application No.
**PCT/JP2021/041323**

**A. CLASSIFICATION OF SUBJECT MATTER**

***C07H 15/04***(2006.01)i; ***A61K 39/12***(2006.01)i; ***A61K 39/145***(2006.01)i; ***A61K 39/165***(2006.01)i; ***A61K 39/20***(2006.01)i; ***A61K 39/215***(2006.01)i; ***A61K 39/235***(2006.01)i; ***A61K 39/35***(2006.01)i; ***A61K 39/36***(2006.01)i; ***A61K 39/39***(2006.01)i; ***A61P 31/04***(2006.01)i; ***A61P 31/12***(2006.01)i; ***A61P 31/16***(2006.01)i; ***A61P 33/02***(2006.01)i; ***A61P 37/08***(2006.01)i; ***A61P 39/02***(2006.01)i
FI: C07H15/04 F CSP; A61K39/12; A61K39/145; A61K39/235; A61K39/20; A61K39/165; A61K39/215; A61K39/35; A61K39/36; A61K39/39; A61P31/12; A61P37/08; A61P31/04; A61P39/02; A61P33/02; A61P31/16

According to International Patent Classification (IPC) or to both national classification and IPC

**B. FIELDS SEARCHED**

Minimum documentation searched (classification system followed by classification symbols)

C07H15/04; A61K39/12; A61K39/145; A61K39/165; A61K39/20; A61K39/215; A61K39/235; A61K39/35; A61K39/36; A61K39/39; A61P31/04; A61P31/12; A61P31/16; A61P33/02; A61P37/08; A61P39/02

Documentation searched other than minimum documentation to the extent that such documents are included in the fields searched

Published examined utility model applications of Japan 1922-1996
Published unexamined utility model applications of Japan 1971-2021
Registered utility model specifications of Japan 1996-2021
Published registered utility model applications of Japan 1994-2021

Electronic data base consulted during the international search (name of data base and, where practicable, search terms used)

JSTPlus/JMEDPlus/JST7580 (JDreamIII); CAplus/REGISTRY (STN)

**C. DOCUMENTS CONSIDERED TO BE RELEVANT**

| Category* | Citation of document, with indication, where appropriate, of the relevant passages | Relevant to claim No. |
|---|---|---|
| X | JP 2016-512226 A (GLAXOSMITHKLINE BIOLOGICALS S.A) 25 April 2016 (2016-04-25) claims 15, 16, 29, 39, etc. | 1, 6-15 |
| Y | | 1, 6-15 |
| A | | 2-5 |
| X | JP 2008-505897 A (CORIXA CORPORATION) 28 February 2008 (2008-02-28) compounds 1b, 2b, claims 56, 90, etc. | 1, 6-15 |
| Y | | 1, 6-15 |
| A | | 2-5 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **27 December 2021** | **18 January 2022** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (January 2015)

**INTERNATIONAL SEARCH REPORT**

International application No.
**PCT/JP2021/041323**

**C. DOCUMENTS CONSIDERED TO BE RELEVANT**

| Category* | Citation of document, with indication, where appropriate, of the relevant passages | Relevant to claim No. |
|---|---|---|
| X | JP 2004-508292 A (CORIXA CORPORATION) 18 March 2004 (2004-03-18) claims 27, 28, etc. | 1, 6-15 |
| Y | | 1, 6-15 |
| A | | 2-5 |
| X | Bioorganic & Medicinal Chemistry Letters. 2015, 25, 547-553 fig. 1.2 AGP generic structure, fig. 2 | 1, 6-15 |
| Y | | 1, 6-15 |
| A | | 2-5 |
| X | JP 2018-525378 A (GLAXOSMITHKLINE INTELLECTUAL PROPERTY DEVELOPMENT LIMITED) 06 September 2018 (2018-09-06) claims 9, 11, etc. | 1, 6-15 |
| Y | | 1, 6-15 |
| A | | 2-5 |
| X | JP 2018-522053 A (GLAXOSMITHKLINE INTELLECTUAL PROPERTY DEVELOPMENT LIMITED) 09 August 2018 (2018-08-09) claim 5, etc. | 1, 6-15 |
| Y | | 1, 6-15 |
| A | | 2-5 |

Form PCT/ISA/210 (second sheet) (January 2015)

**INTERNATIONAL SEARCH REPORT**
**Information on patent family members**

International application No.
**PCT/JP2021/041323**

| Patent document cited in search report | Publication date (day/month/year) | Patent family member(s) | Publication date (day/month/year) |
|---|---|---|---|
| JP 2016-512226 A | 25 April 2016 | US 2016/0022719 A1<br>claims 15, 16, 29, 39, etc.<br>WO 2014/141127 A1<br>EP 2968376 A1<br>CN 105188710 A | |
| JP 2008-505897 A | 28 February 2008 | WO 2006/016997 A2<br>compounds 1b, 2b, claims 56, 90, etc.<br>WO 2006/012425 A2<br>WO 2004/062599 A2<br>WO 2004/005308 A2<br>US 2008/0033155 A1<br>US 2005/0227943 A1<br>US 2005/0107600 A1<br>US 2004/0267007 A1<br>KR 10-2005-0090441 A<br>KR 10-2005-0037460 A<br>JP 2007-524570 A<br>JP 2005-536493 A<br>EP 1776375 A2<br>EP 1589934 A2<br>EP 1521762 A2<br>CN 101080234 A<br>CN 101010328 A<br>CN 1675231 A | |
| JP 2004-508292 A | 18 March 2004 | US 2002/0077304 A1<br>claims 27, 28, etc.<br>WO 2001/090129 A2<br>EP 1284740 A1<br>EP 2000144 A1<br>CN 1606446 A<br>CN 1907289 A<br>KR 10-0829674 B1 | |
| JP 2018-525378 A | 06 September 2018 | US 2017/0275371 A1<br>claims 9, 11, etc.<br>US 2019/0338042 A1<br>WO 2017/021791 A1<br>EP 3331560 A1<br>KR 10-2018-0032641 A<br>CN 108025073 A | |
| JP 2018-522053 A | 09 August 2018 | US 2018/0221399 A1<br>claim 5, etc.<br>WO 2017/021792 A1<br>EP 3331565 A1<br>KR 10-2018-0032642 A<br>CN 108136024 A | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 199850399 A


### Non-patent literature cited in the description


- **MCKEE AS et al.** *BMC biology.,* 2010, vol. 8, 37
- **ARAKAWA T.** *Expert review of vaccines,* 2011, vol. 10 (1), 1-5
- **JANEWAY CA, JR.** *Cold Spring Harbor symposia on quantitative biology,* 1989, vol. 54, 1-13
- **POLTORAK A et al.** *Science,* 1998, vol. 282 (5396), 2085-8
- **LUDERITZ O ; GALANOS C ; LEHMANN V ; NURMINEN M ; RIETSCHEL ET ; ROSENFELDER G et al.** *The Journal of infectious diseases,* 1973, vol. 128, 17-29
- **MATA-HARO V et al.** *Science,* 2007, vol. 316 (5831), 1628-32
- **QURESHI N et al.** *The Journal of biological chemistry,* 1982, vol. 257 (19), 11808-15
- **DRACHENBERG KJ et al.** *Allergy.,* 2001, vol. 56 (6), 498-505
- **MCGHEE JR ; FUJIHASHI K.** *PLoS biology.,* 2012, vol. 10 (9), e1001397
- **LYCKE N.** *Nature reviews Immunology.,* 2012, vol. 12 (8), 592-605
- **HOLMGREN J ; CZERKINSKY C.** *Nature medicine.,* 2005, vol. 11 (4), 45-53
- **NOON L.** *Lancet.,* 1911, vol. 177 (4580), 1572-3
- **SHAMJI MH ; DURHAM SR.** *The Journal of allergy and clinical immunology.,* 2017, vol. 140 (6), 1485-98
- **LARSEN JN et al.** *Drug discovery today,* 2016, vol. 21 (1), 26-37
- **AKDIS M ; AKDIS CA.** *The Journal of allergy and clinical immunology,* 2014, vol. 133 (3), 621-31
- **ORENGO JM et al.** *Nature communications,* 2018, vol. 9 (1), 1421
- **BR MED J.** CSM update: desensitizing vaccines. *Committee on the safety of medicines,* 1986, vol. 293, 948
- **NOWAK-WEGRZYN A et al.** *Current opinion in allergy and clinical immunology,* 2019, vol. 19 (6), 606-13
- *Iyakuhin No Kaihatsu (Development of Pharmaceuticals in English),* vol. 7
- **HIROKAWA-SHOTEN LTD.** *Bunshi Sekkei (Molecular Design in English),* 1990, 163-198
- Organic Functional Group Preparations. Academic Press, Inc, 1989
- Comprehensive Organic Transformations. VCH Publishers Inc, 1989
- **T.W. GREENE ; P.G. WUTS.** Protective Groups in Organic Synthesis. John Wiley & Sons, Inc, 2006
- **IMOTO et al.** *Tetrahedron Lett.,* 1985, vol. 26, 1545-1548
- *Tetrahedron Letters,* 1985, vol. 26 (12), 1545-1548
- *Tetrahedron Letters,* 2006, vol. 47 (13), 2087-2092
- *Angewandte Chemie, International Edition,* 2001, vol. 40, 1475-1480
- *Bioorganic & Medicinal Chemistry Letters,* 2008, vol. 18, 5350-5354
- *Peptide Science,* 2009, 179-182
- *Journal of Endotoxin Research,* 1994, vol. 1 (3), 149-163
- *Tetrahedron Letters,* 2007, vol. 48, 7279-7282
- *European Journal of Organic Chemistry,* 1998, vol. 8, 609-1613
- *Tetrahedron,* 2007, vol. 63, 8499-8513